# EUROPEAN PATENT SPECIFICATION

(11) **EP 2 132 332 B1**
(45) Date of publication and mention of the grant of the patent: **22.01.2014**
(21) Application number: 08744733.0
(22) Date of filing: 31.03.2008
(51) Int. Cl.: C12Q 1/68

(54) **MEASUREMENT OF AN INSOLUBLE ANALYTE IN A SAMPLE**
MESSUNG EINES UNLÖSLICHEN ANALYTS IN EINER PROBE
MESURE D'UN ANALYTE INSOLUBLE DANS UN ECHANTILLON

(30) Priority: 30.03.2007 US 920814 P; 03.01.2008 US 18717; 28.03.2008 US 58205
(43) Date of publication of application: 16.12.2009
(73) Proprietor: High Throughput Genomics, Inc., Tucson, AZ 85712 (US)
(72) Inventor: SELIGMANN, Bruce, Tucson Arizona 85750 (US); MARTEL, Ralph, Tucson Arizona 85718 (US); ROUNSEVILLE, Matthew, Tucson Arizona 85713 (US); BOTROS, Ihab, Tucson Arizona 85750 (US)
(74) Representative: Seuss, Thomas
(86) International application number: PCT/US2008/058837
(87) International publication number: WO 2008/121927

(56) References cited:
- WO-A-97/40189
- KITAZAWA SOHEI ET AL: "In situ hybridization with polymerase chain reaction-derived single-stranded DNA probe and S1 nuclease" HISTOCHEMISTRY AND CELL BIOLOGY, vol. 111, no. 1, January 1999 (1999-01), pages 7-12, XP002484289 ISSN: 0948-6143
- ROBERTS ROBIN A ET AL: "Quantitative nuclease protection assay in paraffin-embedded tissue replicates prognostic microarray gene expression in diffuse large-B-cell lymphoma" LABORATORY INVESTIGATION, vol. 87, no. 10, October 2007 (2007-10), pages 979-997, XP002484290 ISSN: 0023-6837
- MARTEL RALPH R ET AL: "Multiplexed screening assay for mRNA combining nuclease protection with luminescent array detection." ASSAY AND DRUG DEVELOPMENT TECHNOLOGIES, vol. 1, no. 1, November 2002 (2002-11), pages 61-71, XP002484291 ISSN: 1540-658X

## Description

This application claims the benefit of the filing date of U.S. Provisional Application Serial No. 60/920,814 filed March 30, 2007, and of U.S. Provisional Application Serial No. 61/018,717 filed January 3, 2008.

### BACKGROUND OF THE INVENTION

Pluralities of molecular probes arranged on surfaces or "chips" have been used in a variety of biological and chemical assays. Assays are performed to determine if target molecules of interest interact with any of the probes. After exposing the probes to target molecules under selected test conditions, detection devices determine whether a target molecule has interacted with a given probe.

These systems are useful in a variety of screening procedures for obtaining information about either the probes or the target molecules. For example, they have been used to screen for peptides or potential drugs which bind to a receptor of interest, among others: to screen samples containing insoluble targets for the presence of, for example, genetic mutations, allelic variants in a population, or a particular pathogen or strain of pathogen, among many others; to study gene expression, for example to identify the mRNAs whose expression is correlated with a particular physiological condition, developmental stage, or disease state, etc.

WO97/40189 describes a high volume substrate-based *in-situ* hybridization assay with some sensitivity for the detection of mRNA transcripts. Kitazawa et al. ("In situ hybridization with polymerase chain reaction-derived single-stranded DNA probe and S1 nuclease", Histochemistry and Cell Biology, vol. 111, No. 1, January 1999, pages 7 - 12) describes a protocol for in situ hybridization (ISH) with polymerase chain reaction (PCR)-derived single-stranded DNA probes.

### DESCRIPTION OF THE INVENTION

The accurate measurement of genes, and in particular gene expression, or oligonucleotides, from fixed tissue would have many benefits. In the case of clinical samples the described process permits target oligonucleotides to be measured without necessitating a change in clinical practice - directly from fixed tissue without having to prepare frozen samples. There are vast stores of archived fixed material that could be used for retrospective studies to identify and validate biomarkers and target genes, or for development and validation of a monitoring, prognostic, or diagnostic assay, or for the association of safety with gene expression, or for the understanding of disease processes, etc. However, such measurements from fixed tissue have been problematic. Measurement by PCR or hybridization methods requires large amounts of tissue and complex extraction and sample preparation methods. In addition, it is often observed that the quality of measurement decreases as a function of how long the tissue has been stored. In contrast, *in situ* measurements (where the RNA is labeled and visualized in the tissue) can be performed on freshly fixed tissue or archived tissue and produce similar quality data.

Described herein is a method for measuring oligonucleotides from fixed tissues comprising recovering a probe from the tissue wherein said probe serves as the basis for measurement, rather than the native oligonucleotide itself. The instant invention is further drawn to the use of nuclease protection as a method to measure oligonucleotides from fixed tissue. Methods such as PCR require the target RNA to be solubolized, extracted, and purified, then reverse transcribed and the resultant cDNA amplified. bDNA requires the target RNA to be solubolized. Thus, crosslinking needs to be reversed or only a portion of the RNA may be recovered as soluble RNA for measurement by these methods.

The method disclosed by the instant invention permits the measurement of cross-linked oligonucleotides as well as soluble oligonucleotides.

Tissue sections obtained from clinical or animal experimentation are frequently fixed, embedded and stored in a form suitable for later examination by microscopy. Traditional fixation methods frequently have employed aldehyde fixatives, which fix the tissue by causing cross-linking reactions within and between tissue proteins. Cross-links tend to preserve tissue morphology and integrity, harden the tissue for slicing, and inhibit microbial attack. After tissue samples have been fixed, they are typically embedded in an embedding medium so that the samples may be cut into thin sections. Paraffin is the most common embedding medium, although acrylamide and celloidin may also be used.

Other non-formalin fixation, such as ethanol or aldehyde fixation are also known in the art. Aldehyde fixation tends to cause substantial changes to the structure of the tissue sample. These changes often tend to cause the targets that may be present in the tissue samples to lose their reactivity toward antibodies that target such antigens. One effect of formalin fixation is to substantially lock the three dimensional shape of protein molecules within the tissue samples. Because of the recent development of new histochemical reagents, histochemical analyses may now be performed that were impossible to perform at the time many tissues were originally stored. Therefore, a number of procedures have been developed which could reverse some of the changes produced by aldehyde fixation, and enhance the histochemical staining properties of the tissue sample.

One routine laboratory protocol for improving the staining abilities of tissue samples which have been fixed in formalin and embedded in acrylamide gel relates to treatment of acrylamide gel embedded tissue in 1.0% 2-mercaptoethanol for 15 minutes, followed by rinsing with phosphate buffered saline. A method for restoring the histochemical staining properties of tissue samples is described in U.S. Pat. No. 5,244,787 to Key et al. Another method for restoring the histochemical staining properties of tissue samples is described in U.S. Pat. No. 5,578,452 by Shi et al.

The measurement of a biological target in fixed or preserved samples is a technically challenging venture. Proteins are known to denature, often losing antigenecity (i.e., antibody recognition) in the process. Carbohydrates can be chemically altered, particularly those associated with peptides and proteins in a glycoprotein moiety. Nucleic acids can undergo cross-linking between one another, and other molecules, including proteins, lipids, and carbohydrates, in the cellular milieu. The recovery and analysis of these molecules is an expensive and a time-consuming process.

Study of nucleic acids in fixed tissues is particularly difficult due to various reasons. The added layer or coating in formalin-fixed paraffin-embedded samples presents considerable challenge to conventional probes and reagents. Moreover, since most probes rely on direct attachment for detection, considerable modifications in the secondary and/or tertiary structures of targets could present difficulty from a recognition and detection standpoint. Another aspect is specificity of recognition, which may be lost due to unnatural exposure to chemicals and other factors during fixation.

The art knowledge on analysis of nucleic acids in fixed biological specimen is relatively scant. Daniel D'Orazio (American Journal of Pathology. 2002;160:383-384) observed measurement of RNA in fixed samples, however quantitative measurement from matched frozen tissue did not correlate with the fixed tissue measurement. The authors further suggest that the great variability between fixed tissue samples seen by Sprecht (e.g. Her-2/Neu mRNA) may be artifact caused by the impact of fixation on available RNA for measurement, fixation parameters such as fixation delay, time, and temperature may account for the large expression variability. It is possible that the variability is likely due to variability in the ratio of cross-linked to soluble RNA.

Art knowledge on reagents and methods for the analysis of nucleic acids and other biological targets in fixed biological specimen are based on in situ detection methods. In situ hybridization (ISH) has been developed to overcome the limitations of flow cytometry (FCM), karyotyping and molecular genetics, when it relates to fixed specimen. However, such the probes used in ISH are not specific for the particular gene that is interrupted or altered by the deletions, translocations, inversions or amplifications. Thus detection of the genetic abnormality may not be possible if the sequences that are analyzed are not properly represented in the sample. Furthermore, the number of variables (i.e., targets) that can be detected are fairly low, and automation is usually not possible.

Furthermore, ISH techniques are utilized on freshly isolated cells or cultured cells, rather than fixed paraffinated tissues. To date, previous hybridization studies have been used on cell lines, disassociated fresh tissue or frozen tissue sections. However, typically in the clinical setting it is not always possible to work on biopsy tissue as soon as it is available, since frequently, the only tissue available is paraffin embedded tissue. Furthermore the use of fixed paraffinated issue is advantageous because the tissue structure is preserved. For example, nucleic acids are prone to the action of nucleases. It was believed that in situ hybridization of fixed tissue could only be done with probes for repetitive nucleic acid sequences because the fixation prevents detection of a single copy sequence. Thus single deletions of nucleic acid sequences (for example, SNP) could not be detected. In situ methods can be used to measure DNA and RNA from fixed tissue, imaging the tissue and quantifying from those images of the hybridized, tissue bound probes. Detection can be by fluorescence (FISH) or luminescence, and methods can measure DNA (for instance copy number) or RNA.

Hellborg (Ann. Onc., 2007) describes PCR and microarray measurements as alternatives to fluorescence in situ hybridization (FISH) detection of RNA. However, both PCR-dependent and microarray techniques are laden with limitations. For example, extraction and assay by PCR is both expensive as well as cumbersome (Shibutani et al., Lab Invest 2000, 80:199-208). Another important aspect is that microaray works on FFPE wherein soluble RNA is of good quality. So microarray can be utilized for measuring the soluble pool. However, these techniques do not enable rapid, quantitative measurement of RNA from fixed samples, particularly when the target is of insoluble nature.

It is evident that methods for detecting relevant biological targets and biomarkers in fixed tissue would be advantageous.

Measurements from fixed samples can be made using a single array, both low and high density, and both fixed (capture probes printed as the array) or programmable (combinations of printed anchors and added programming/capture linkers), or multiple arrays such as might be printed in the wells of a microplate or on bead arrays, including beads in solution measuring multiple genes in each sample, or by the tagging of the nuclease protection proteins with or without fixation to a surface and imaging, or by use of gels, electrophoresis, chromatography, mass spectroscopy, sequencing, as mixtures, or as individual targets detected in each reaction mixture, such as in a conventional microplate assay, or by PCR (or other amplification method) of the nuclease protection probe or by hybrid capture, or other method one skilled in the art might use. The reagents and methods described herein permit measurement of oligonucleotides from host and infectious agent (different species) from a fixed tissue sample, in the same experiment, from the same sample, and even in the same well of an ArrayPlate. The measurement of different forms of oligonucleotide from fixed samples, both single samples as well as to make comparisons between samples, including for instance, diseased versus normal, treated versus control, or the same sample, such as mRNA and DNA, or microRNA and RNA, or ribosomal RNA and mRNA, or mitochondrial RNA and mRNA, or any combinations of these, can be performed. The measurement of protein using aptamers, or other probes, are also permissible with the instant invention. The instant invention also relates to measurement of proteins and oligonucleotides simultaneously using appropriate probes. In yet another aspect, the instant invention relates to the hybridization (or binding) of probes to cross-linked (and soluble) RNA, and then removal and measurement of the probe, or probe/target molecule, even where the target molecule may be damaged, fractured or cleaved, but the probe or probe complex is intact or held together sufficiently. For instance, in the case of bDNA the probe complex can be formed on the cross-linked target RNA, and then methods used to release the complex even thought the process may result in breaks in the RNA template, yet the combined complex remains sufficiently in-tact to permit its measurement outside of the tissue. Any method where the probe associates with both cross-linked or surface bound target molecule (e.g. oligonucleotide or e.g. RNA) and soluble target molecule, or associated only with the cross-linked or surface bound target molecule, is reduced to an analyzable amount relative to the target molecule, then removed from the tissue and measured.

This description further provides compositions, apparatus and methods for performing one or more biological or chemical assays on any type of single or multiple arrays, including high density arrays measuring up to several hundred thousand target molecules. This description further provides compositions, apparatus and methods for concurrently performing multiple biological or chemical assays, and allows for high throughput analysis of multiple samples containing insoluble targets - for example, multiple patient samples to be screened in a diagnostic assay, or multiple potential drugs or therapeutic agents to be tested in a method of drug discovery. A combination is provided which is useful for the detection of one or more targets in a sample. This combination comprises a surface comprising a plurality of spatially discrete regions, which can be termed test regions and which can be wells, at least two of which are substantially identical. Each surface comprises at least two, preferably at least twenty or more, e.g., at least about 25, 50, 96, 864, or 1536, etc., of such substantially identical regions. Each test region defines a space for the introduction of a sample containing (or potentially containing) one or more targets and contains a biological or chemical array. (Phrases such as "sample containing a target" or "detecting a target in a sample" are not meant to exclude samples or determinations (detection attempts) where no insoluble target is contained or detected. In a general sense, this invention involves arrays to determine whether a target is contained in a sample irrespective of whether it is or is not detected.) This array comprises generic "anchors," each in association with a bifunctional linker molecule which has a first portion that is specific for the anchor and a second portion that comprises a probe which is specific for at least one of the target(s). The combination of this invention is placed in contact with a sample containing one or more targets, which optionally react with a detector molecule(s), and is then interrogated by a detection device which detects reactions between target molecules and probes in the test regions, thereby generating results of the assay.

The invention provides methods particularly useful for high throughput biological assays. In especially preferred embodiments, the invention can be used for high throughput screening for drug discovery. For example, a high throughput assay can be run in many (100 for example) 96-well microplates at one time. Each well of a plate can have, e.g., 16 different tests performed in it by using an array of about 16 anchor and linker pairs. That is, 100 plates, with 96 wells per plate, and each with 16 tests per well, can allow for a total of 203,600 tests; for example, each of 9,600 different drug candidates can be tested simultaneously for 16 different parameters or assays. High throughput assays provide much more information for each drug candidate than do assays which test only one parameter at a time. For example, it is possible in a single initial high throughput screening assay to determine whether a drug candidate is selective, specific and/or nontoxic. Non-high throughput methods necessitate extensive follow-up assays to test such parameters for each drug candidate of interest. Several types of high throughput screening assays are known in the art. The ability to perform simultaneously a wide variety of biological assays and to do very many assays at once (i.e., in very high throughput) are two important advantages of the invention.

In one embodiment, for example, using 96-well DNA Bind plates (Corning Costar) made of polystyrene with a derivatized surface for the attachment of primary amines, such as amino acids or modified oligonucleotides, a collection of 16 different oligonucleotides can be spotted onto the surface of every well of every plate to serve as anchors. The anchors can be covalently attached to the derivatized polystyrene, and the same 16 anchors can be used for all screening assays. For any particular assay, a given set of linkers can be used to program the surface of each well to be specific for as many as 16 different targets or assay types of interest, and different test samples can be applied to each of the 96 wells in each plate. The same set of anchors can be used multiple times to re-program the surface of the wells for other targets and assays of interest, or it can be re-used multiple times with the same set of linkers. This flexibility and reusability represent further advantages of the invention.

Another embodiment of the invention is a method for detecting at least one insoluble target, which comprises contacting a sample which may comprise the target(s) with a combination as described above, under conditions effective for said target(s) to bind to said combination. Another embodiment is a method for determining an RNA expression pattern, which comprises incubating a sample which comprises as target(s) at least two RNA molecules with a combination as described above, wherein at least one probe of the combination is a nucleic acid (e.g., oligonucleotide) which is specific (i.e. selective) for at least one of the insoluble RNA targets, under conditions which are effective for specific hybridization of the RNA target(s) to the probe(s). Another embodiment is a method for identifying an agent (or condition(s)) that modulates an RNA expression pattern, which is the method described above for determining an RNA expression pattern, further comprising comparing the RNA expression pattern produced in the presence of said agent (or condition(s)) to the RNA expression pattern produced under a different set of conditions.

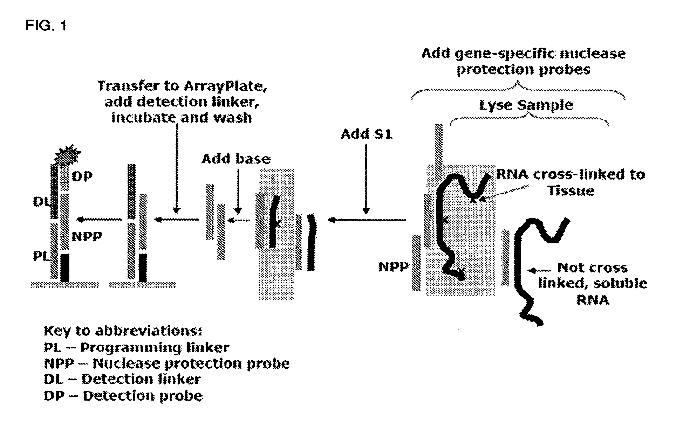

By way of example, Figure 1 schematically illustrates one of those anchors, anchor 1, which, in a most preferred embodiment of the invention, is an oligonucleotide. To anchor 1 is attached a linker molecule, linker 1, which comprises two portions. The first portion, which is specific for the anchor, is in this illustration an oligonucleotide which can hybridize specifically to the anchor. The second portion, which is a probe specific for the insoluble target of interest - here, target mRNA - is in this illustration an oligonucleotide which can hybridize to that target. Although not illustrated in this figure, each of the remaining 15 anchors can hybridize to its own linker via the anchor-specific portion; each linker can contain a probe portion specific for, e.g., an mRNA different from (or the same as) mRNA 1. This illustrated combination can be used to assay as many as 96 different samples at the same time for the presence of mRNA 1 (or, simultaneously, for mRNA targets which are specified (programmed) by the other five probes in the array). To perform the assay, each sample, which in this example can be an RNA extract from, say, one of 96 independent cell lines, is added in a small volume to one of the regions, or wells, and incubated under conditions effective for hybridization of the probe and the insoluble target. In order to determine if mRNA 1 is present in a sample, a detection device which can recognize patterns, and/or can interrogate specific locations within each region for the presence of a signal, is employed. If the cell lines are incubated under conditions in which their mRNAs are labeled in vivo with a tag, and if mRNA 1 is present in a sample, the detector will detect a signal emanating from the tagged mRNA at the location defined by anchor/probe complex 1. Alternatively, the mRNA can be directly labeled in vitro, before or after being added to the regions (wells). Alternatively, the mRNA can be tagged indirectly, before or after it has hybridized to the probe, e.g., by incubating the RNA with a tagged "detector" oligonucleotide (target-specific reporter oligonucleotide) which is complementary to a sequence other than that recognized by the probe. In the illustrated example, 15 samples can be analyzed simultaneously. Because at least 20 or more, e.g., as many as 1536 or more, samples can be analyzed simultaneously with this invention, it is a very high throughput assay system.

As used herein, "insoluble" refers to an entity which exists heterogeneously, with respect to its solvent, in a mixture or a solution comprising the two. "Insoluble" may also be used to characterize substances of particulate nature, for example, cells, vacuoles, membranes, and/or aggregates thereof. "Insoluble" may also be used to describe that which, although capable of existing homogenously in the solution, exists heterogeneously because it is attached to a surface or fixed to a residue. The term can also be used to define matter which is left behind following dissolution or lysis (for example, a precipitate).

Insoluble as used herein is commensurate with any solvent. Preferably, the solvent is polar (for example, water, saline, PBS, Krebs-Ringer buffer, reticulocyte lysate, serum, etc.) and the solubility is determined at a temperature from between 4°C to 100°C. The "insoluble" character can be determined by routine techniques, for example, light scattering or filtration or centrifugation. Insoluble includes target that is chemically or physically crosslinked to other molecules or tissue, as well, as physically (such as due to sequestration within) or chemically associated with particles, such as organelles, membranes, surfaces, cells, or subcellular bodies.

The methods of the instant invention relating to the analysis of "insoluble" targets excludes art techniques for the analysis of soluble targets.

As used herein, "fixed" means a biological sample which has been preserved by placing it into formalin, buffered formalin, paraformaldehyde, or equivalent preservation solutions. In some cases the fixed sample will also be placed in paraffin in preparation for cutting with a microtome and may be fixed with any number of agents besides formalin, such as glutaraldehyde, alcohol, etc..

As used herein, "target" refers to a substance whose presence, activity and/or amount is desired to be determined and which has an affinity for a given probe. Targets can be man-made or naturally-occurring substances. Also, they can be employed in their unaltered state or as aggregates with other species. Targets can be attached, covalently or noncovalently, to a binding member, either directly or via a specific binding substance. Examples of targets which can be employed in this invention include, but are not limited to, receptors (on vesicles, lipids, cell membranes or a variety of other receptors); ligands, agonists or antagonists which bind to specific receptors; polyclonal antibodies, monoclonal antibodies and antisera reactive with specific antigenic determinants (such as on viruses, cells or other materials); drugs; nucleic acids or polynucleotides (including mRNA, tRNA, rRNA, oligonucleotides, DNA, viral RNA or DNA, ESTs, cDNA, PCR-amplified products derived from RNA or DNA, miRNA, siRNA, RNAi, and mutations, variants or modifications thereof); proteins (including enzymes, such as those responsible for cleaving neurotransmitters, proteases, kinases and the like); substrates for enzymes; peptides; cofactors; lectins; sugars; polysaccharides; cells (which can include cell surface antigens); cellular membranes; organelles; etc., as well as other such molecules or other substances which can exist in complexed, covalently bonded crosslinked, etc. form. As used herein, the terms nucleic acid, polynucleotide; polynucleic acid and oligonucleotide are interchangeable. Targets can also be referred to as anti-probes.

As used herein, a "probe" is a substance, e.g., a molecule, that can be specifically recognized by a particular target. The types of potential probe/target or target/probe binding partners include receptor/ligand; ligand/antiligand; nucleic acid (polynucleotide) interactions, including DNA/DNA, DNA/RNA, PNA (peptide nucleic acid)/nucleic acid; LNA (locked nucleic acid), enzymes, other catalysts, or other substances, with substrates, small molecules or effector molecules; etc. Examples of probes that are contemplated by this invention include, but are not limited to, organic and inorganic materials or polymers, including metals, chelating agents or other compounds which interact specifically with metals, plastics, agonists and antagonists for cell membrane receptors, toxins and venoms, viral epitopes, hormones (e.g., opioid peptides, steroids, etc.), hormone receptors, lipids (including phospholipids), peptides, enzymes (such as proteases or kinases), enzyme substrates, cofactors, drugs, lectins, sugars, nucleic acids (including oligonucleotides, DNA, RNA, PNA LNA or modified or substituted nucleic acids), oligosaccharides, proteins, enzymes, polyclonal and monoclonal antibodies, single chain antibodies, or fragments thereof. Probe polymers can be linear or cyclic. Probes can distinguish between phosphorylated and non-phosphorylated proteins, either by virtue of differential activity or differential binding. Probes such as lectins can distinguish among glycosylated proteins. As used herein, the terms nucleic acid, polynucleotide, polynucleic acid and oligonucleotide are interchangeable. Any of the substances described above as "probes" can also serve as "targets," and vice-versa.

In addition, other agents can be used to aid in the disruption of the tissue to permit access of the probes to the target molecules, such as methods used for immunohistochemistry or for performing functional or enzyme assays in fixed tissue, or for instance use of proteinase k or collagenase, or saponin or detergents or use of specific buffers to "renature" the target molecules within the tissue.

Any compatible surface can be used in conjunction with this invention. The surface (usually a solid) can be any of a variety of organic or inorganic materials or combinations thereof, including, merely by way of example, plastics such as polypropylene or polystyrene; ceramic; silicon; (fused) silica, quartz or glass, which can have the thickness of, for example, a glass microscope slide or a glass cover slip; paper, such as filter paper; diazotized cellulose; nitrocellulose filters; nylon membrane; or polyacrylamide or other type of gel pad, e.g., an aeropad or aerobead, made of an aerogel, which is, e.g., a highly porous solid, including a film, which is prepared by drying of a wet gel by any of a variety of routine, conventional methods. Substrates that are transparent to light are useful when the method of performing an assay involves optical detection. The surface can be of any thickness or opacity which is compatible with, e.g., conventional methods of detection. For example, the surface can be a thick bottom, clearplate, or an opaque plate. In a preferred embodiment, the surface is the plastic surface of a multiwell, e.g., tissue culture dish, for example a 24-, 96-, 256-, 384-, 864- or 1536-well plate (e.g., a modified plate such as a Corning Costar DNA Bind plate). Anchors can be associated, e.g., bound, directly with a surface, or can be associated with one type of surface, e.g., glass, which in turn is placed in contact with a second surface, e.g., within a plastic "well" in a microtiter dish. The shape of the surface is not critical. It can, for example, be a flat surface such as a square, rectangle, or circle; a curved surface; or a three dimensional surface such as a bead, particle, strand, precipitate, tube, sphere; etc or filters, beads, within capillaries, or microfluidic surfaces, or nanoparticles.

The surface comprises a single region (such as commonly used for high density arrays) or multiple regions which are spatially discrete and addressable or identifiable. Each region comprises a set of anchors or a set of capture probes (as commonly used in arrays or for capturing molecules onto surfaces). How the regions are separated, their physical characteristics, and their relative orientation to one another are not critical. In one embodiment, the regions can be separated from one another by any physical barrier which is resistant to the passage of liquids. For example, in a preferred embodiment, the regions can be wells of a multiwell (e.g., tissue culture) dish, for example a 24-, 96-, 256-, 384-, 864- or 1536-well plate. Alternatively, a surface such as a glass surface can be etched out to have, for example, 864 or 1536 discrete, shallow wells. Alternatively, a surface can comprise regions with no separations or wells, for example a flat surface, e.g., piece of plastic, glass or paper, and individual regions can further be defined by overlaying a structure (e.g,. a piece of plastic or glass) which delineates the separate regions. Optionally, a surface can already comprise one or more arrays of anchors, or anchors associated with linkers, before the individual regions are delineated. In another embodiment, arrays of anchors within each region can be separated from one another by blank spaces on the surface in which there are no anchors, or by chemical boundaries, such as wax or silicones, to prevent spreading of droplets.

In yet another embodiment, the regions can be defined as tubes or fluid control channels, e.g., designed for flow-through assays, as disclosed, for example, in Beattie et al (1995). Clin. Chem. 4, 700-706. Tubes can be of any size, e.g., capillaries or wider bore tubes; can allow the flow of liquids; or can be partially or completely filled with a gel, e.g., agarose or polyacrylamide, through which compounds can be transported (passed through, flowed through, pumped through), e.g., by electrophoresis; or with a space filling matrix of channels, e.g., of linear channels, as described, e.g., in Albota et al. (1998). Science 281, 1653-1656; Cumpston et al. (1998). Mat. Res. Soc. Symp. Proc. 488, 217-225; and/or Cumpston et al. (1999). Nature 398, 51-54. In such a space-filling matrix, liquid and/or molecules therein can not only follow in direction perpendicular to the wall of the tube, but can also diffuse laterally. In a preferred embodiment, a tube is filled with a gel or space-filling matrix; the gel or space-filling matrix is activated for the binding of anchors, and different anchors are passed through sequentially, allowing the formation of a an array (e.g., a linear array) of anchors within the gel; and linkers, targets, etc. are passed through in succession. The array may be linear, 2- or 3-dimensional.

A plurality of assays can be performed in a single tube. For example, a single array of anchors, or of anchors in association with linkers, in a tube can be re-used (e.g., stripped and re-used, or reprogrammed) in sequential assays with the same or different samples. In another embodiment, a plurality of tubes is used in a single assay, e.g., a sample of interest is analyzed in a plurality of tubes containing different arrays. The anchors and anchor/linker associations in the tubes can be any of the types described elsewhere herein.

Regions within or on, etc. a surface can also be defined by modification of the surface itself. For example, a plastic surface can comprise portions made of modified or derivatized plastic, which can serve, e.g., as sites for the addition of specific types of polymers (e.g., PEG can be attached to a polystyrene surface and then derivatized with carboxyl or amino groups, double bonds, aldehydes, and the like). Alternatively, a plastic surface can comprise molded structures such as protrusions or bumps, which can serve as platforms for the addition of anchors. In another embodiment, regions can be gel pads, e.g., polyacrylamide gel pads or aeropads, which are arrayed in a desired pattern on a surface such as, e.g., glass, or are sandwiched between two surfaces, such as, e.g., glass and a quartz plate. Anchors, linkers, etc. can be immobilized on the surface of such pads, or can be imbedded within them. A variety of other arrangements of gel pads on surfaces will be evident to one of skill in the art, and can be produced by routine, conventional methods. The relative orientation of the test regions can take any of a variety of forms including, but not limited to, parallel or perpendicular arrays within a square or rectangular or other surface, radially extending arrays within a circular or other surface, or linear arrays, etc.

The spatially discrete regions of the invention are present in one or multiple copies. That is, there are at least one, two, preferably at least twenty, or at least about 24, 50, 96, 256, 384, 864, 1536, 2025, or more, etc., substantially identical, spatially discrete (separated) regions. Increasing numbers of repeated regions can allow for assays of increasingly higher throughput. Substantially identical regions, as used herein, refers to regions which contain identical or substantially identical arrays of anchors and/or anchor/linker complexes. Substantially identical, as used herein, means that an array or region is intended to serve essentially the same function as another array or region in the context of analyzing a target in accordance with this invention. Differences not essentially affecting function, i.e., detectability of targets, are along the line of small nucleotide imperfections (omissions/inserts/substitutions) or oligo imperfections (poor surface binding), etc., which do not within assay accuracy significantly affect target determination results.

Of course, one of skill in the art will recognize that a single region is useful when only one sample is to be tested, or a single combined sample is tested, or when the arrays are large, and that not all of the regions on a surface need to be substantially identical to one another. For example, if two different sets of arrays are to be tested in parallel, it might be advantageous to include both sets of arrays on a single surface. For example, the two different sets of arrays can be arranged in alternating striped patterns, to facilitate comparison between them. In another embodiment, the practitioner may wish to include regions which can be detected in a distinguishable manner from the other regions on the surface and can thereby be used as a "registration region(s)." For example, a registration region can comprise oligonucleotides or peptides which display a distinctive pattern of fluorescent molecules that can be recognized by a scanning detection device as a "starting point" for aligning the locations of the regions on a surface.

The size and physical spacing of the test regions are not limiting. Typical regions are of an area of about 1 to about 700 mm², preferably 1 to about 40 mm², and are spaced about 0.5 to about 5 mm apart, and are routinely selected depending on the areas involved. In a preferred embodiment, the regions are spaced approximately 5 mm apart. For example, each region could comprise a rectangular grid, with, for example, 8 rows and 6 columns, of roughly circular spots of anchors which are about 100 micrometers in diameter and 500 micrometers apart; such a region would cover about a 20 millimeter square area. Larger and smaller region areas and spacings are included.

The regions can also be further subdivided such that some or all anchors within a region are physically separated from neighboring anchors by means, e.g., of an indentation or dimple. For example, the number of subdivisions (subregions) in a region can range from about 10 to about 100 or more or less. In one embodiment, a region which is a well of a 1536-well dish can be further subdivided into smaller wells, e.g., about 4 to about 900, preferably about 16 to about 36 wells, thereby forming an array of wells-within-wells. Such a dimpled surface reduces the tolerance required for physically placing a single anchor (or group of anchors) into each designated space (locus), and the size of the areas containing anchors is more uniform, thereby facilitating the detection of targets which bind to the probe.

The term "anchor" as used herein refers to any entity or substance, e.g., molecule, which is associated with (e.g., immobilized on, or attached either covalently or non-covalently to) the surface, or which is a portion of such surface (e.g., derivatized portion of a plastic surface), and which can undergo specific interaction or association with a linker or other substance as described herein. The portion of an anchor which associates with, e.g., a linker molecule, can be associated with the surface directly, or the anchor can comprise an intermediate "spacer" moiety. Such a spacer can be of any material, e.g., any of a variety of materials which are conventional in the art. In one embodiment, the spacer is a linear carbon molecule having, e.g., about 5-20 Cs, preferably about 12 Cs. In another embodiment, the spacer is a nucleic acid (of any of the types describes elsewhere herein) which does not undergo specific interaction or association with, e.g., a linker molecule.

The term "anchor" as used herein can also refer to a group of substantially identical anchors. The location of each group of anchors is termed herein a "locus." As is well known in the art, the number of individual anchor molecules present at a locus is limited only by physical constraints introduced by, e.g., the size of the anchors. For example, a locus which is, e.g., about 25-200 µm in diameter, can comprise millions of anchors.

As used herein, an "anchor/linker complex" exists when an anchor and a linker have combined through molecular association in a specific manner. The interaction with the linker can be either irreversible, such as via certain covalent bonds, or reversible, such as via nucleic acid hybridization.

In a preferred embodiment, the anchor is a nucleic acid, which can be of any length (e.g., an oligonucleotide) or type (e.g., DNA, RNA, PNA, LNA or a PCR product of an RNA or DNA molecule). The nucleic acid can be modified or substituted (e.g., comprising non naturally occurring nucleotides such as, e.g., inosine; joined via various known linkages such as sulfamate, sulfamide, phosphorothionate, methylphosphonate, carbamate, etc.; or a semisynthetic molecule such as a DNA-streptavidin conjugate, etc.). Single stranded nucleic acids are preferred.

A nucleic acid anchor can be of any length which is compatible with the invention. For example, the anchor can be an oligonucleotide, ranging from about 8 to about 50 nucleotides in length, preferably about 10, 15, 20, 25 or 30 nucleotides. In another embodiment, the anchor can be as long as about 50 to about 300 nucleotides in length, or longer or shorter, preferably about 200 to about 250 nucleotides. For example, an anchor can comprise about 150 to about 200 nucleotides of "spacer" nucleic acid, as described above, and, adjacent to the spacer, a shorter sequence of, e.g., about 10, 15, 20, 25 or 30 nucleotides which is designed to interact with a linker molecule ("linker-specific sequence"). Such spacers can be of any length or type of nucleic acid, and can have any base composition which is functional in the invention. In a preferred embodiment, the spacers of each of the anchors at a locus, and/or of the anchors in different loci within a region, are substantially identical; the anchors thus differ from one another primarily with regard to their linker-specific sequences.

Spacers can impart advantages to anchors, allowing for improved performance. For example, the linker-specific portions of such an anchor lie further away from the surface, and therefore are less physically constrained and subject to less steric hindrance, than if they were closer to the surface. This facilitates, for example, the association of a plurality of different linkers (e.g., about 2 to about 100), having different target specificities, with the anchors at a given locus. As is discussed in more detail below, an individual anchor can comprises (in addition to a spacer) a plurality of linker-specific sequences which are arranged, e.g., in a tandem linear fashion; this allows for the association of a plurality of different types of linkers with at least one such anchor at a given locus. Also discussed in more detail below is another way in which a plurality of different types of linkers can be associated with the anchors at a given locus: at a "mixed locus," two or more anchors are each associated with a different linker, having a different target specificity. Because of the physical flexibility of anchors comprising spacers, the anchors at a given locus can readily bind to a plurality of different linker molecules without being physically constrained by adjacent anchor molecules. An advantage of binding a plurality of linker molecules to the anchors at a given locus is that it allows for the detection of an increased number of targets at a particular locus. In one embodiment, the plurality of linkers bound at a given locus have probes which are specific for different portions of the same target nucleic acid of interest (e.g., to different oligonucleotide sequences within the nucleic acid). This allows for amplified detection of the target compared to detection with a single probe. In another embodiment, the plurality of linkers have probes which are specific for different, e.g. unrelated, targets. This allows for the detection of a plurality of different targets within a particular locus. A further advantage of anchors comprising spacers is that they can more readily accommodate linkers which are associated with relatively large molecules such as, e.g., proteins, and/or which bind to relatively large targets such as, e.g., proteins, membranes or cells.

The base composition of a nucleic acid anchor is not necessarily constrained. Any base composition of the anchors is acceptable, provided that the anchors are functional for the purpose of the invention. For example, single stranded nucleic acid anchors at a locus, or at different loci in a region, can comprise partially or completely random sequences (e.g., randomly generated sequences, for example with no restrictions on the relative amounts of A, G, T and/or C). In one embodiment, the anchors are not "sequence isomers" (e.g., "random sequence isomers"), i.e., oligonucleotides having identical amounts of G, C, A and T, but arranged in different relative orders. That is, the anchors in, for example, the different loci of a region do not conform to the equation Gn Cn Am Tm, where n and m are integers. See, e.g., the anchors shown in Fig. 1, which are not random sequence isomers. In the anchors of the invention, the amounts of G and C do not need to be approximately the same, nor do the relative amounts of A and T. Furthermore, the net relative amounts of G, C, A and T are not necessarily constrained. For example, the base composition of the anchors in a region can range from being relatively GC rich (i.e., greater than 50% G+C), to having equal amounts of G, C, A and T, to being relatively AT rich (i.e., greater than 50% A+T). In one embodiment, the anchors are randomly generated, e.g., in a manner such that no constraints are placed on the relative amounts of G, C, A and T.

Anchors comprising a nucleic acid spacer and one or more linker-specific portions are unlikely to conform to any particular constraints on base compostion. For example, if the anchors located at different loci in a region have spacers which are substantially identical, e.g., a substantially identical 25-mer or a 200-mer, but each anchor has a different linker-specific moiety (e.g., a 25-mer), even if the linker-specific moieties meet specific requirements (e.g., the number of As and Gs are approximately equal; the number of Ts and Cs are approximately equal; the oligo conforms to the equation Gn Cn Am Tm; and/or that the G+C content meets a particular requirement), the anchors as a whole will not meet those particular requirements. Similarly, even if the linker-specific moieties of anchors at different loci in a region are substantially different from one another (e.g., each linker-specific moiety has a sequence which differs by at least about 20%, or 50%, or 80% from each other linker-specific moiety in the region), the net sequence identities of the anchors, considering the entire length of the nucleic acid, may be far less. For example, if each of the anchors comprises a substantially identical 250-mer spacer, and a 25-mer linker-specific moiety which is 100% different from every other linker-specific moiety in the region, the anchors will still differ from one another by only 10%.

An anchor can also be a peptide or a protein. For example, it can be a polyclonal or monoclonal antibody molecule or fragment thereof, or single chain antibody or fragment thereof, which binds specifically to the portion of a linker that is an antigen or an anti-antibody molecule; in the obverse, the anchor can be a peptide, and the portion of the linker which binds to it can be an antibody or the like. In another embodiment, the anchor can be a lectin (such as concanavalin A or agglutinins from organisms such as Limulus, peanut, mung bean, Phaseolus, wheat germ, etc.) which is specific for a particular carbohydrate. In another embodiment, the anchor can comprise an organic molecule, such as a modified or derivatized plastic polymer which can serve, e.g., as the stage for specific solid phase chemical synthesis of an oligonucleotide. In this case, the derivatized plastic can be distributed as an array of discrete, derivatized, loci which are formed integrally into the plastic surface of a combination during the manufacturing process. In another embodiment, the anchor can take advantage of specific or preferential binding between metal ions, e.g., Ni, Zn, Ca, Mg, etc. and particular proteins or chelating agents. For example, the anchor can be polyhistidine, and the anchor-specific portion of the linker can be nickel, which is attached via a nickel chelating agent to a target-specific probe. Alternatively, the chelating agent can be the anchor and the polyhistidine the probe-related portion. Alternatively, the anchor can comprise an inorganic substance. For example, it can comprise a metal such as calcium or magnesium, and the anchor-specific portion of the linker can be a preferential chelating agent, such as EDTA or EGTA, respectively, which is attached to a target-specific probe. One of skill in the art will recognize that a wide range of other types of molecules can also serve as anchors, such as those general types also discussed in conjunction with probes and targets.

An anchor can also be a hybrid structure, such as a DNA duplex, or a duplex comprising, e.g., DNA and protein which interact specifically in any of the ways described elsewhere herein. For example, the "base moiety" of a duplex anchor (the portion which is in direct contact with the surface) can comprise an optionally modified single stranded nucleic acid; preferably, the base moiety also comprises a spacer, e.g., a linear carbon spacer as described above. In one embodiment, a second single stranded nucleic acid is associated with (e.g., hybridized to) this base moiety, to form an anchor which comprises at least a partially double stranded (duplex) nucleic acid. For example, the base moiety can comprise a linear carbon spacer which is attached to the surface at one end, and at the other end is attached to a single stranded DNA oligonucleotide of about 10-100 nucleotides, preferably about 25 nucleotides; and the second moiety of the duplex can comprise a sequence which is complementary to at least a portion of the base moiety, (e.g., to the terminal about 40 nucleotides), followed by an optional spacer (e.g., about 5-15, preferably about 10 nucleotides), followed by a linker-specific sequence (e.g., a sequence of about 8 to about 50 nucleotides, preferably about 15, 20, 25 or 30 nucleotides, most preferably about 25 nucleotides in length).

The relative lengths and base compositions of the complementary portions of an anchor duplex and of its linker-specific sequence(s) can be varied to suit the needs of an assay, using optimization procedures which are conventional in the art. For example, sequences can be selected such that linkers can be dissociated from (e.g., melted apart from) duplex anchor molecules under conditions in which the duplex anchors, themselves, remain intact. The remaining arrays of duplex anchors can then be re-used, if desired, to hybridize to the same or different linker molecules. Alternatively, sequences can be selected such that both the anchor/linker hybrids and the two complementary portions of the duplex anchors are dissociated under the same conditions, leaving behind only the base moieties in contact with the surface. In one embodiment, all or substantially all of the base moieties in a particular locus or in all the loci of a region are identical, or substantially identical. The arrays of base moieties remaining after such a dissociation can be re-used (e.g., for hybridization to linker molecules) only if the complementary portions of the duplex anchors are first added back, a process which requires knowledge of the sequence of the base moiety that is involved in duplex formation. The ability to manufacture arrays of anchors which either can or cannot be re-used by a user unfamiliar with the sequence of the base moieties, represents an advantage of employing such hybrid anchors. For example, a manufacturer can prevent unauthorized re-use of its arrays. The prevention of such re-use can also, e.g., forestall problems of degraded performance or unreliability occasioned by excessive use.

In one embodiment, the group of anchors at a given locus within a region are substantially identical (e.g., are specific for the "anchor-specific" portion of one type of linker, or for one target, only). In another embodiment, a plurality of different anchors, having specificities for a plurality of different linkers and/or for a plurality of different targets, can be present at a given locus, called a "mixed locus," e.g., a plurality of about 2 to about 100, for example at least about 2, at least about 4 or at least about 10. An advantage of mixed loci is that they allow for the detection of an increased number of different targets at a particular locus. In one embodiment, each mixed locus contains one anchor which is the same in every, or at least several, loci. For instance, an anchor which is the same in more than one locus can be used for quality assurance and/or control or for signal normalization.

Of course, "mixed loci" are also advantageous for surfaces having only a single (non-repeated) region. The anchors in each of the loci of such a single region can interact with linkers, or directly with targets of interest.

The number of anchors (i.e., groups of anchors at individual loci) in a test region can be at least two, preferably between about 8 and about 900 (more or less being included), more preferably between about 8 and about 300, and most preferably between about 30 and about 100 (e.g., about 64). In some preferred embodiments, there are about 16, 36, 45 or 100 anchors/test region for a surface with 96 test regions (e.g., wells), or about 9, 16 or 25 anchors/test region for a surface with 384 test regions (e.g., wells). In a most preferred embodiment, each anchor in a test region has a different specificity from every other anchor in the array. However, two or more of the anchors can share the same specificity and all of the anchors can be identical. In one embodiment, in which a combination of the invention comprises a very large number of test regions (e.g., about 864, 1536, or more), so that a large number of test samples can be processed at one time, it might of interest to test those samples for only a limited number (e.g., about 2, 4, 6 or 9) of parameters. In other words, for combinations comprising a very large number of regions, it might be advantageous to have only about 2 to 9 anchors per region. In a further preferred embodiment the array may be comprised of molecules that directly capture the target, such as the nuclease protection probe, without use of an anchor/linker complex. Furthermore, arrays can consist of only a few to hundreds of thousands of capture sites, as commonly referred to as high density arrays, and there may only be one such array, or multiple arrays on each surface.

The physical spacing and relative orientation of the anchors (i.e., groups of anchors at individual loci) in or on a test region are not limiting. Typically, the distance between the anchors is about 0.003 to about 5 mm or less, preferably between about 0.03 and about 1. Larger and smaller anchor spacings (and areas) are included. The anchors can be arranged in any orientation relative to one another and to the boundaries of the region. For example, they can be arranged in a two-dimensional orientation, such as a square, rectangular, hexagonal or other array, or a circular array with anchors emanating from the center in radial lines or concentric rings. The anchors can also be arranged in a one-dimensional, linear array. For example, oligonucleotides can be hybridized to specific positions along a DNA or RNA sequence to form a supramolecular array, or in a linear arrangement in a flow through gel, or on the surface of a flow through device or structures within a flow through device. Alternatively, the anchors can be laid down in a "bar-code"-like formation. For example, anchors can be laid down as long lines parallel to one another. The spacing between or the width of each long line can be varied in a regular way to yield a simple, recognizable pattern much like a bar-code, e.g., the first and third lines can be twice as large as the rest, lines can be omitted, etc. An extra empty line can be placed after the last line to demarcate one test region, and the bar code pattern can be repeated in succeeding test regions.

The pattern of anchors does not need to be in strict registry with the positions of the separated assay wells (test regions) or separate assay droplets. The term "assay positions" will be used to refer to the positions of the assay surface where assay samples are applied. (These can be defined by the position of separate droplets of assay sample or by the position of walls or separators defining individual assay wells on a multi-well plate for example.) The anchor pattern itself (e.g., a "bar code"-like pattern of oligonucleotide anchors) is used to define where exactly each separate anchor is positioned by pattern recognition - just as each line of a barcode is recognized by its position relative to the remaining lines. Hence the first anchor need not be at one edge or one corner of each assay position. The first anchor will be found by pattern recognition, rather than position relative to the assay position. As long as the area used by each assay position (the area of the droplet or the area of the well for example) is large enough to be certain to contain at least one whole unit of the repeating pattern of anchors, then each assay point will test the sample for that assay position for all of the targets specified by the (bar-coded) pattern wherever the pattern lies within the area of the assay position.

The anchors do not need to be arranged in a strict or even fixed pattern within each test region. For example, each anchor can be attached to a particle, bead, or the like, which assumes a random position within a test region. The location of each anchor can be determined by the use, e.g., of a detectable tag. For example, the linker molecule specific for each type of anchor can be labeled with a different fluorescent, luminescent etc. tag, and the position of a particle comprising a particular linker/anchor pair can be identified by the nature of the signal emanating from the linker, e.g., the excitation or emission spectrum. One skilled in the art can prepare a set of linkers with a variety of such attached tags, each with a distinguishable spectrum. Alternatively, the anchors can be labeled directly. For example, each type of anchor can be labeled with a tag which fluoresces with a different spectrum from the tags on other types of anchors. Alternatively, the particles, beads or the like can be different from one another in size or shape or color or florescence or by emission of a signal. Any of the labeling and detection methods described herein can be employed. For example, fluorescence can be measured by a CCD-based imaging system, by a scanning fluorescence microscope or Fluorescence Activated Cell Sorter (FACS).

An anchor can interact or become associated specifically with one portion - the anchor-specific portion - of a linker molecule. By the terms "interact" or "associate", it is meant herein that two substances or compounds (e.g., anchor and anchor-specific portion of a linker, a probe and its target, or a target and a target-specific reporter) are bound (e.g., attached, bound, hybridized, joined, annealed, covalently linked, or otherwise associated) to one another sufficiently that the intended assay can be conducted. By the terms "specific" or "specifically", it is meant herein that two components (e.g., anchor and anchor-specific region of a linker, a probe and its target, or a target and a target-specific reporter) bind selectively to each other and, in the absence of any protection technique, not generally to other components unintended for binding to the subject components. The parameters required to achieve specific interactions can be determined routinely, e.g., using conventional methods in the art.

For nucleic acids, for example, one of skill in the art can determine experimentally the features (such as length, base composition, and degree of complementarity) that will enable a nucleic acid (e.g., an oligonucleotide anchor) to hybridize to another nucleic acid (e.g., the anchor-specific portion of a linker) under conditions of selected stringency, while minimizing non-specific hybridization to other substances or molecules (e.g., other oligonucleotide linkers). Typically, the DNA or other nucleic acid sequence of an anchor, a portion of a linker, or a detector oligonucleotide will have sufficient complementarity to its binding partner to enable it to hybridize under selected stringent hybridization conditions, and the Tm will be about 10 to 20°C above room temperature (e.g., about 37ºC). In general, an oligonucleotide anchor can range from about 8 to about 50 nucleotides in length, preferably about 15, 20, 25 or 30 nucleotides. As used herein, "high stringent hybridization conditions" means any conditions in which hybridization will occur when there is at least 95%, preferably about 97 to 100%, nucleotide complementarity (identity) between the nucleic acids. However, depending on the desired purpose, hybridization conditions can be selected which require less complementarity, e.g., about 90%, 85%, 75%, 50%, etc. Among the hybridization reaction parameters which can be varied are salt concentration, buffer, pH, temperature, time of incubation, amount and type of denaturant such as formamide, etc. (see, e.g., Sambrook et al. (1989). Molecular Cloning: A Laboratory Manual (2d ed.) Vols. 1-3, Cold Spring Harbor Press, New York; Hames et al. (1985). Nucleic Acid Hybridization, IL Press; Davis et al. (1986), Basic Methods in Molecular Biology, Elsevir Sciences Publishing, Inc., New York). For example, nucleic acid (e.g., linker oligonucleotides) can be added to a test region (e.g., a well of a multiwell plate - in a preferred embodiment, a 96 or 384 or greater well plate), in a volume ranging from about 0.1 to about 100 or more µl (in a preferred embodiment, about 1 to about 50 µl, most preferably about 40 µl), at a concentration ranging from about 0.01 to about 5 µM (in a preferred embodiment, about 0.1 µM), in a buffer such as, for example, 6X SSPE-T (0.9 M NaCl, 60mM NaH2PO4, 6 mM EDTA and 0.05% Triton X-100), and hybridized to a binding partner (e.g., an oligonucleotide anchor on the surface) for between about 10 minutes and about at least 3 hours (in a preferred embodiment, at least about 15 minutes) at a temperature ranging from about 4ºC. to about 37ºC. (in a preferred embodiment, at about room temperature). Conditions can be chosen to allow high throughput. In one embodiment of the invention, the reaction conditions can approximate physiological conditions.

The design of other types of substances or molecules (e.g., polypeptides, lectins, etc.) which can, e.g., serve as anchors or as portions of linkers, and the reaction conditions required to achieve specific interactions with their binding partners, are routine and conventional in the art (e.g., as described in Niemeyer et al (1994). Nucl. Acids Res. 22, 5530-5539; Fodor et al (1996). U.S. Patent No. 5,510,270; Pirrung et al (1992), U.S. Patent No. 5,143,854). Among the incubation parameters are buffer, salt concentration, pH, temperature, time of incubation, presence of carrier and/or agents or conditions to reduce non-specific interactions, etc. For example, to a test region (e.g., a well of a multiwell plate - in a preferred embodiment, a 96 or 384 or greater well plate) which contains, as anchors, antibodies, can be added anti-antibodies (e.g., antigens or antibody-specific secondary antibodies) in a volume ranging from about 0.1 to about 100 or more µl (in a preferred embodiment, about 1 to about 50 µl, most preferably about 40 µl), at a concentration ranging from about 10 pM to about 10 nM (in a preferred embodiment, about 1 nM), in a buffer such as, for example, 6X SSPE-T, PBS or physiological saline, and incubated with the anchors on the surface for between about 10 minutes and at least about 3 hours (in a preferred embodiment, at least about 15 minutes), at a temperature ranging from about 4ºC to about 45ºC. (in a preferred embodiment, about 4ºC.). For peptide anchors, a length of about 5 to about 20 amino acids is preferred.

In some embodiments of the invention, each anchor in an array can interact with the anchor-specific portion of its corresponding linker to substantially the same degree as do the other anchors in the array, under selected reaction conditions. This can insure that the anchors specify a substantially uniform array of linkers and, therefore, probes.

The anchors (i.e., groups of anchors at individual loci) within a test region can be a "generic" set, each anchor of which can interact with one or more of a variety of different linkers, each having a portion specific to such anchor but with differing "probe" portions; thus, a single array of generic anchors can be used to program or define a varied set of probes. The flexible nature of such a generic assay of anchors can be illustrated with reference to Figure 1. Fig. 1 schematically illustrates one of these (oligonucleotide) anchors, anchor 1, which is in contact with linker 1, which comprises one portion that is specific for anchor 1 and a second portion that is specific for target mRNA 1. Alternatively, one could substitute, e.g., a linker 2, which, like linker 1, comprises a portion that is specific for anchor 1, but which comprises a second portion that is specific for target mRNA 2 instead of target mRNA 1. Thus, anchor 1 can be used to specify (or program, or define, or determine) probes for either of two or more different target mRNAs. The process of generating and attaching a high resolution pattern (array) of oligonucleotides or peptides can be expensive, time-consuming and/or physically difficult. The ability to use a pre-formed array of anchors to program a wide variety of probe arrays is one advantage of this invention.

Although the generic anchors illustrated in Fig. 1 define a pattern of oligonucleotide probes, the identical anchor array could also be used to program an array of other probes, for example receptor proteins. Clearly, many permutations are possible, given the range of types of anchor/linker interactions, e.g., even more complex layers of "sandwiched" or "piggybacked" probes such as protein/antibody combinations. For example, in one embodiment a generic set of anchors can be associated (covalently or non-covalently) with a set of linkers to form a modified array of "conjugated" anchors, as is described in more detail below. Thus, the surface of anchors per this invention, itself, offers novel advantages.

In one embodiment of the invention, anchors can interact reversibly with linkers; thus, a generic set of anchors can be re-used to program a varied set of probes. For example, an oligonucleotide anchor can be separated from the oligonucleotide portion of a linker by, for example, a heating step that causes the two oligonucleotides to dissociate, and can then be rebound to a second linker. The ability to re-use anchor arrays, which can be expensive, time-consuming and/or physically difficult to make, is another advantage of the invention.

An anchor does not necessarily have to interact with a linker. For example, an anchor can be coupled (directly or indirectly) to a detectable molecule, such as a fluorochrome, and can thereby serve to localize a spot within a grid, e.g., for purpose of registration between the test surface and the detector. Alternatively, an anchor can be labeled with a known amount of a detectable molecule so as to serve as internal quantitation marker, e.g., for purposes of calibration.

The term "linker" as used herein refers to a bifunctional substance which comprises a first portion (or moiety or part) that is specific for a chosen (designated) anchor or subset of the anchors ("anchor-specific") and a second portion that comprises a probe which is specific for a target of interest ("target-specific"). The two portions of the linker can be attached via covalent or noncovalent linkages, and can be attached directly or through an intermediate (e.g., a spacer).

The chemical nature of the anchor-specific portion of the linker is, of course, a function of the anchor or anchors with which it interacts. For example, if the anchor is an oligonucleotide, the portion of the linker which interacts with it can be, for example, a peptide which binds specifically to the oligonucleotide,or a nucleic acid which can hybridize efficiently and specifically to it under selected stringent hybridization conditions. The nucleic acid can be, e.g., an oligonucleotide, DNA, RNA, PNA, PCR product, or substituted or modified nucleic acid (e.g., comprising non naturally-occurring nucleotides such as, e.g., inosine; joined via various known linkages such as sulfamate, sulfamide, phosphorothionate, methylphosphonate, carbamate; or a semisynthetic molecule such as a DNA-streptavidin conjugate, etc.). Single strand moieties are preferred. The portion of a linker which is specific for an oligonucleotide anchor can range from about 8 to about 50 nucleotides in length, preferably about 15, 20, 25 or 30 nucleotides. If the anchor is an antibody, the portion of the linker which interacts with it can be, e.g., an anti-antibody, an antigen, or a smaller fragment of one of those molecules, which can interact specifically with the anchor. Substances or molecules which interact specifically with the other types of anchors described above, and which can serve as the anchor-specific portion of a linker, are well-known in the art and can be designed using conventional procedures (e.g., see above).

The chemical nature of the target-specific portion of the linker is, of course, a function of the target for which it is a probe and with which it interacts. For example, if the target is a particular mRNA, the target-specific portion of the linker can be, e.g., an oligonucleotide which binds specifically to the target but not to interfering RNAs or DNAs, under selected hybridization conditions. One of skill in the art can, using art-recognized methods, determine experimentally the features of an oligonucleotide that will hybridize optimally to the target, with minimal hybridization to non-specific, interfering DNA or RNA (e.g., see above). In general, the length of an oligonucleotide probe used to distinguish a target mRNA present in a background of a large excess of untargeted RNAs can range from about 8 to about 50 nucleotides in length, preferably about 18, 20, 22 or 25 nucleotides. An oligonucleotide probe for use in a biochemical assay in which there is not a large background of competing targets can be shorter. Using art-recognized procedures (e.g., the computer program BLAST), the sequences of oligonucleotide probes can be selected such that they are mutually unrelated and are dissimilar from potentially interfering sequences in known genetics databases. The selection of hybridization conditions that will allow specific hybridization of an oligonucleotide probe to an RNA can be determined routinely, using art-recognized procedures (e.g., see above). For example, target RNA [e.g., total RNA or mRNA extracted from tissues or cells grown (and optionally treated with an agent of interest) in any vessel, such as the well of a multiwell microtiter plate (e.g., 96 or 384 or more wells)] can be added to a test region containing a oligonucleotide probe array (see above) in a buffer such as 6X SSPE-T or others, optionally containing an agent to reduce non-specific binding (e.g., about 0.5 mg/ml degraded herring or salmon sperm DNA, or yeast RNA), and incubated at an empirically determined temperature for a period ranging from between about 10 minutes and at least 18 hours (in a preferred embodiment, about 3 hours). The stringency of the hybridization can be the same as, or less than, the stringency employed to associate the anchors with the anchor-specific portion of the linkers. The design and use of other types of probes are also routine in the art, e.g., as discussed above.

In one embodiment, all, or substantially all, of the linkers associated with the anchors at a given locus contain an identical (or substantially identical) probe, which is specific for a single, specific target of interest. In another embodiment, one or more of the linkers associated with the anchors at a given locus comprises a plurality of different probes, and thus is specific for a plurality of different targets. These probes can be situated in the linker as part of a branched structure, or preferably, can be aligned in a linear relationship; and they can be of the same material (e.g., are all nucleic acid or are all peptide sequences), or combinations of various materials. In effect, having multiple probes on each linker increases the number of targets which can be detected at a particular locus. In one embodiment, a plurality of probes on given linker are all specific for a particular target of interest (e.g., they are specific for different portions of a single mRNA of interest, or are specific for nuclease protection fragments corresponding to different portions of that mRNA); this allows for increased sensitivity of an assay for the target, e.g., a target which is present in a sample at low abundance. The number of probes on a linker can be, e.g., about 2- 50, preferably about 2, 4 or 10.

Of course, linkers which comprise such a plurality of different probes are also advantageous for use with surfaces that contain only a single (non-repeated) region.

The anchor-specific and the target-specific portions of a linker can be joined (attached, linked) by any of a variety of covalent or non-covalent linkages, the nature of which is not essential to the invention. The two portions can be joined directly or through an intermediate molecule. In one embodiment, in which both portions of the linker are oligonucleotides, they can be joined by covalent linkages such as phosphodiester bonds to form a single, colinear nucleic acid. In another embodiment, in which the anchor-specific portion is an oligonucleotide and the target-specific portion is a receptor, for example a receptor protein, the two portions can be joined via the interaction of biotin and streptavidin molecules. Many variations of such linkages are known (e.g., see Niemeyer et al (1994). NAR 22, 5530-5539). Alternatively, the two portions can be joined directly, e.g., an oligonucleotide can be amidated and then linked directly (e.g., crosslinked) to a peptide or protein via an amide bond, or joined to a membrane component via an amide bond or a lipid attachment. Methods to form such covalent or noncovalent bonds are conventional and are readily optimized by one of skill in the art. Spacer sequences (e.g., nucleic acid) can also be present between the anchor-specific and target-specific portions of a linker.

After two substances are associated (e.g., by incubation of two nucleic acids, two proteins, a protein plus a nucleic acid, or others) to form a complex (such as, e.g., an anchor/linker complex), the resulting complex can be optionally treated (e.g., washed) to remove unbound substances (e.g., linkers), using conditions which are determined empirically to leave specific interactions intact, but to remove non-specifically bound material. For example, reaction mixtures can be washed between about one and ten times or more under the same or somewhat more stringent conditions than those used to achieve the complex (e.g., anchor/linker complex).

One of skill in the art will recognize that a variety of types of sandwiches of anchors and linkers can be generated. For example, to an array of anchors (e.g., anchors having substantially identical sequences), one can attach a first set of linkers, each of which has a first moiety that is specific for the anchor and a second moiety that is specific for one of a second set of linkers, and so forth. In effect, this second layer of a sandwich allows one to convert a first set of anchors (e.g., identical oligonucleotides) to a different array having a different set of specificities, of "conjugated" anchors. The various sets of linkers and anchors can be associated to one another covalently or non-covalently, as desired.

The combinations of this invention can be manufactured routinely, using conventional technology.

Some of the surfaces which can be used in the invention are readily available from commercial suppliers. In a preferred embodiment, the surface is a 96- , 384- or 1536-well microtiter plate such as modified plates sold by Corning Costar. Alternatively, a surface comprising wells which, in turn, comprise indentations or "dimples" can be formed by micromachining a substance such as aluminum or steel to prepare a mold, then microinjecting plastic or a similar material into the mold to form a structure. Alternatively, a structure comprised of glass , plastic, ceramic, or the like, can be assembled. The separator can be, for example, a piece of material, e.g., silicone, with holes spaced throughout, so that each hole will form the walls of a test well when the three pieces are joined. The subdivider can be, for example, a thin piece of material, e.g., silicone, shaped in the form of a screen or fine meshwork. The base can be a flat piece of material, e.g., glass, in, for example, the shape of the lower portion of a typical microplate used for a biochemical assay. The top surface of the base can be flat, or can be formed with indentations that will align with the subdivider shape to provide full subdivisions, or wells, within each sample well. The three pieces can be joined by standard procedures, for example the procedures used in the assembly of silicon wafers.

Oligonucleotide anchors, linker moieties, or detectors can be synthesized by conventional technology, e.g., with a commercial oligonucleotide synthesizer and/or by ligating together subfragments that have been so synthesized. Nucleic acids which are too long to be comfortably synthesized by such methods can be generated by amplification procedures, e.g., PCR, using conventional procedures. In one embodiment of the invention, preformed nucleic acid anchors, such as oligonucleotide anchors, can be situated on or within the surface of a test region by any of a variety of conventional techniques, including photolithographic or silkscreen chemical attachment, disposition by ink jet technology, capillary, screen or fluid channel chip, electrochemical patterning using electrode arrays, contacting with a pin or quill, or denaturation followed by baking or UV-irradiating onto filters (see, e.g., Rava et al (1996). U.S. Patent No. 5,545,531; Fodor et al (1996). U.S. Patent No. 5,510,270; Zanzucchi et al (1997). U.S. Patent No. 5,643,738; Brennan (1995). U.S. Patent No. 5,474,796; PCT WO 92/10092; PCT WO 90/15070). Anchors can be placed on top of the surface of a test region or can be, for example in the case of a polyacrylamide gel pad, imbedded within the surface in such a manner that some of the anchor protrudes from the surface and is available for interactions with the linker. In a preferred embodiment, preformed oligonucleotide anchors are derivatized at the 5' end with a free amino group; dissolved at a concentration routinely determined empirically (e.g., about 1 µM) in a buffer such as 50 mM phosphate buffer, pH 8.5 and 1 mM EDTA; and distributed with a Pixus nanojet dispenser (Cartesian Technologies) in droplets of about 10.4 nanoliters onto specific locations within a test well whose upper surface is that of a fresh, dry DNA Bind plate (Corning Costar). Depending on the relative rate of oligonucleotide attachment and evaporation, it may be required to control the humidity in the wells during preparation. In another embodiment, oligonucleotide anchors can be synthesized directly on the surface of a test region, using conventional methods such as, e.g., light-activated deprotection of growing oligonucleotide chains (e.g., in conjunction with the use of a site directing "mask") or by patterned dispensing of nanoliter droplets of deactivating compound using a nanojet dispenser. Deprotection of all growing sequences that are to receive a single nucleotide can be done, for example, and the nucleotide then added across the surface. In another embodiment, oligonucleotide anchors are attached to the surface via the 3' ends of the oligonucleotides, using conventional methodology.

Peptides, proteins, lectins, chelation embodiments, plastics and other types of anchors or linker moieties can also be routinely generated, and anchors can be situated on or within surfaces, using appropriate available technology (see, e.g., Fodor et al (1996). U.S. Patent No. 5,510,270; Pirrung et al (1992). U.S. Patent No. 5,143,854; Zanzucchi et al (1997). U.S. Patent No. 5,643,738; Lowe et al (1985). U.S. Patent No. 4,562,157; Niemeyer et al (1994). NAR 22, 5530-5539).

In some embodiments of the invention, the disclosed combinations are used in a variety of screening procedures and/or to obtain information about the level, activity or structure of the probes or target molecules. Such assays are termed Multi Array Plate Screen (MAPS) methods or assays, and the surfaces comprising arrays of anchors or anchors plus probes which are used for the assays are termed MAPS arrays or MAPS plates.

The components of a reaction mixture, assay, or screening procedure can be assembled in any order. For example, the anchors, linkers and targets can be assembled sequentially; or targets and linkers, in the presence or absence of reporters, can be assembled in solution and then contacted with the anchors.

One embodiment of the invention relates to a method of detecting at least one target, comprising
contacting a sample which may comprise said target(s) with a bifunctional linker which has a first portion that is specific for an oligonucleotide anchor and a second portion that comprises a probe which is specific for said target(s), under conditions effective to obtain a first hybridization product between said target(s) and said linker,
contacting said first hybridization product with a combination under conditions effective to obtain a second hybridization product between said first hybridization product and said combination, wherein said combination comprises, before the addition of said first hybridization product,
a surface comprising multiple spatially discrete regions, at least two of which are substantially identical, each region comprising
at least 8 different oligonucleotide anchors,
contacting said first hybridization product or said second hybridization product with a labeled detector probe, and
detecting said detection probe.

Each of the assays or procedures described below can be performed in a high throughput manner, in which a large number of samples (e.g., as many as about 864, 1036, 1536, 2025 or more, depending on the number of regions in the combination) are assayed on each plate or surface rapidly and concurrently. Further, many plates or surfaces can be processed at one time. For example, in methods of drug discovery, a large number of samples, each comprising a drug candidate (e.g., a member of a combinatorial chemistry library, such as variants of small molecules, peptides, oligonucleotides, or other substances), can be added to separate regions of a combination as described or can be added to biological or biochemical samples that are then added to separate regions of a combination, and incubated with probe arrays located in the regions; and assays can be performed on each of the samples. With the recent advent and continuing development of high-density microplates, DNA spotting tools and of methods such as laser technology to generate and collect data from even denser microplates, robotics, improved dispensers, sophisticated detection systems and data-management software, the methods of this invention can be used to screen or analyze thousands or tens of thousands or more of compounds per day.

For example, in embodiments in which the probes are oligonucleotides, the assay can be a diagnostic nucleic acid or polynucleotide screen (e.g., a binding or other assay) of a large number of samples for the presence of genetic variations or defects (e.g., polymorphisms or specific mutations associated with diseases such as cystic fibrosis. See, e.g., litia et al (1992). Molecular and Cellular Probes 6, 505-512) ); pathogenic organisms (such as bacteria, viruses, and protozoa, whose hosts are animals, including humans, or plants), or mRNA transcription patterns which are diagnostic of particular physiological states or diseases. Nucleic acid probe arrays comprising portions of ESTs (including full-length copies) can be used to evaluate transcription patterns produced by cells from which the ESTs were derived (or others). Nucleic acid probes can also detect peptides, proteins, or protein domains which bind specifically to particular nucleic acid sequences (and vice-versa).

Similarly, in embodiments in which the probes are antigen-binding molecules (e.g., antibodies), the assay can be a screen for variant proteins, or for protein expression patterns which are diagnostic for particular physiological states or disease conditions.

In another embodiment, the combinations of the invention can be used to monitor biochemical reactions such as, e.g., interactions of proteins, nucleic acids, small molecules, or the like - for example the efficiency or specificity of interactions between antigens and antibodies; or of receptors (such as purified receptors or receptors bound to cell membranes) and their ligands, agonists or antagonists; or of enzymes (such as proteases or kinases) and their substrates, or increases or decreases in the amount of substrate converted to a product; as well as many others. Such biochemical assays can be used to characterize properties of the probe or target, or as the basis of a screening assay. For example, to screen samples for the presence of particular proteases (e.g., proteases involved in blood clotting such as proteases Xa and VIIa), the samples can be assayed on combinations in which the probes are fluorogenic substrates specific for each protease of interest. If a target protease binds to and cleaves a substrate, the substrate will fluoresce, usually as a result, e.g., of cleavage and separation between two energy transfer pairs, and the signal can be detected. In another example, to screen samples for the presence of a particular kinase(s) (e.g., Src, tyrosine kinase, or ZAP70), samples containing one or more kinases of interest can be assayed on combinations in which the probes are peptides which can be selectively phosphorylated by one of the kinases of interest. Using art-recognized, routinely determinable conditions, samples can be incubated with the array of substrates, in an appropriate buffer and with the necessary cofactors, for an empirically determined period of time. (In some assays, e.g., for biochemical studies of factors that regulate the activity of kinases of interest, the concentration of each kinase can be adjusted so that each substrate is phosphorylated at a similar rate.) After treating (e.g., washing) each reaction under empirically determined conditions to remove kinases and undesired reaction components (optionally), the phosphorylated substrates can be detected by, for example, incubating them with detectable reagents such as, e.g., fluorescein-labeled anti-phosphotyrosine or anti-phosphoserine antibodies (e.g., at a concentration of about 10 nM, or more or less), and the signal can be detected. In another example, binding assays can be performed. For example, SH2 domains such as GRB2 SH2 or ZAP70 SH2 can be assayed on probe arrays of appropriate phosphorylated peptides; or blood sera can be screened on probe arrays of particular receptors for the presence of immune deficiencies. Also, enzyme-linked assays can be performed in such an array format. Combinations of the invention can also be used to detect mutant enzymes, which are either more or less active than their wild type counterparts, or to screen for a variety of agents including herbicides or pesticides.

Of course, MAPS assays can be used to quantitate (measure, quantify) the amount of active target in a sample, provided that probe is not fully occupied, that is, not more than about 90% of available probe sites are bound (or reacted or hybridized) with target. Under these conditions, target can be quantitated because having more target will result in having more probe bound. On the other hand, under conditions where more than about 90% of available probe sites are bound, having more target present would not substantially increase the amount of target bound to probe. Any of the heretofore-mentioned types of targets can be quantitated in this manner. Furthermore, it demonstrates that even if a target is present in large excess (e.g., if it is present in such large amounts that it saturates the amount of available probe in a MAPS probe array), by adding known amounts of unlabeled target to the binding mixture, one can "shift the sensitivity" of the reaction in order to allow even such large amounts of target to be quantitated.

In another embodiment, combinations of the invention can be used to screen for agents which modulate the interaction of a target and a given probe. An agent can modulate the target/probe interaction by interacting directly or indirectly with either the probe, the target, or a complex formed by the target plus the probe. The modulation can take a variety of forms, including, but not limited to, an increase or decrease in the binding affinity of the target for the probe, an increase or decrease in the rate at which the target and the probe bind, a competitive or non-competitive inhibition of the binding of the probe to the target, or an increase or decrease in the activity of the probe or the target which can, in some cases, lead to an increase or decrease in the probe/target interaction. Such agents can be man-made or naturally-occurring substances. Also, such agents can be employed in their unaltered state or as aggregates with other species; and they can be attached, covalently or noncovalently, to a binding member, either directly or via a specific binding substance. For example, to identify potential "blood thinners," or agents which interact with one of the cascade of proteases which cause blood clotting, cocktails of the proteases of interest can be tested with a plurality of candidate agents and then tested for activity as described above. Other examples of agents which can be employed by this invention are very diverse, and include pesticides and herbicides.

In another embodiment, the combinations of the invention can be used to screen for agents which modulate a pattern of gene expression. Arrays of oligonucleotides can be used, for example, to identify mRNA species whose pattern of expression from a set of genes is correlated with a particular physiological state or developmental stage, or with a disease condition ("correlative" genes, RNAs, or expression patterns). By the terms "correlate" or "correlative," it is meant that the synthesis pattern of RNA is associated with the physiological condition of a cell, but not necessarily that the expression of a given RNA is responsible for or is causative of a particular physiological state. For example, a small subset of mRNAs can be identified which are expressed, upconverted and/or downconverted in cells which serve as a model for a particular disease state; this altered pattern of expression as compared to that in a normal cell, which does not exhibit a pathological phenotype, can serve as a indicator of the disease state ("indicator" genes, RNAs, or expression patterns). The terms "correlative" and "indicator" can be used interchangeably. For example, cells treated with a tumor promoter such as phorbol myristate might exhibit a pattern of gene expression which mimics that seen in the early stages of tumor growth. In another model for cancer, mouse insulinoma cells (e.g., cell line TGP61), when infected with adenovirus, exhibit an increase in the expression of, e.g., c-Jun and MIP-2, while the expression of housekeeping genes such as GAPDH and L32 remains substantially unaffected.

Agents which, after contacting a cell from a disease model, either directly or indirectly, and either in vivo or in vitro (e.g., in tissue culture), modulate the indicator expression pattern, might act as therapeutic agents or drugs for organisms (e.g., human or other animal patients, or plants) suffering from the disease. Agents can also modulate expression patterns by contacting the nucleic acid directly, e.g., in an in vitro (test tube) expression system. As used herein, "modulate" means to cause to increase or decrease the amount and/or activity of a molecule or the like which is involved in a measurable reaction. The combinations of the invention can be used to screen for such agents. For example, a series of cells (e.g., from a disease model) can be contacted with a series of agents (e.g., for a period of time ranging from about 10 minutes to about 48 hours or more) and, using routine, art-recognized methods (e.g., commercially available kits), total RNA or mRNA extracts can be made. If it is desired to amplify the amount of RNA, standard procedures such as RT-PCR amplification can be used (see, e.g., Innis et al eds., (1996) PCR Protocols: A Guide to Methods in Amplification, Academic Press, New York). The extracts (or amplified products from them) can be allowed to contact (e.g., incubate with) a plurality of substantially identical arrays which comprise probes for appropriate indicator RNAs, and those agents which are associated with a change in the indicator expression pattern can be identified.

Similarly, agents can be identified which modulate expression patterns associated with particular physiological states or developmental stages. Such agents can be man-made or naturally-occurring substances, including environmental factors such as substances involved in embryonic development or in regulating physiological reactions, or substances important in agribusiness such as pesticides or herbicides. Also, such agents can be employed in their unaltered state or as aggregates with other species; and they can be attached, covalently or noncovalently, to a binding member, either directly or via a specific binding substance.

One way to carry out the invention is based on a kit useful for the detection of at least one target in a sample, which comprises:
a surface, comprising multiple spatially discrete regions, at least two of
which are substantially identical, each region comprising at least eight different anchors (oligonucleotide, or one of the other types described herein), and
a container comprising at least one bifunctional linker molecule, which has a first portion specific for at least one of said anchor(s) and a second portion that comprises a probe which is specific for at least one of said target(s).

In one embodiment, there is provided a surface as in a) above and a set of instructions for attaching to at least one of said anchors a bifunctional linker molecule, which has a first portion specific for at least one of said anchor(s) and a second portion that comprises a probe which is specific for at least one target. The instructions can include, for example (but are not limited to), a description of each of the anchors on the surface, an indication of how many anchors there are and where on the surface they are located, and a protocol for specifically attaching (associating, binding, etc.) the linkers to the anchors. For example, if the anchors are oligonucleotides, the instructions can include the sequence of each anchor, from which a practitioner can design complementary anchor-specific moieties of linkers to interact specifically with (e.g., hybridize to) the anchors; if the anchors are peptides, the instructions can convey information about, e.g., antibodies which will interact specifically with the peptides. The instructions can also include a protocol for associating the anchors and linkers, e.g., conditions and reagents for hybridization (or other type of association) such as temperature and time of incubation, conditions and reagents for removing unassociated molecules (e.g., washes), and the like. Furthermore, the instructions can include information on the construction and use of any of the types of control linkers discussed herein, and of methods, e.g., to quantitate, normalize, "fine-tune" or calibrate assays to be performed with the combinations. The instructions can encompass any of the parameters, conditions or embodiments disclosed in this application, all of which can be performed routinely, with conventional procedures, by one of skill in the art.

As discussed elsewhere in this application, a practitioner can attach to a surface of the invention comprising a given array (or arrays) of anchors, a wide variety of types of linkers, thereby programming any of a wide variety of probe arrays. Moreover, a practitioner can remove a given set of linkers from a surface of the invention and add to it another set of linkers (either the same or different from the first set), allowing a given surface to be reused many times. This flexibility and reusability constitute further advantages of the invention.

Another aspect, of the description relates to a method of determining which of a plurality of polynucleotides are complementary to a given nucleic acid,
wherein one or more of said polynucleotides may be complementary to said nucleic acid, and wherein each of said polynucleotides comprises two different oligonucleotide sequences, the first of which defines an oligonucleotide probe corresponding to said polynucleotide, and the second of which defines a detector oligonucleotide corresponding to said polynucleotide, comprising,
contacting a sample which comprises molecules of said nucleic acid with at least one region of a combination, wherein said region comprises an array of oligonucleotide probes, at least one of which corresponds to each of said polynucleotides,
incubating said sample with said region, thereby permitting molecules of said nucleic acid to bind to said polynucleotide-corresponding oligonucleotide probes which are complementary to portions of said nucleic acid,
incubating said region comprising molecules of said nucleic acid bound to one or more of said polynucleotide-corresponding oligonucleotide probes with a detector oligonucleotide which corresponds to a polynucleotide to which a given one of the oligonucleotide probes of said array corresponds, thereby binding detector oligonucleotides to nucleic acid molecules which have bound to said given oligonucleotide probe or to other oligonucleotide probes which are complementary to said nucleic acid,
detecting the presence of said detector oligonucleotides, thereby identifying which polynucleotide-corresponding oligonucleotide probes of said array are complementary to portions of a nucleic acid which binds to said given oligonucleotide polynucleotide-corresponding probe, thereby identifying which polynucleotides are complementary to said given nucleic acid,
wherein said array of oligonucleotide probes is immobilized on a region of a combination, wherein said combination comprises
a surface comprising a number of spatially discrete, substantially identical, regions equal to the number of polynucleotides to be studied, each region comprising
a number of different anchors equal to the number of polynucleotides to be studied, each anchor in association with
a bifunctional linker which has a first portion that is specific for the anchor, and a second portion that comprises an oligonucleotide probe which corresponds to at least one of said polynucleotides.

In another aspect of the invention, the above methods to map ESTs or other polynucleotides further comprise removing unbound portions of the sample between one or more of the steps.

The probes separated from the crosslinked target can be amplified or modified before detection, by any number of methods familiar to one skilled in the art, but for instance by PCR. In this case the recovered probes can be extended, tagged, or labeled as well as amplified.

Nucleic acids used in the methods of the invention, e.g., targets, oligonucleotides involved in the detection of a target, or nuclease protection fragments (described elsewhere herein) can be amplified by any of a variety of conventional enzymatic procedures, including PCR and ligase reactions. One such amplification method is Transcription-Mediated Amplification (see, e.g., Abe et al. (1993). J. Clin. Microbiol. 31, 3270-3274).

In another embodiment of the invention, one or more nucleic acid targets of interest are hybridized to specific polynucleotide protection fragments and subjected to a nuclease protection procedure, and those protection fragments which have hybridized to the target(s) of interest are assayed on MAPS plates. Of course, such "MAPS plates" can contain anchors which are not associated with linkers (e.g., which can be associated directly with a target or nuclease protection fragment of interest); the advantages of nuclease protection as used in conjunction with any type of probe array will be evident to one of skill in the art from this specification and any of its ancestors to which benefit is claimed. If the target of interest is an RNA and the protection fragment is DNA, a Nuclease Protection/ MAPS Assay (NPA-MAPS) can reduce the need for extensive handling of RNA, which can be sensitive to degradation by contaminating nucleases and thus difficult to work with. Treatment of a sample with a nuclease protection procedure also allows for a sample with reduced viscosity. Nuclease protection of a sample can allow for greater sensitivity and reproducibility in an assay. An advantage of the invention is that assays can be sensitive enough that amplification of the target (e.g., by PCR) is not necessary in order to detect a signal. In an NPA-MAPS assay, the probes in the probe array are oligonucleotides of the same strandedness as the nucleic acid targets of interest, rather than being complementary to them, as in a standard MAPS assay.

In an NPA-MAPS assay, the target of interest can be any nucleic acid, e.g., genomic DNA, cDNA, viral DNA or RNA, rRNA, tRNA, mRNA, oligonucleotides, nucleic acid fragments, modified nucleic acids, synthetic nucleic acids, or the like. In a preferred embodiment of the invention, the procedure is used to assay for one or more mRNA targets which are present in a tissue or cellular RNA extract. A sample which contains the target(s) of interest is first hybridized under selected stringent conditions (see above for a discussion of appropriate reaction conditions to achieve specific hybridization) to an excess of one or more specific protection fragment(s). A protection fragment is a polynucleotide, which can be, e.g., RNA, DNA (including a PCR product), PNA or modified or substituted nucleic acid, that is specific for a portion of a nucleic acid target of interest. By "specific" protection fragment, it is meant a polynucleotide which is sufficiently complementary to its intended binding partner to bind to it under selected stringent conditions, but which will not bind to other, unintended nucleic acids. A protection fragment can be at least 10 nucleotides in length, preferably 50 to about 100, or about as long as a full length cDNA. In a preferred embodiment, the protection fragments are single stranded DNA oligonucleotides. Protection fragments specific for as many as 100 targets or more can be included in a single hybridization reaction. After hybridization, the sample is treated with a cocktail of one or more nucleases so as to destroy nucleic acid other than the protection fragment(s) which have hybridized to the nucleic acid(s) of interest and (optionally) the portion(s) of nucleic acid target which have hybridized and been protected from nuclease digestion during the nuclease protection procedure (are in a duplexed hybrid). For example, if the sample comprises a cellular extract, unwanted nucleic acids, such as genomic DNA, tRNA, rRNA and mRNA's other than those of interest, can be substantially destroyed in this step. Any of a variety of nucleases can be used, including, e.g., pancreatic RNAse, mung bean nuclease, S1 nuclease, RNAse A, Ribonuclease T1, Exonuclease III, Exonuclease VII, RNAse CLB, RNAse PhyM, RNAse U2, or the like, depending on the nature of the hybridized complexes and of the undesirable nucleic acids present in the sample. RNAse H can be particularly useful for digesting residual RNA bound to a DNA protection fragment. Reaction conditions for these enzymes are well-known in the art and can be optimized empirically. Also, chemical procedures can be used, e.g., alkali hydrolysis of RNA. As required, the samples can be treated further by well-known procedures in the art to remove unhybridized material and/or to inactivate or remove residual enzymes (e.g., phenol extraction, precipitation, column filtration, etc.). The process of hybridization, followed by nuclease digestion and (optionally) chemical degradation, is called a nuclease protection procedure; a variety of nuclease protection procedures have been described (see, e.g., Lee et al (1987). Meth. Enzymol. 152, 633-648. Zinn et al (1983). Cell 34, 865-879.). Samples treated by nuclease protection, followed by an (optional) procedure to inactivate nucleases, are placed in contact with a MAPS probe array and the usual steps of a MAPS assay are carried out. Bound protection fragments can be detected by, e.g., hybridization to labeled target-specific reporters, as described herein for standard MAPS assays, or the protection fragments, themselves, can be labeled, covalently or non-covalently, with a detectable molecule.

If desired, one or more controls can be included for normalizing an NPA-MAPS assay. For example, one or more protection fragments corresponding to a nucleic acid which is expected to be present in each of a series of samples in a substantially constant amount (e.g., a constitutively produced mRNA, a portion of a genomic DNA, a tRNA or rRNA) can be used. The ability to detect and quantify an internal normalization control, e.g., genomic DNA, in an assay for measuring nucleic acids which are present in variable amounts (e.g., mRNAs), is an advantage of using protection fragments in the assays.

Because the amount of the normalization standard(s) may be lower than that of expressed mRNAs of interest, the assay may be adjusted so the signals corresponding to the expressed genes do not swamp out the signal(s) corresponding to the normalization standard(s). Methods of adjusting the signal levels are conventional and will be evident to one of skill in the art. For example, any of the methods described herein for balancing signal intensities (e.g., signal attenuation, fine-tuning) can be used (e.g., using blocked linkers; labeling the signal moiety designed to detect the normalization standard at a greater level than that designed to detect the mRNA; placing at a locus designated for detecting a normalization standard a plurality of linkers which are specific for different portions of the normalization nucleic acid, or for protection fragments that correspond to different portions of that nucleic acid, etc.). The normalization standard(s) and the nucleic acid targets (e.g., mRNAs) of interest can be detected simultaneously or sequentially, e.g., by any of the methods described elsewhere herein.

In a preferred embodiment, the protection fragment is directly labeled, e.g., rather than being labeled by hybridization to a target-specific reporter. For example, the reporter is bound to the protection fragment through a ligand-antiligand interaction, e.g., a streptavidin enzyme complex is added to a biotinylated protection oligonucleotide. In another example, the protection fragment is modified chemically, (e.g., by direct coupling of horseradish peroxidase (HRP) or of a fluorescent dye) and this chemical modification is detected, either with the nucleic acid portion of the protection fragment or without it, (e.g., after cleavage of the modification by, for example, an enzymatic or chemical treatment). In any of the above methods, a protection fragment can be labeled before or after it has hybridized to a corresponding linker molecule.

In order to control that the nuclease protection procedure has worked properly, i.e. that non-hybridized nucleic acids have been digested as desired, one can design one or more protection fragments to contain overhanging (non-hybridizing) segments that should be cleaved by the nucleases if the procedure works properly. The presence or absence of the overhanging fragments can be determined by hybridization with a complementary, labeled, detection probe, or the overhanging portion of the protection fragment, itself, can be labeled, covalently or non-covalently, with a detectable molecule. This control can be performed before the sample is placed in contact with the probe array, or as a part of the MAPS assay, itself. Of course, because different labels can be easily distinguished (e.g., fluors with different absorption spectra), several differently labeled oligonucleotides can be included in a single assay. Further, the standard nuclease protection assay as analyzed by gel electrophoresis can be used during assay development to verify that the protection fragments are processed as expected.

Other controls for correct nuclease digestion will be evident to one of skill in the art. For example, one can include in an assay a nuclease protection fragment which is known not to have specificity for any nucleic acid in the sample (e.g., in an assay for plant nucleic acids, one can include a protection fragment specific for an animal gene which is known to be absent in plants).

After detection of targets, the detection probe (e.g., HRP-labeled) signal can be eliminated (e.g. denatured, killed, quenched, suppressed, blocked), plates washed to remove any resulting reagents, agents, or buffers which might interfere in the next step (e.g., denaturing regent), and then the overhang can be detected with a different detection probe (e.g., also HRP-labeled). The use of signal denaturation followed by addition of a different detection probe with the same signaling moiety can be used at various stages of the assay. Utilization of two different flourescent probes and dual color detection can be used without denaturation or signal blocking.

In one embodiment of the invention, as was noted above, an oligonucleotide probe is used to screen for a nucleic acid which comprises one or more polymorphisms. In a preferred embodiment, the nucleic acid (e.g., a DNA, such as a genomic DNA, or an RNA, such as an mRNA) comprises one or more SNPs. Routine, art-recognized procedures can be used to carry out the procedure. For example, to screen for a DNA comprising a known SNP, or an mRNA expressed from such a DNA, a "SNP-specific" protection fragment is hybridized to a sample comprising nucleic acids which may comprise that SNP. By "SNP-specific" protection fragment is meant in this context a protection fragment which comprises the altered base of the SNP or, if an mRNA is to be analyzed, the reverse complement of such a sequence. The sample is then treated with one or more appropriate nucleases which, under appropriate, empirically determinable conditions, digest unhybridized single stranded nucleic acid and cleave double stranded (duplex) nucleic acid (e.g., DNA-DNA hybrids, DNA-RNA hybrids, or the like) at the site of a mismatch (e.g., a single base mismatch). Appropriate nucleases include, e.g., S1 or RHAse H. If a nucleic acid which comprises a SNP is present in the sample and hybridizes to the SNP-specific protection fragment, the protection fragment will survive the digestion procedure intact, and can be subjected to a MAPS assay and detected by a detection probe or detection oligonucleotide which is specific for a sequence of the protection fragment. Nucleic acids which do not comprise the SNP will be cleaved at the site of the mismatch between the SNP-specific protection fragment and the corresponding wild type sequence in the nucleic acid. If desired, a portion of the protection fragment which lies either distal to or proximal to the site of cleavage can be removed, using conventional methods (e.g., heat denaturation, enzymatic cleavage, etc.) An assay can be designed either so that the cleaved molecules (or portions thereof) will not bind to linkers, or so that such cleaved molecules, even if a portion thereof binds to a linker, will not be detected by an appropriately designed detection probe or detection oligonucleotide. An alternate embodiments is drawn to the detection of SNPs, which is applicable, e.g., to the detection of SNPs in genomic DNA.

In one embodiment of the invention, different types of targets in a sample, e.g., various combinations of DNA, RNA, intracellular proteins and secreted proteins, can be assayed with a single probe array.

In addition to the variety of high throughput assays described above, many others will be evident to one of skill in the art.

An advantage of using multiprobe assays is the ability to include a number of "control" probes in each probe array which are subject to the same reaction conditions as the actual experimental probes. For example, each region in the array can comprise positive and/or negative controls. The term, a "positive control probe," is used herein to mean a control probe that is known, e.g., to interact substantially with the target, or to interact with it in a quantitatively or qualitatively known manner, thereby acting as a(n internal) standard for the probe/target interaction. Such a probe can control for hybridization efficiency, for example. The term, a "negative control probe," is used herein to mean a control probe which is known not to interact substantially with the target. Such a probe can control for hybridization specificity, for example. As examples of the types of controls which can be employed, consider an assay in which an array of oligonucleotide probes is used to screen for agents which modulate the expression of a set of correlative genes for a disease. As an internal normalization control for variables such as the number of cells lysed for each sample, the recovery of mRNA, or the hybridization efficiency, a probe array can comprise probes which are specific for one or more basal level or constitutive house-keeping genes, such as structural genes (e.g., actin, tubulin, or others) or DNA binding proteins (e.g., transcription regulation factors, or others), whose expression is not expected to be modulated by the agents being tested. Furthermore, to determine whether the agents being tested result in undesired side effects, such as cell death or toxicity, a probe array can comprise probes which are specific for genes that are known to be induced as part of the apoptosis (programmed cell death) process, or which are induced under conditions of cell trauma (e.g., heat shock proteins) or cell toxicity (e.g., p450 genes).

Other control probes can be included in an array to "fine tune" the sensitivity of an assay. For example, consider an assay for an agent which modulates the production of mRNAs associated with a particular disease state. If previous analyses have indicated that one of the correlative mRNAs (say, mRNA-A) in this set is produced in such high amounts compared to the others that its signal swamps out the other mRNAs, the linkers can be adjusted to "fine tune" the assay so as to equalize the strengths of the signals. "Blocked linkers," which comprise the anchor-specific oligonucleotide sequence designated for the mRNA-A target, but which lack the probe-specific sequence, can be added to dilute the pool of target-specific linkers and thus to reduce the sensitivity of the assay to that mRNA. The appropriate ratios of blocked and unblocked linkers can be determined with routine, conventional methods by one of skill in the art.

The "fine tuning" of an assay for a particular target by diluting an active element with an inactive element can also be done at other steps in the assay. For example, it can be done at the level of detection by diluting a labeled, target-specific reporter with an "inactive" target-specific reporter, e.g., one with the same target-specific moiety (e.g., an oligonucleotide sequence) but without a signaling entity, or with an inactivated or inactive form of the signaling entity. The term "signaling entity," as used herein, refers to a label, tag, molecule, or any substance which emits a detectable signal or is capable of generating such a signal, e.g., a fluorescent molecule, luminescence enzyme, or any of the variety of signaling entities which are disclosed herein). In an especially preferred embodiment, the "fine tuning" can be done at the step of contacting a target-containing complex with a detection linker. A set of detection linkers can be designed, e.g., to fine tune the sensitivity for each individual target in an assay. For example, if a particular target is known to be present in a sample at very high levels, the detection linker for that target can be diluted with an empirically-determinable amount of "blocked detection linker," comprising the target-specific moiety (e.g., oligonucleotide sequence) but no moiety specific for a reporter reagent, or comprising the target-specific moiety and a reporter reagent-specific moiety which is pre-bound to an inactive reporter reagent. That is, instead of comprising a moiety specific for a reporter reagent, that moiety can be absent, or prevented (e.g., blocked) from interacting with (e.g., hybridizing to) the reporter reagent. Such fine tuning is sometimes referred to herein as signal "attenuation."

Samples to be tested in an assay of the invention can comprise any of the targets described above, or others. Liquid samples to be assayed can be of any volume appropriate to the size of the test region, ranging from about 100 nanoliters to about 100 microliters. In a preferred embodiment, liquid drops of about 1 microliter are applied to each well of a 1536 well microtiter dish. Samples can be placed in contact with the probe arrays by any of a variety of methods suitable for high throughput analysis, e.g., by pipetting, inkjet based dispensing or by use of a replicating pin tool. Samples are incubated under conditions (e.g., salt concentration, pH, temperature, time of incubation, etc.- see above) effective for achieving binding or other stable interaction of the probe and the target. These conditions are routinely determinable. After incubation, the samples can optionally be treated (e.g., washed) to remove unbound target, using conditions which are determined empirically to leave specific interactions intact, but to remove non-specifically bound material. For example, samples can be washed between about one and ten times or more under the same or somewhat more stringent conditions than those used to achieve the probe/target binding.

Samples containing target RNA, e.g., mRNA, rRNA, tRNA, viral RNA or total RNA, can be prepared by any of a variety of procedures. For example, in vitro cell cultures from which RNA is to be detected can be plated on the regions of a surface, such as in individual wells of a microtiter plate. Optionally, these cells, after attaining a desired cell density, can be treated with an agent of interest, such as a stimulating agent or a potential therapeutic agent, which can be added to the cells by any of a variety of means, e.g., with a replicating pin tool (such as the 96 or 384 pin tools available from Beckman), by pipetting or by ink-jet dispensing, and incubated with the cells for any appropriate time period, e.g., between about 15 minutes and about 48 hours, depending upon the assay. Total RNA, mRNA, etc. extracts from tissues or cells from an in vitro or in vivo source can be prepared using routine, art-recognized methods (e.g., commercially available kits).

In one embodiment, cells are lysed (or permeabilized), in the presence or absence of nuclease protection fragment(s), and the crude lysate is used directly (e.g., in the well of a microtiter plate), without further purification from, e.g., other cellular components. If the cells are lysed in the absence of nuclease protection fragments, such protection fragments can optionally be added subsequently to the lysate.

In a preferred embodiment, e.g., in which nuclease protection fragments are detected, samples are prepared by contacting cells of interest (e.g., cells on the surface of a well of a microtiter plate; cells in a tissue or whole organism sample; or the like) with an aqueous medium (lysis solution) which comprises a surfactant or detergent (e.g., SDS, e.g., at about 0.01% to about 0.5% w/v) and an agent (e.g., formamide (e.g., at about 8 -about 60%, v/v), guanidium HCl (e.g., at about 0.1- about 6M), guanidium isothyocyanate (e.g., at about 0.05 - about 8M) or urea (e.g., at about 40- about 46%, w/v, or about 7M)), which, alone or in combination with one or more other agents, can act as a chaotropic agent. The aqueous medium can be buffered by any standard buffer. In a preferred embodiment, the buffer is about 0.5-6X SSC, more preferably about 3X SSC. Optionally, the aqueous medium can also comprise tRNA at an appropriate concentration, e.g., about 0.1- 2.0 mg/ml, preferably about 0.5 mg/ml. Nuclease protection fragments may also be added to the aqueous medium before it is added to the cells. The optimal concentration of each protection fragment can be determined empirically, using conventional methods. In a preferred embodiment, the concentration of each protection fragment is about 3 to about 300 pM, more preferably about 30 pM.

Cells are incubated in the aqueous solution until the cells become permeabilized and/or lysed, and DNA and/or mRNA is released from the cells into the aqueous medium. Cells are incubated in the aqueous medium for an empirically determinable period of time (e.g., about 1 min to about 60 min), at an empirically determinable optimizable temperature (e.g., about 37°C to about 115°C, preferably about 90°C to about 115 ºC).

For example, in one embodiment, in which both DNA and RNA are released from the cells in a denatured form capable of binding to a protection fragment, the cells are incubated for about 1 min to about 60 min, preferably about 5 to about 20 min, in the aqueous medium at about 90 to about 115ºC, preferably about 105ºC. If desired, e.g., when it is desirable to assay for DNA in the absence of RNA, any of a variety of conventional ribonucleases can be included in the incubation mixture. Selection of an appropriate ribonuclease, and optimization of digestion conditions, are conventional and readily determined by a skilled worker.

In another embodiment, mRNA can be prepared by incubating cells for about 5 to about 20 min, preferably about 10 min, in an aqueous medium at about 90º to about 100ºC, preferably about 95ºC., optionally in the presence of one or more protection fragments. In this case, mRNA is substantially released from the cells in a denatured form capable of binding to a protection fragment, and DNA remains substantially inside or attached to the cells, or is unavailable to a probe by virtue of its double-stranded nature, or is released from the cells, but in a form which is not able to bind to a protection fragment (e.g., is not denatured). Without wishing to be bound to any particular mechanism, it appears that, as the nucleic acid is released from the lysed/permeabilized cells, it is sufficiently denatured to allow it to bind to a protection fragment to form a stable duplex which is resistant to degradation by endogenous or exogenous reagents or enzymes, and proteins within the cell (e.g., nucleases) are denatured and/or inactivated.

Following preparation of a nucleic acid of interest by the above procedure, the sample can be diluted, in the appropriate volume, so that the aqueous medium does not inhibit the function of exogenously added proteins such as, e.g., nucleases (e.g., S1 nuclease), polymerases (e.g., polymerases required for PCR reactions), or binding proteins (e.g., streptavidin). The amounts of dilution, and the identity and amounts of the components to be used in the aqueous solution, as described above, can be determined empirically, using conventional methods.

For any of the methods of this invention, targets can be labeled (tagged) by any of a variety of procedures which are well-known in the art and/or which are described elsewhere herein (e.g., for the detection of nuclease protection fragments). For example, the target molecules can be coupled directly or indirectly with chemical groups that provide a signal for detection, such as chemiluminescent molecules, or enzymes which catalyze the production of chemiluminsecent molecules, or fluorescent molecules like fluorescein or cy5, or a time resolved fluorescent molecule like one of the chelated lanthanide metals, or a radioactive compound. Alternatively, the targets can be labeled after they have reacted with the probe by one or more labeled target-specific reporters (e.g., antibodies, oligonucleotides as shown in Fig. 1, or any of the general types of molecules discussed above in conjunction with probes and targets).

One type of fluorescent molecule can be an "upconverting phosphore," i.e., a fluor which absorbs and is excited at a long wavelength (e.g, IR), then emits at a shorter wavelength (e.g., visible light). Because upconverting phosphores absorb at a longer wavelength than do most potentially interfering materials present in a typical sample to be analyzed, upconverting phosphores allow a reduction in interference caused by material in the sample, compared to phosphores which absorb at a lower wavelength. The narrow emission spectrum of most upconverting phosphores also allows the simultaneous detection of a large number of different upconverting phosphores. Upconverting phosphores are well-known and conventional in the art, and include, e.g., rare earth metal ions such as, e.g., Ytterbium (Yb), Erbium (Er), Thulium (Tm) and Praseodymium (Pr), particularly in the form of an oxysulfide salt. As many as 80 or more independently detectable upconverting phosphores have been described. (See, e.g., Biological Agent Detection and Identification, April 27-30, 1999, DARPA, Biological Warfare Defense, Defense Sciences Office. The phosphores can optionally be attached to any surface, e.g., to a microsphere or a latex bead. Like other fluorescent labels, upconverting phosphores can be detected by energy transfer to (or modulation by) the label on a sufficiently close linker, target or reporter. Furthermore, as with other signaling entities disclosed herein, upconverting phosphores can be used to quantitate the amount of a target, and can be used in any of the variety of procedures described herein, e.g., to detect nuclease protection fragments.

Of course, upconverting phosphores can also be used to detect targets which are distributed in any other fashion on a surface, e.g., targets (including nuclease protection fragments) which are bound directly to a surface, bound directly to an array of different oligonucleotides on a surface, or bound via bifunctional linkers to anchors (different or substantially identical) which are distributed substantially evenly, or in any desired organization or pattern, on a surface. Any surface can be used, e.g., a flow-through system, or a solid surface such as, e.g., a bead. Beads used in any of the assays of the invention can be of any type, e.g., made of any material, magnetic and/or non-magnetic; and the beads used in a single assay can be of substantially the same, or different, sizes and/or shapes.

A variety of more complex sandwich-type detection procedures can also be employed. For example, a target can be hybridized to a bifunctional molecule containing a first moiety which is specific for the target and a second moiety which can be recognized by a common (i.e., the same) reporter reagent, e.g., a labeled polynucleotide, antibody or the like. The bifunctional molecules can be designed so that any desired number of common reporters can be used in each assay.

For any of the methods of this invention, a variety of complex sandwich-type detection procedures can be employed to label (tag) targets. For example, a target can interact with, e.g., hybridize to, a bifunctional (or multifunctional) molecule (a "detection linker") containing a first moiety that is specific for the target and a second moiety that is specific for a "reporter reagent." The term "specific for" has the meaning as used herein with respect to the interactions of, e.g., probes and targets. The term "reporter reagent," as used herein, refers to a labeled polynucleotide, antibody or any of the general types of molecules discussed herein in conjunction with probes and targets. These two moieties of a detection linker can recognize (interact or associate with) their respective binding partners in any of the manners discussed above in conjunction, e.g., with probes and targets. A detection linker can also comprise other sequences, e.g., sequences that are specific for a target but are different from (non-overlapping with) the target-specific moiety of the corresponding anchor-bound linker. Any sequence present in a detection linker can serve as a recognition sequence for a detection probe or a reporter agent. In a preferred embodiment, a detection linker is a polynucleotide.

Detection linkers can be designed so that any desired number of common reporter reagents can be used in an assay. For example, a set of detection linkers can be designed such that each detection linker is specific for a different target, but comprises a binding site for the same (common), or for one of a limited number of, reporter reagents. The ability to use a limited number of (e.g., one) reporter reagents to label a variety of targets in a single assay provides the advantage of reduced cost and lower backgrounds. Of course, detection linker/reporter reagent combinations can be used to detect targets which are distributed in any fashion on a surface, e.g., as described above for the types of target arrangements that can be detected by upconverting phosphores.

In a most preferred embodiment, detection linkers can be designed to detect nuclease protection fragments in such a way that protection fragments which have been cleaved by a nuclease from control "overhang" sequences during a nuclease protection procedure are preferentially labeled. In this embodiment, a detection linker comprises a first moiety that is specific for a target and a second moiety that is specific for the common control overhang sequence which, in a preferred embodiment, is present on substantially all of the nuclease protection fragments at the start of an assay. If, as desired, the control overhang sequence has been cleaved from a nuclease protection fragment during a nuclease protection reaction, the target-specific moiety of the detection linker will hybridize to the cleaved protection fragment, but the control overhang-specific moiety of the detection linker will be unbound and will remain available for further hybridization. If, on the other hand, the control overhang-specific sequence is not cleaved from a protection fragment, e.g., because of incomplete nuclease digestion during a nuclease protection procedure, both the target-specific and the control overhang -specific moieties of the detection linker will hybridize to the protection fragment and will not be available for further hybridization. In a preferred embodiment, complexes comprising nuclease protection fragments and bound detection linkers are then hybridized in a further step to a reporter reagent which comprises a signaling entity (e.g., a fluorochrome, hapten, enzyme, or any other molecule bearing a detectable signal or signal-generating entity, as described herein) and an moiety (e.g., an oligonucleotide) which is specific for the control overhang-specific moiety of a detection linker. The reporter reagent will preferentially bind to and label those complexes in which the control overhang sequence of the nuclease protection fragment has been cleaved off, (i.e., a complex in which the control overhang-specific moiety of the detection linker is available for further hybridization to the reporter reagent.)

Numerous other variations of sandwich detection procedures will be evident to one of skill in the art.

Methods by which targets can be incubated with a target-specific reporter(s), or target/detection linker complexes can be incubated with reporter reagents, under conditions effective for achieving binding or other stable interaction, are routinely determinable (see above). For example, fluorescent oligonucleotide reporters (at a concentration of about 10 nM to about 1 µM or more, preferably about 30 nM, in a buffer such as 6X SSPE-T or others) can be incubated with the bound targets for between about 15 minutes to 2 hours or more (preferably about 30 to 60 minutes), at a temperature between about 15ºC. and about 45ºC. (preferably about room temperature). After incubation, the samples can optionally be treated (e.g., washed) to remove unbound target-specific reporters, using conditions which are determined empirically to leave specific interactions intact, but to remove non-specifically bound material. For example, samples can be washed between about one and ten times or more under the same or somewhat more stringent conditions than those used to achieve the target/reporter binding.

Tagging with a target-specific reporter(s) can provide an additional layer of specificity to the initial hybridization reaction, e.g., in the case in which a target-specific oligonucleotide reporter is targeted to a different portion of the sequence of a target nucleic acid than is the probe oligonucleotide, or in which probe and reporter antibodies recognize different epitopes of a target antigen. Furthermore, tagging with target-specific reporters can allow for "tuning" the sensitivity of the reaction. For example, if a target mRNA which is part of a correlative expression pattern is expressed at very low levels, the level of signal can be enhanced (signal amplification) by hybridizing the bound target to several (e.g., about two to about five or more) target-specific oligonucleotide reporters, each of which hybridizes specifically to a different portion of the target mRNA.

The ability to detect two types of labels independently allows for an additional type of control in MAPS assays. Some (e.g., about 10 to about 100%) of the linkers designated for a particular anchor locus can have a label (e.g., a fluor) attached to one end. For example, a rhodamine or Cy5 fluor can be attached at the 5' end of the linker. Such modified linkers are termed "control linkers." After a mixture of linkers and control linkers has been associated with anchors and a sample containing a target has been incubated with the resulting probe array, a target-specific reporter bearing a different fluor (e.g., fluorescein or another detection label such as a chemiluminescent one) can be used (or the target can be directly labeled with a fluor or other detection label); and the ratio of the two signals can be determined. The presence of control linkers permits calibration of the number of functional (e.g., able to interact with linkers) anchors within and between test regions (i.e. tests the capacity of each locus of the array to bind target, for purposes of normalizing signals), serves as a basis for quantitation of the amount of bound target, aids in localization of the anchor loci and/or provides a positive control, e.g., in cases in which there is no signal as a result of absence of target in a sample. In one embodiment of the invention, two different labels (e.g., fluorophores) can also be used to detect two different populations of target molecules; however, the ability to recognize the presence of targets by spatial resolution of signals allows the use of a single type of label for different target molecules.

The ability to detect labels independently (e.g., fluorescent labels which emit at distinguishable wavelengths, such as, e.g., fluorescein and rhodamine, or different upconverting phosphores) allows additional flexibility in the methods of the invention. For example, each of two or more targets can be labeled, directly or indirectly, with its own, uniquely detectable, label. This allows for the detection of targets on the basis of features specific to the labels (e.g., color of the emission) in addition to (or instead of), e.g., identifying the position of a localized target on a surface, or identifying a target by virtue of the size of a bead to which it is localized. In another embodiment of the invention, a multiplicity of targets can be detected independently at a single locus within a region. For example, two or more (e.g., 2, 3, 4, 5, 6 or more) targets can be detected at a locus which is defined by a single group of (substantially identical) anchors. That is, a set of linkers can be used, each of which has an anchor-specific portion specific for the same anchor plus a target-specific portion specific for a different target. If a set of, e.g., four such linkers is used, all four can bind to members of the group of anchors at a single locus, allowing four different targets to bind at that locus. If each of these targets is labeled (directly or indirectly) with a different, distinguishable, label, an investigator can determine the presence of each of the four targets at the locus independently. Therefore, an array of, e.g., five anchors (groups of anchors) in a region can be used in the scenario described above to detect as many as twenty different targets. Similarly, a plurality of targets, e.g., as many as 80 or more, can be detected independently when a single type of anchor is distributed, not at a single locus, but evenly, or in any desired fashion, on a solid surface such as, e.g., a bead or a flow through apparatus; and other aspects such as bead size or scatter can be used to provide information about target identity or groups of targets.

The association of multiple linkers (e.g., ranging from about two to about 50 or more), having different target specificities, with the anchors at a given locus (either a group of substantially identical anchors or a "mixed locus"), sometimes referred to herein as "mixed linkers," forms the basis for other embodiments of the invention, which will be evident to those of skill in the art. For example, at a given locus the anchors can be bound to a mixture of linkers which are specific for a plurality of different protection fragments, each of which corresponds to (is specific for) a different portion of a nucleic acid (e.g., an mRNA) of interest. The presence of such a plurality of different linkers at a locus allows for considerably increased sensitivity in the detection of a target (e.g., an mRNA) of interest, e.g., one present at low abundance in a sample. Each locus can be designed so that the number of linkers corresponding to different portions of an mRNA designated for that locus is inversely proportional (in an empirically determinable fashion) to the abundance of that mRNA in the sample. For example, if one mRNA of interest is found in a preliminary experiment to be present in a sample in large excess over a second mRNA of interest, the relative number of linkers corresponding to different portions of the two mRNAs can be adjusted so that the relative intensities of the signals corresponding to each mRNA are substantially the same. That is, the signal intensities can be adjusted so that the signal corresponding to the first mRNA does not swamp out the signal corresponding to the second mRNA. In this manner, one can adjust an assay to allow for simultaneous detection of a plurality of mRNAs which are present in widely different amounts in a sample, balancing the signal intensity corresponding to each mRNA.

In another embodiment of this description, as was noted above, a given locus can comprise linkers which are specific for a plurality of unrelated or different targets or protection fragments, allowing for the detection of a greatly increased number of targets or protection fragments with a single array of anchors. If, for example, each locus of an array of 350 anchors comprises linkers specific for 10 different targets, then the array can be used to detect as many as 3500 targets. In effect, such an arrangement allows one to convert an array which can detect a low density of targets to one which can detect a high density of targets.

Multiple molecules (e.g., protection fragments) bound at a single locus can be detected sequentially or simultaneously, e.g., using the detection methods described elsewhere in this application. (For a discussion of "detection linkers" and "reporter reagents," see, e.g., the section above concerning complex sandwich-type detection methods.) In one embodiment, a first target (e.g., a protection fragment) at a given locus is detected, e.g., with a first detection system (e.g., a detection linker/reporter reagent, or a detection probe specific for it); then that first detection linker/reporter reagent or probe is removed or inactivated, using conventional procedures (e.g., changing the pH to inactivate a reporter reagent comprising an enzyme that generates a chemoluminescent signal), and a second detection linker/reporter reagent or detection probe specific for a second target at the same locus is used to detect that second target; and so forth for as many cycles as desired. In another embodiment, the first detection linker/reporter reagent or detection probe is added to a combination as above, but it is not removed or inactivated before the second detection linker/reporter reagent or detection probe is added. In this embodiment, the amount of signal corresponding to the second target can be determined by subtracting out the amount of signal corresponding to the first target. In another embodiment, the first and second detection linker/reporter reagents or detection probes are added to the combinations as above, substantially simultaneously, and are detected individually, e.g., using differentially detectable labels as described elsewhere herein. In any of the detection methods described herein, the detection linkers can comprise moieties which are specific for the same or for different reporter reagents. For example, if four targets are associated with the linkers at a given locus, the detection linkers specific for each of the four targets can each comprise a moiety specific for a different reporter reagent. Therefore, after the set of all four detection linkers is hybridized to the targets, the targets can be detected sequentially or simultaneously, as described above, using the four different reporter reagents. Other detection methods, as well as combinations of the above methods, will be evident to one of skill in the art.

Of course, "mixed linkers" are also advantageous for use with surfaces which contain a single (non-repeated) region.

In another embodiment of the invention, "anchors" which are specific for a target(s) of interest are not associated with linkers, but rather are associated directly with the target(s); the target(s), in turn, can interact optionally with detection linker(s) or with detection probe(s).

Targets, whether labeled or unlabeled, can be detected by any of a variety of procedures, which are routine and conventional in the art (see, e.g., Fodor et al (1996). U.S. Pat. No. 5,510,270; Pirrung et al (1992). U.S. Pat. No. 5,143,854; Koster (1997). U.S.Pat. No. 5,605,798; Hollis et al (1997) U.S. Pat. No. 5,653,939; Heller (1996). U.S.Pat. No. 5,565,322; Eggers et al (1997). U.S.Pat. No. 5,670,322; Lipshutz et al (1995). BioTechniques 19, 442-447; Southern (1996). Trends in Genetics 12, 110-115). Detection methods include enzyme-based detection, colorimetric methods, SPA, autoradiography, mass spectrometry, electrical methods, detection of absorbance or luminescence (including chemiluminescence or electroluminescence), and detection of light scatter from, e.g., microscopic particles used as tags. Also, fluorescent labels can be detected, e.g., by imaging with a charge-coupled device (CCD) or fluorescence microscopy (e.g., scanning or confocal fluorescence microscopy), or by coupling a scanning system with a CCD array or photomultiplier tube, or by using array-based technology for detection (e.g., surface potential of each 10-micron part of a test region can be detected or surface plasmon resonance can be used if resolution can be made high enough.) Alternatively, an array can contain a label (e.g., one of a pair of energy transfer probes, such as fluorescein and rhodamine) which can be detected by energy transfer to (or modulation by) the label on a linker, target or reporter. Among the host of fluorescence-based detection systems are fluorescence intensity, fluorescence polarization (FP), time-resolved fluorescence, fluorescence resonance energy transfer and homogeneous time-released fluorescence (HTRF). Analysis of repeating bar-code-like patterns can be accomplished by pattern recognition (finding the appropriate spot or line for each specific labeled target by its position relative to the other spots or lines) followed by quantification of the intensity of the labels. Bar-code recognition devices and computer software for the analysis of one or two dimensional arrays are routinely generated and/or commercially available (e.g., see Rava et al (1996). U.S. Patent No. 5,545,531).

Another method which can be used for detection is 2-photon fluorescence, including applications where the fluorescence of endogenous or conjugated fluorochromes of components bound to the array surface is enhanced by being bound close to the surface of the array, for instance by close apposition to the substrate on which the array is formed, or by close apposition to other agents included in the anchor or linker or otherwise incorporated in the bound complex. Other fluorescence methods or utility include lifetime fluorescence, polarization, energy transfer, etc. For instance, such methods permit the simultaneous detection and descrimination of multiple targets within the same locus, and in some instances can discriminate between bound label and unbound label, eliminating the need to wash unbound lable away from the array, and thus facilitating the measurment of rapidly reversible or weak interactions by the array.

Methods of making and using the arrays of this invention, including preparing surfaces or regions such as those described herein, synthesizing or purifying and attaching or assembling substances such as those of the anchors, linkers, probes and detector probes described herein, and detecting and analyzing labeled or tagged substances as described herein, are well known and conventional technology. In addition to methods disclosed in the references cited above, see, e.g., patents assigned to Affymax, Affymetrix, Nanogen, Protogene, Spectragen, Millipore and Beckman (from whom products useful for the invention are available); standard textbooks of molecular biology and protein science, including those cited above; and Cozette et al (1991). U.S. Pat. 5,063,081; Southern (1996), Current Opinion in Biotechnology 7, 85-88; Chee et al (1996). Science 274, 610-614; and Fodor et al (1993). Nature 364, 555-556.

### BRIEF DESCRIPTION OF THE DRAWINGS

Various features and attendant advantages of the present invention will be more fully appreciated as the same becomes better understood when considered in conjunction with the accompanying drawings, in which like reference characters designate the same or similar parts throughout the several views, and wherein:
Fig. 1 illustrates a scheme for detection of an insoluble target (e.g., cross-linked RNA) in a biological sample.
Fig. 2 (A) illustrates quantitative nuclease protection assay (qNPA) of gene expression in a biological sample. Fixed tissues were lysed and the mRNA content in pelleted tissue sections and the supernatant was quantified using a qNPA assay using ARRAYPLATE. Frozen tissue samples were used as a positive control. B) illustrates the proportionality of the RNA content in the pellet versus supernatant of second lysed sample. C) provides a specific embodiment of A) for the analysis of an RNA target in a formalin-fixed, paraffin-embedded tissue.
Fig. 3 illustrates the applicability of the technique for the measurement of RNA in tissues.
Fig. 4 (A) schematic representation of ArrayPlate mRNA assay on FFPE colon. (B) illustrates a titration of frozen liver and formalin-fixed paraffin-embedded sections. A representative measurement is shown in the inset (right panel).
Fig. 5 (A) illustrates comparable gene expression in frozen and fixed liver. Panel (B) illustrates correlation of gene expression in frozen vs. FFPE liver.
Fig. 6 represents identical quantitative results from fresh fixed versus 18-years stored fixed samples.
Fig. 7 demonstrates linearity of ArrayPlate mRNA assay on FFPE Liver.
Fig. 8 illustrates relative gene expression in frozen vs. FFPE Liver.
Fig. 9 (A) illustrates reproducibility of ArrayPlate mRNA Assay on FFPE Liver and the sensitivity of the assay with respect to frozen vs. fresh samples. (B) illustrates validation of qNPA diagnostics in the measurement of viral nucleic acids.
Fig. 10 shows a list of genes that were analyzed in diffuse large B-cell lymphoma (DLBCL) gene expression assay.
Fig. 11 illustrates layout of the genes in an ARRAYPLATE DLBCL gene expression assay.
Fig. 12 illustrates the compatibility of the assay with respect to various media. The expression of various genes measured using a qNPA assay on freshly prepared samples, formalin-fixed paraffin-embedded samples, and OCT frozen samples was analyzed.
Fig. 13 illustrates a two-way correlation between freshly prepared samples, formalin-fixed paraffin-embedded samples, and OCT frozen samples with respect to the expression of various genes. Gene expression analysis was performed using a qNPA assay.
Fig. 14 illustrates testing of clinical biopsy tissue blocks using an ARRAYPLATE analysis. Four different biopsy samples were analyzed using three different biomarkers.
Fig. 15 illustrates reproducibility of the ArrayPlate mRNA assay for clinical biopsy tissue blocks.
Fig. 16 (A) illustrates results of gene expression from Array 1 (See, Figure 15). Panel (B) shows average normalized signal of gene expression in the four biopsy samples.
[0152] Fig. 17 (A) illustrates results of gene expression from Array 2 (See, Figure 15). Panel (B) shows average normalized signal of gene expression in the four biopsy samples.
Fig. 18 illustrates immunohistochemical (IHC) analysis of antigen expression in a diffuse large B-cell lymphoma sample (case 1). Samples were stained with haematoxylin and eosin (H&E), and analyzed for HLA-DR, BCL-6, BCL-2, CD-20, and BCL-68 expression.
Fig. 19 illustrates immunohistochemical (IHC) analysis of antigen expression in a diffuse large B-cell lymphoma sample (case 2). Samples were stained with haematoxylin and eosin (H&E), and analyzed for HLA-DR, BCL-6, BCL-2, CD-20, and BCL-68 expression.
Fig. 20 illustrates immunohistochemical (IHC) analysis of antigen expression in a benign reactive lymph node (LN) in case 3. Samples were stained with haematoxylin and eosin (H&E), and analyzed for HLA-DR, BCL-6, BCL-2, CD-20, and BCL-68 expression.
Figs. 21-23 summarize the results of Figs. 18-20 and show immunohistochemical analysis (IHC) of HLA-DR, BCL-6 and BCL-2 expression in the three biological samples. The findings of IHC were correlated to gene expression analysis of biopsy samples using an ARRAYPLATE qNPA assay.
Fig. 24 shows correlation between expression of HLA-DR, BCL-6, and BCL-2 at the protein (analyzed by immunohistochemistry) and RNA (analyzed using quantitative nuclease protection assay [qNPA]) level.
Fig. 25 expands the study of Fig. 24 to include CD20 and CD3 antigens.
Fig. 26 provides a representative example of the utility of quantative nuclease protection assay (ARRAYPLATE) for conduction gene expression studies. Rifampicin-induced modulation of the expression of various cytochrome P450 isoforms in (A) human hepatocytes or (B) canine hepatocytes were measured using a qNPA assay.
Fig. 27 summarizes the results of Fig. 26 by presenting the data in an expanded format. Genes that were significantly induced/suppressed by 5µM rifampicin are provided in the right panel.

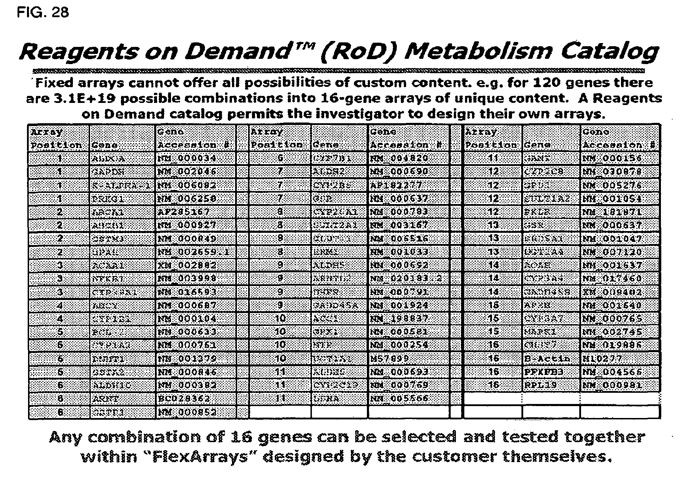

FIG 28. provides a representative example of a custom-designed array and the genes (with the GenBank accession numbers) contained therein.

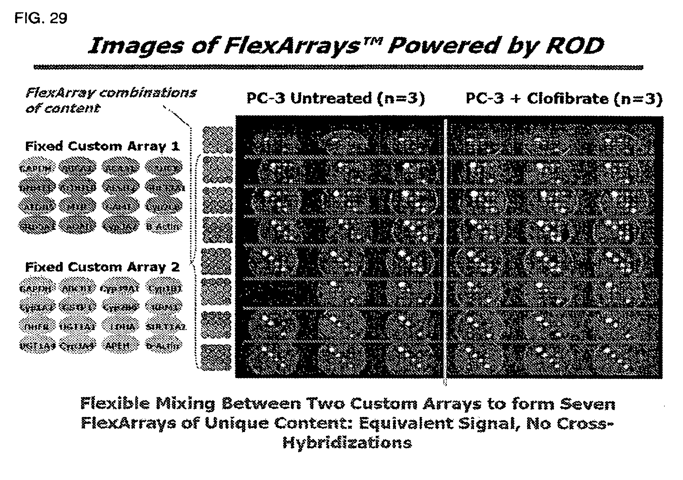

FIG. 29. illustrates results of gene expression from custom array 1 and 2 in control (untreated) and experimental (clofibrate-treated) PC-3 samples (n=3).

The prior art has referred to the need to extract RNA - solubilize it for PCR and microarray methods. References also indicate that the quality of this soluble, extracted RNA is critical to qualtiy of PCR or microarray result. Thus qNPA is unique because there is no need to extract.

### EXAMPLES

Fixed tissue (e.g. formalin fixed, paraffin embedded formalin fixed, or preservcyte fixed, an alcohol-based fixative) material was tested using a nuclease protection assay, but any assay where a hybridizing or binding probe is used to bind to the target oligonucleotide, and then recovered in a quantitative manner, could be used. Furthermore, one skilled in the art could conceive methods to measure cross-linked oligonucleotide where a probe is biosynthetically generated. Any type of fixative, so long as target oligonucleotide is not excessively degraded, can be used as sample material.

To demonstrate the measurement of oligonucleotide that is not extracted from the tissue (presumably cross-linked oligonucleotide), and not just soluble oligonucleotide, the sample was lysed and then centrifuged. The supernatant was separated from the pellet and then nuclease protection probes were added to each fraction, and they were processed separately by the protocol described below. This assay is referred to as the quantitative Nuclease Protection Assay (qNPA), but other hybridization and biosynthetic methods could be used where the probe produced can be separated from the target oligonucleotide and measured. The schematic chart for this assay is presented in Fig. 1.

### General Experimental Protocols

Analysis of pelleted lysate versus supernatant from formalin fixed tissue. The sample was lysed, centrifuged and then measurements made from the pellet and supernatant compared to a matched frozen sample. The nuclease protection assay measures the total RNA, irrespective of whether it is associated with the pellet and not extracted from the lysate (cross-linked) or the supernatant where it is extracted from the tissue (soluble). Thus the nuclease protection probe/target oligonucleotide duplex hybrids occur within the pellet. Panel A depicts for one sample, Panel B for another, demonstrating that the ratio of oligonucleotide in the supernatant versus the pellet can vary from sample-to-sample, yet this method, by measuring both pools, measures the total oligonucleotide in the sample.

Correlation between new and old paraffin block of diffuse large B-cell lymphoma. Quantitatively identical results were obtained from a fixed sample that had been archived 18 hears versus a freshly fixed sample prepared from a block of tissue frozen at the time the original sample was fixed 18 years ago. The data was normalized to housekeeper genes and then plotted to determine the R2 correlation coefficient between the 18 year fixed and freshly fixed measurements.

Diffuse Large-B-Cell Lymphoma cells were used to validate that quantitatively identical levels of gene expression are measured from fixed, fresh, and frozen tissue. Cell pellets were frozen in OCT prior to lysis, or lysed directly as a fresh sample, or were paraffin embedded and fixed and then lysed. Pair-wise comparison of the data (normalized to housekeeping genes) were used to determine the R² correlation coefficients between fixed versus fresh, fixed versus frozen, and frozen versus fresh samples. Thus, when a uniform population is analyzed, results are nearly identical regardless of whether or how sample was tested fresh, frozen or whether it was fixed and paraffin embedded.

Specificity of target oligonucleotide measurement. RNase and DNase experiments demonstrate that the ArrayPlate is detecting RNA but not DNA. Paraffin embedded formalin fixed tissue was lysed and denatured at 95°C, and then split into individual samples that were then treated with DNAse, RNAse or buffer. After this treatment the samples were subject to heat denaturation to kill the DNAse and RNAse, and then the nuclease protection probes were added and the nuclease protection protocol was carried out. The images demonstrate identical results between untreated and DNAse treated samples, and total loss of signal resulting from RNAse treatment, demonstrating he specific measurement of RNA from the fixed tissue using this protocol.

Comparison of results of the ArrayPlate qNPA nuclease protection assay and immunohistochemistry. The specific example is a nuclease protection assay, and more specifically the quantitative Nuclease Protection Assay. What is depicted is the hybridization of the nuclease protection probe to RNA that may be cross-linked to the tissue, as well as soluble RNA. The treatment with S1 destroys unhybriedized probe, reducing the probe to a stoichiometric level proportional to the amount of target oligonucleotide, both the cross-linked and soluble oligonucleotide. The addition of base with heating releases the nuclease protection probe from the oligonucleotide template and tissue, making it accessible for measurement on the programmed ArrayPlate. The rest of the protocol is carried out in a standard way, as described in more detail in the methods. Rat liver was cut in half, and half was frozen, half was fixed in formalin. The frozen tissue was weighed, lysed, and then dilutions were made for each sample tested. The fixed block was sliced into 5 micron sections, and these were tested as two slices, 1 slice, ½ slice, or ¼ slice per sample. Measurements were made using the nuclease protection assay, measuring RNA. The genes measured are indicated. The reproducibility of measurements was assessed by calculating the %CV for each gene, and the average %CV across all genes.

Vaginal swabs were collected from an HPV positive subject and preserved in Preservcyte. The HPV positive diagnosis was made by PAP smear and the hybrid capture test. An array was designed to measure the host RNA, viral RNA and viral DNA. The viral DNA was measured using a probe that hybridized to a sequence in the untranscribed portion of the gene. The sample was tested in replicate (n = 8) and the data was not normalized to housekeeping gene. The genes measured in the diffuse large B-cell lymphoma tissue experiments are listed in this table. For the figures shown Array was used.

Where a diffuse large B-cell lymphoma (DLBCL) is referred to it is the DB cell line (American Type Culture Collection, Manassas, VA), which is an MHC II-negative DLBCL cell line, or a DB transfectant, DB-CIITA-3.1 which includes a CIITA expression vector that induces expression of major histocompatibility class II genes including HLA-DRA, -DRB, - DPA, and -DQA.(Glinsmann-Gibson, 2006). The cells were grown in RPMI with 10% fetal bovine serum to a density of 4 million/mL. 16 million cells were centrifuged to create cell pellets that were either 1) made into formalin fixed paraffin embedded (FFPE) material by subjecting to 4 hours of fixation in formalin, routine overnight tissue processing on a TissueTek instrument, and paraffin embedding, or 2) made into snap-frozen material by snap freezing in liquid nitrogen quenched in isopentane, either with or without embedding media (optimal cutting temperature or OCT, Sakura Finetechnical Co, Torrance, CA). Rat liver was similarly frozen or prepared as FFPE material.

FFPE clinical tissue used for testing the performance of the ArrayPlate included 1 benign lymph node (reactive follicular hyperplasia) and 2 DLBCL (1 centroblastic, 1 immunoblastic) for which there was both multiple snap frozen and FFPE blocks. A new FFPE block was created from a previously snap-frozen block of benign lymphoid tissue from 1989 by thawing until cold, then immediately fixing in 4% formalin and processing as described below. The tissue was fixed in approximately 5 X 5 X 5mm portions.

Frozen and FFPE sections were cut at -5 microns thickness. For assay by nuclease protection the samples were placed into HTG lysis Buffer (25 µL/section), vortexed briefly, heated at 95ºC for 10 minutes, re-vortexed briefly, and then frozen at -70C until analysis.

The ArrayPlate nuclease protection assay has been described previously(Martel, 2002). Briefly, after cells or tissue have been lysed, denatured, and permeabilized by heating in HTG buffer as above, the frozen sample was tested. However, they can be tested immediately after lysis as well, and probes can be added at the time of lysis, or sampoesl can be frozen prior to the 95ºC heating step. The particulars of lysis and heating may differ according to the target molecule being measured. For instance, in some instances where the target oligonucleotide is DNA it may be necessary to heat the lysate to 105ºC. Other methods of lysis and disruption that would be familiar to one expert in the art or are common for measuring different types of target molecules or recovering measurement activity from fixed tissue may be used. Specifically in the case of these experiments to measure oligonucleotide targets the probes specific for the genes of interest were incubated with the samples 6 hours at 60°C, forming specific probe-RNA duplexes, then unhybridized probes and RNA were digested by S1 Nuclease. Next, alkaline hydrolysis destroyed the mRNA from the duplexes, leaving intact probes with concentrations proportional to the amounts of specific mRNA originally present. After neutralization, samples were then hybridized to the detection plates. Detection plates are formed from a set of 16 unique anchor DNA oligos spotted in a 4 x 4 grid on the bottoms of the wells of a 96-well plate. This universal array was programmed for the genes of interest by addition of 16 linker probes that contained sequence that binds a gene of interest on one end, and sequence that binds one of the anchor oligos on the other.

After hybridization, the sample probes were bound to the plate by the linker probes. The detection linker was added (though this can be added at the same time the sample is added to the programmed ArrayPlate), which contained sequence that bound the sample probe on the end not bound by the linker probe on one end, and common sequence that bound a detection probe on the other. Then the detection probe was added, which bound to all the detection linkers. The detection probe contained enzyme which acted on a chemiluminescent peroxidase substrate, added last.

The plates were imaged from the bottom by an Omix Imager and analyzed using Vuescript (HTG) which calculated average pixel intensity for all elements to determine expression levels for each gene. Where the data has been normalized, the expression levels were normalized to the housekeeping gene TBP at an arbitrary level of 1000.

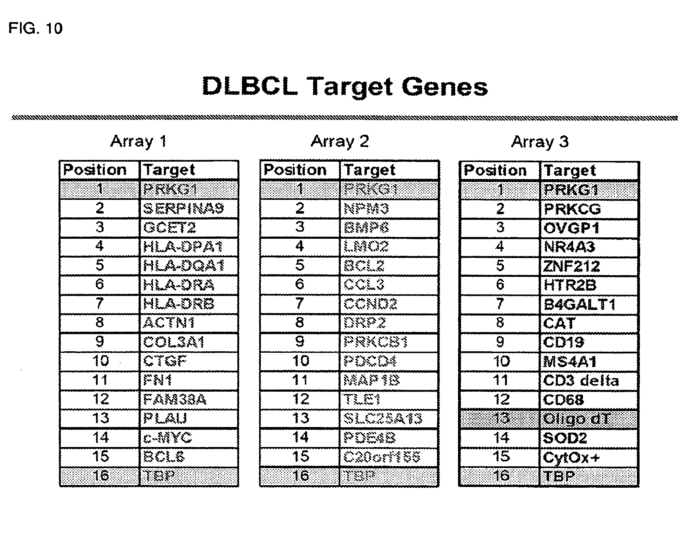

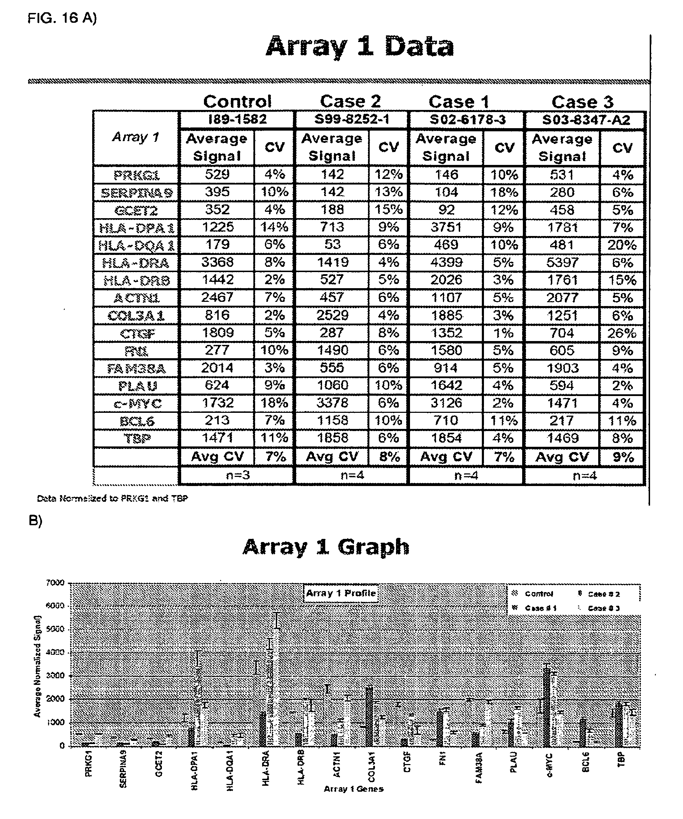

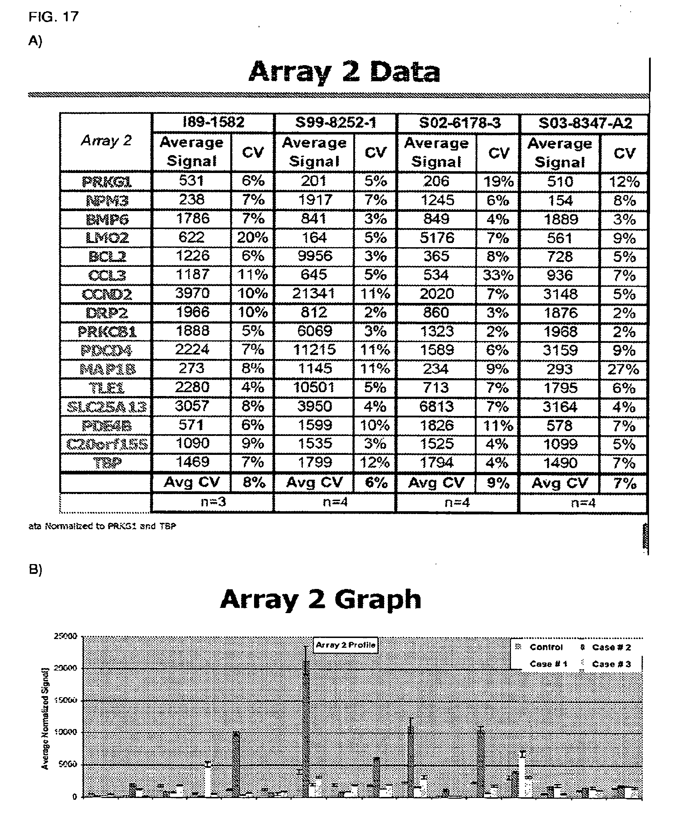

For the DLBL experiments the genes measured were those listed in Figure 10, though for the data shown is for Array 1 and 2 (Figures 16 and 17). Because of the heterogeneity of cellular composition in human tumor samples, we included probes designed to test the tumor composition for B-cells (CD19, CD20), T-cells (CD3) and histiocytes (CD68). Finally, 2 housekeeping genes, TBP and PRKG1 were chosen based previously published work assessing the utility of different endogenously expressed genes as housekeeping genes, which identified these 2 genes as very stably expressed at low or moderate levels in different types of lymphomas.(Lossos, 2003). These two housekeeping genes were repeated in each of the three arrays.

IHC was performed for the protein products for genes present on the array for which we had clinical IHC assays routinely performed in paraffin. These included CD20, BCL2, and HLA-DR. All staining was performed on the Ventana Benchmark XT instrument with Ventana I-View detection (Ventana Medical Systems Incorporated, Tucson, Arizona)(VMSI). Standard clinical laboratory staining procedures with on-instrument antigen retrieval were employed. Monoclonal antibodies were used against CD20 (VMSI, clone L26), BCL2 (VMSI, clone B4-2/100/D5), and HLA-DR (Biogenix, clone LN3). Photography was performed on a Labophot-2 microscope using a 10X eyepiece and a 40X/0.65 objective lens (Nikon, Melville, NY). A SPOT-RT 2.2.0 color camera and SPOT Advanced 4.0.9 software (Diagnostic Instruments, Sterling Heights, MI) were used to capture and digitally acquire images, which were then inserted into PowerPoint 10 (Microsoft, Redman, WA) for processing.

### Results:

To demonstrate that nuclease protection proceeds *in situ* in a fixed sample, formalin-fixed paraffin-embedded (FFPE) rat liver (5 µm sections) was used as a starting material. Tissue sections were treated with 30 µl of lysis buffer. Following lysis, nuclease protection probes was added onto the sample, followed by heat denaturation (95ºC, 10 min). Samples were centrifuged and the supernatant was removed. Tissue pellets were treated with 30 µl of lysis buffer and incubated with nuclease protection probes. After heat denaturation (95ºC, 10 min), qNPA assay was performed on the pellet and the supernatant using an ARRAYPLATE, which provides a substrate. Frozen tissue was used as a control. The assay circumvents the artifacts of mRNA extraction from formalin-fixed materials.

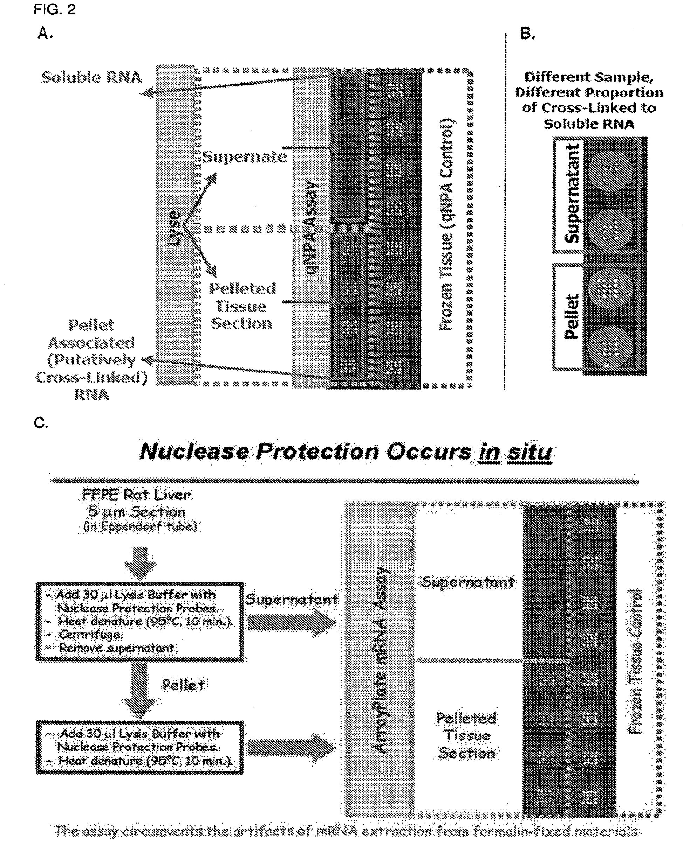

These results are presented in Fig. 2. The supernatant and the pelleted tissue sections were compared to matched frozen tissue. The results show that the target oligonucleotide is measurable from the pellet. In Panel A, the majority of the luminescence counts are from the pellet, not the supernatant. In contrast, in a different sample (Panel B) there are significant levels of target oligonucleotide measured from the supernatant as well. In this experiment the target oligonucleotides were RNA, but they could as well have been DNA, microRNA, or ribosomal RNA, for example. Fixation cross-links oligonucleotides to the tissue. The measurement of oligonucleotide associated with the pellet suggests that the cross-linked oligonucleotide is being measured without solubilization, the same pool as measured by *in situ* methods (where the RNA is labeled and visualized in the tissue), and that the sum of the measurement (when the supernatant is not separated from the pellet) provides a measure of the total target oligonucleotide in the sample. The difference between the samples tested in Panel A and B suggests that the ratio of cross-linked to soluble oligonucleotide can vary, perhaps due to differences in the fixation protocol, even if the samples are otherwise identical. Thus measuring just the cross-linked pool, or just the soluble pool may not accurately measure the level of target oligonucleotide in the sample.

Panel (C) provides a detailed description of the experimental protocol used in this study. The results demonstrate that the nuclease protection step in the qNPA assay occurs *in situ* while the detection step can be *ex situ, in situ* or both.

### Analysis of gene expression in formalin-fixed paraffin-embedded samples

Formalin-fixed paraffin embedded colon tissue was homogenized in lysis buffer as described previously and treated with buffer alone, or buffer supplemented with RNAase, or buffer supplemented with DNAase. The pellets were subjected to a qNPA assay as previously described. Approximately 0.4 sections/well was tested. These studies show the applicability of the technique for the measurement of RNA in tissues.

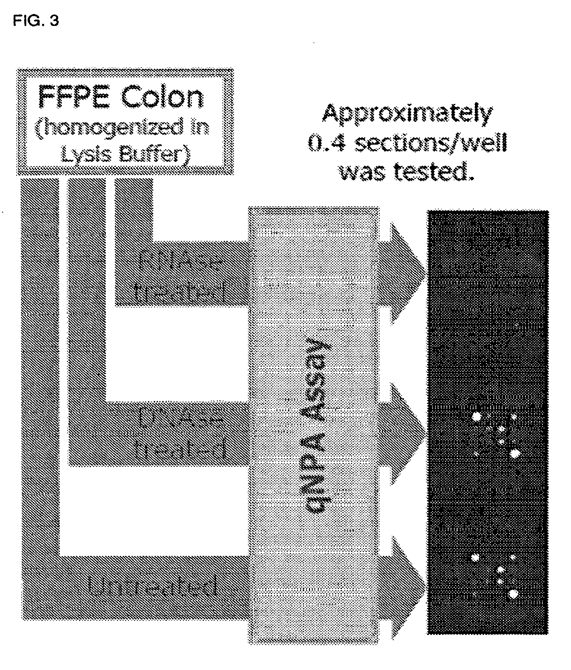

Results of this experiment are shown in Fig. 3. RNAase treatment, which eliminates the target RNA from the homogenized samples, results in loss of viable target from the sample. Treatment of the sample with a DNAase did not have any significant effect on the qNPA assay.

### Quantitative nuclease protection assay allows for quantitative determination

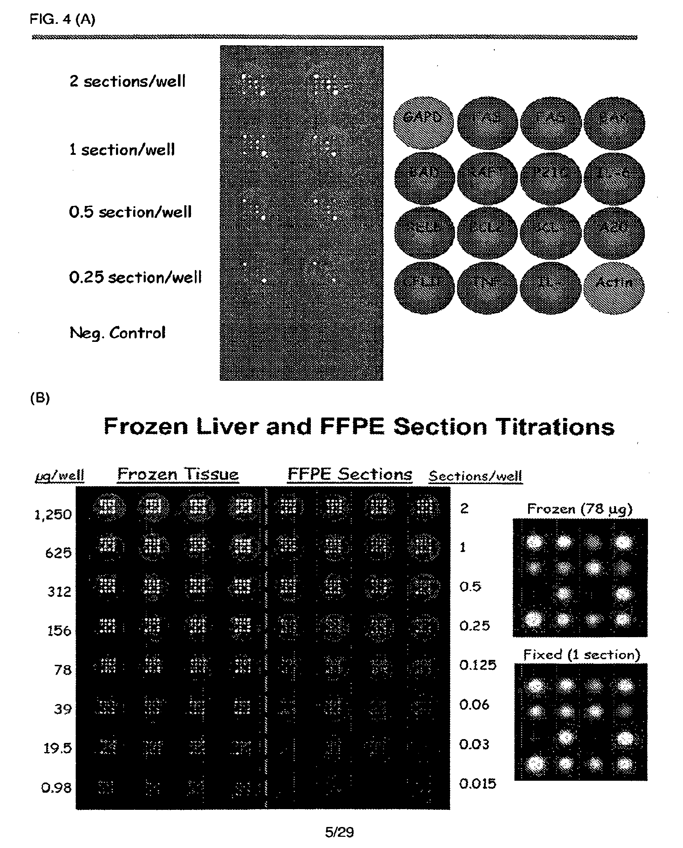

To evaluate quantitative nature of the nuclease protection assay used herein, FFPE sections were titrated against an identical frozen tissue using qNPA assay. RNA was isolated from frozen tissue and loaded onto the slides. FFPE sections were loaded in a similar manner. The schematic representation of ArrayPlate mRNA assay on FFPE colon is provided in panel A. The result of this study, which is presented in Fig. 4 (A) and (B), illustrates a correlation of gene expression in frozen tissue vs. FFPE liver. A representative example is highlighted in the right side of panel (B). As can be seen from this Figure, the individual expression profiles of various genes, as identified from a frozen sample or a fixed sample, visually correlate well with one another.

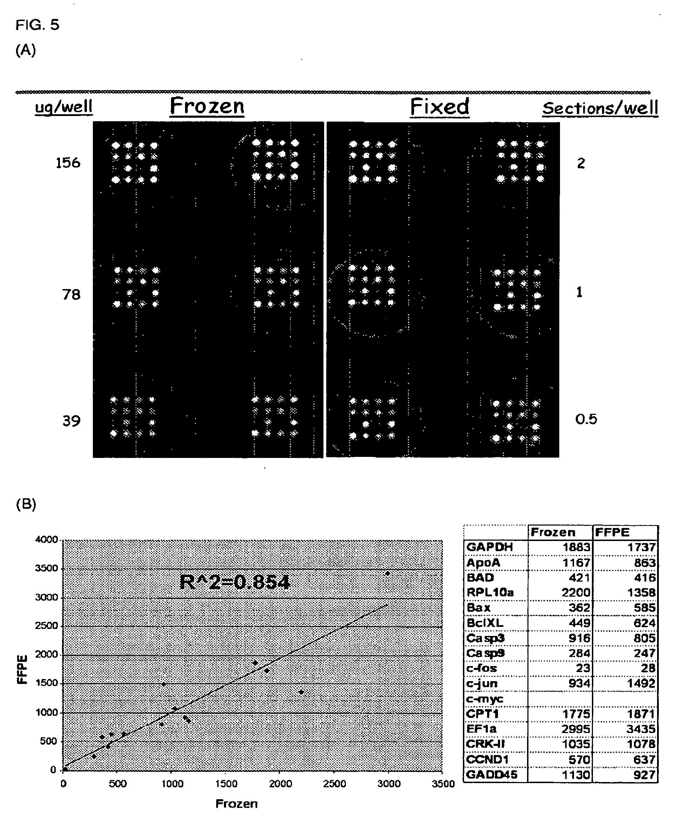

To further quantitate these results obtained from different biological samples, a regression analysis of the expression profile of various genes in frozen versus FFPE sectioned tissues was performed. The same set of genes were measured across all samples, and the data was normalized to housekeeping genes and then plotted to determine the R² correlation coefficient between each pair-wise comparison. Results of the regression analysis are shown in Fig. 5(B). A strong correlation (R²=0854) was observed between the two samples. These results essentially indicate that equivalent quantitative results are obtainable from freshly fixed tissue as with the frozen tissue.

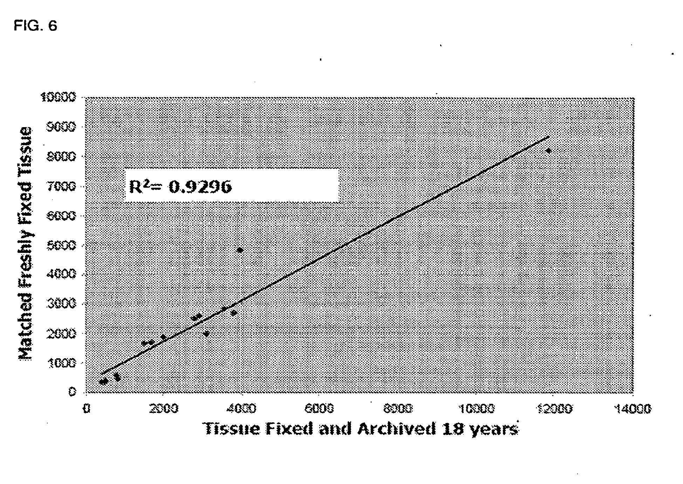

While the measurement of RNA by *in situ* hybridization (where the RNA is labeled and visualized in the tissue) has been found to be comparable whether freshly fixed tissue is used or tissue that was fixed and then stored for many years is used, this has typically not been the case for PCR or hybridization methods that are not performed *in situ.* This limits the utility, since there are vast stores of archived materials that could otherwise be used for retrospective studies to identify and validate biomarkers and target genes, or for development and validation of a monitoring, prognostic, or diagnostic assay, or for the association of safety with gene expression, or for the understanding of disease processes, etc. A lymph node tissue that was obtained from a cancer patient 18 years previously was tested. A portion had been fixed at that time for histology, and another portion was frozen as a block of tissue. The patient was diagnosed to have diffuse large B-cell lymphoma. Portions of the frozen block were fixed to provide freshly fixed sample for comparison to the 18 year archived fixed material. A set of genes were measured from each sample, with the results depicted in Fig 6. The relative luminescence values after normalization to housekeeper genes are depicted. The R² correlation coefficient was 0.93, indicating essentially equivalent quantitative results were obtained from the freshly fixed tissue as from the tissue that had been fixed 18 years previously.

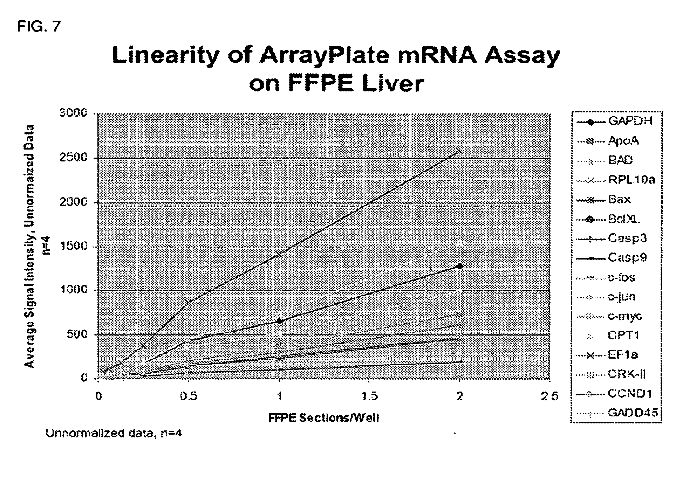

To measure the relationship between the dependency of qNPA signal on the amount of starting material, a gene expression study was conducted using FFPE sections as characterized previously. Comparisons were made with non-normalized qNPA signals obtained with various amounts of sample (n=4 for each sample amount). A linear relationship between sample size and qNPA signal was obtained for samples containing at least 0.5 FFPE sections/well. These results were consistently observed in all of the genes analyzed. These results of this study, along with the types of genes analyzed, are presented in Fig. 7.

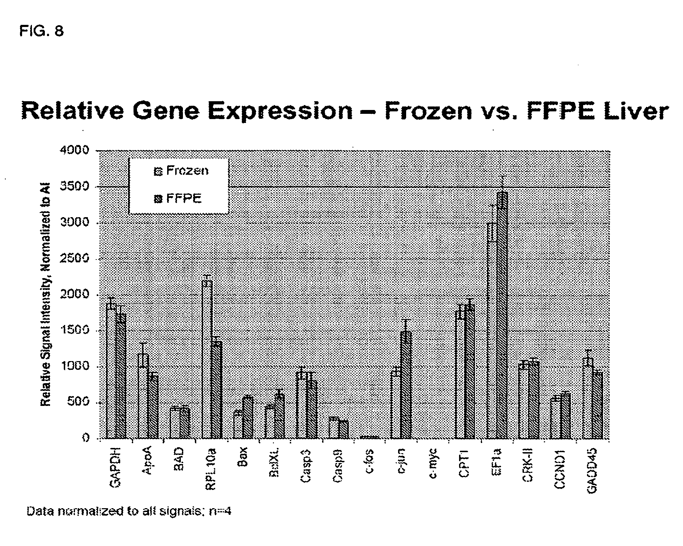

To quantitatively evaluate the levels of gene expression in freshly frozen vs. FFPE liver samples, a comparative analysis of 16 genes was performed using a qNPA assay. The difference in the expression profile of a majority of these genes between the two sample preparations (i.e., frozen vs. FFPE) was virtually indistinguishable. The results of this study are presented in Fig. 8.

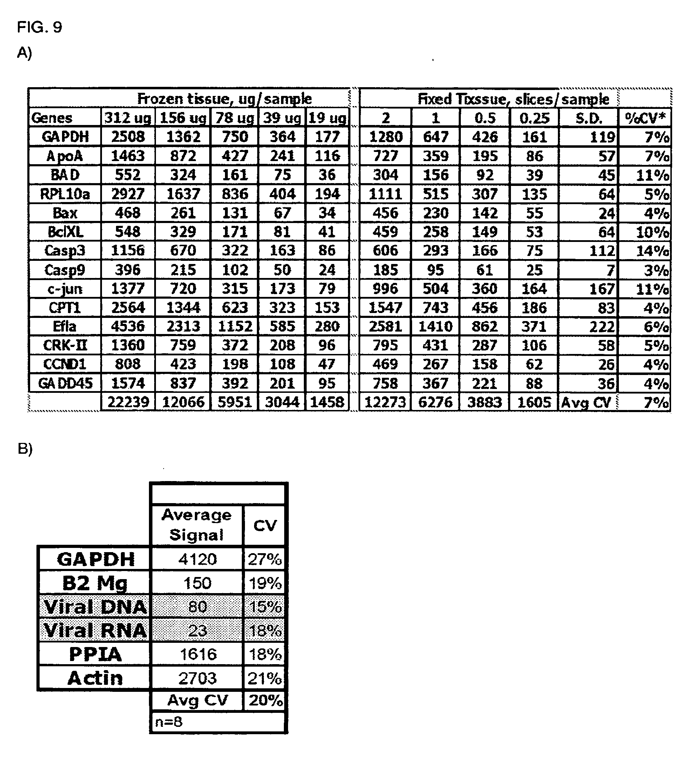

The method described herein allows for a sensitive measurement of the target oligonucleotide. The table (A) provided in Fig. 9 compares the measurement of RNA by the nuclease protection assay from dilutions of frozen rat liver versus the different numbers of -5 micron thick slices of matched formalin fixed liver samples. Note that all the genes are measurable using just 1/4^{th} of a slice of tissue. In addition, the reproducibility of measurement was excellent, providing an average CV of 7% across all the genes measured. This reproducibility was no worse then the reproducibility of gene measurement from the frozen matched samples. Lastly, 1/4^{th} of a slice produced a similar number of luminescence counts for each gene and in total as did 19 µg of frozen tissue, providing an indication of how sensitive the measurement of RNA from fixed tissue can be using this method. Measurement of RNA from fixed tissue by PCR and other hybridization-based methods has proven to be very insensitive, requiring large amounts of tissue. It is likely that the sensitivity of the nuclease protection method is a result of measuring the total RNA, not just the soluble pool, and thus this same sensitivity would be obtain by any method that measures the cross-linked target oligonucleotide.

As indicated in Table (B) of Fig. 9, a variety of target genes can be reliably detected using this technique. The reproducibility of measurement was excellent, providing an average CV of less than 20% across all the genes measured.

Other nucleotide target species can be measured, and samples fixed using other types of fixation method can be successfully tested. Clinical vaginal samples were collected and preserved in Preservcyte prior to testing. The nuclease protection assay measured the host cell RNA, viral RNA and viral DNA. To differentiate the viral DNA from RNA present in the sample the nuclease protection probe was designed to hybridize to a sequence in the untranscribed portion o the gene. The data are the unnormalized intensities, and associated %CV determined from the measurement of eight replicate samples. What is demonstrated is the ability to measure host RNA, viral RNA, and viral DNA, demonstrating the measurement of multiple types of oligonucleotide targets from a fixed sample, as well as the ability to actually measure these target oligonucleotides from the same sample simultaneously within the same well of the ArrayPlate.

These figures depict the concept of measurement using a probe that associates with cross-linked oligonucleotide target as well as soluble target, and which can then be separated and measured. The specific example is a nuclease protection assay, and more specifically the quantitative Nuclease Protection Assay, but those skilled in the art can devise other methods including methods that utilize a probe that is generated by biosynthetic means, or a probe that may associate through methods other than oligonucleotide hybridization, to produce the same measurement of cross-linked oligonucleotide target. What is depicted is that using the standard protocol, the same as for fresh or frozen tissue, the oligonucleotide targets within fixed tissue can be measured. What is shown is the hybridization of the nuclease protection probe to RNA that may be cross-linked to the tissue, as well as soluble RNA. The treatment with S1 reduces the probe to a stoichiometric level, proportional to the amount of target oligonucleotide, both the cross-linked and soluble oligonucleotide. The addition of base with heating releases the nuclease protection probe from the oligonucleotide template and tissue, making it accessible for measurement by a means other than an *in situ* method. Note, however, that the probe (or one or a few from among a larger set of probes) could have first been measured *in situ.* The rest of the protocol is carried out in a standard way, as described in more detail in the methods.

Sequential measurement of probe *in situ* and after tissue lysis is possible. Once the excess probe is washed away, or after treatment with S1 nuclease, the target-associated probe can be visualized, using for instance a fluorescent tag or luminescence, and quantified *in situ.* If this is done specifically for one or for two or more probes that can be differentiated based for instance by use of probes with different emission wavelengths, then the levels of these targets measured *in situ* can be used to normalize or otherwise help interpret the measurements of a larger set of probes made after lysis for a set of targets. In this way spatial or "contextural" information can be related to the measurement of genes even after the spatial information has been lost. In the case where the probe(s) is simply washed away before *in situ* measurement, the probe(s) can be designed with a non-target hybridizing sequence overhang that is instead complementary to an oligo-conjugated detection probe, and thus can be labeled with that detection probe. Alternatively, the *in situ* probes can be directly labeled with a detection molecule or a group capable of binding a detection molecule or complex, such as biotin and then a washing and/or S1 step used before *in situ* measurement. Such labeling does not affect the ability of the nuclease protection probes to hybridize to their target oligonucleotide.

There are many instances where the ability to fix a sample before measurement of target oligonucleotides would be an advantage. For instance, if blood were to be collected directly into fixative, fixing and preserving the levels of target oligonucleotide, then the time between when the sample is drawn and when it is tested becomes less important. Handling becomes easier. Furthermore, fixed samples can be processed by methods that might disrupt or lyse cells and tissues otherwise, leading to a change or loss of target oligonucleotide, such as by filtration, simplifying sample preparation. The same is true for many types of cell, tissue, or whole organism samples as well as environmental samples, or where the collection of samples is distant from the point of testing.

### Example 2: Clinical studies

Diffuse Large B Cell Lymphoma (DLBCL) is the most common of the aggressive lymphomas, accounting for nearly 40% of lymphomas overall. The clinical International Prognostic Index (IPI) score for DLBCL helps to stratify patients into risk categories based on clinical features (Shipp, 1993). However, even within categories, patient outcome is variable. Because of the variable patient outcome, it has long been suspected that DLBCL may actually encompass more than 1 disease. In recent years, the results of extensive gene expression profiling (GEP) experiments have confirmed that DLBCL is not a uniform disease entity and that differences in GEP-defined subtypes can be related to patient survival. Three different sets of genes have been identified based on the work of 3 different research groups.

The first group studied 240 DLBCL using the LymphoChip. This chip was a spotted oligonucleotide microarray containing numerous elements known to represent genes expressed by B or T lymphoid cells, genes involved in the immune response, or genes expressed by lymphoma and leukemia cell lines. (Alizadeh, 1999). The investigators discovered 4 gene expression signatures highly correlated to patient survival called "germinal center," "major histocompatibility (MHC) class II," "lymph node," and "proliferation." Representative genes from these 4 signatures with the addition of the gene BMP6 were used to create a 17-gene outcome predictor score, which provide additional prognostic value independent of the clinical IPI score (Rosenwald, 2002). Another group used the Affymetrix high density oligonucleotide array platform and a supervised learning classification approach to develop a DLBCL outcome predictor score with best accuracy obtained using a set of 13 genes.(Shipp, 2002) Another group of investigators performed a meta-analysis of the literature for genes that were previously reported to be significantly associated with survival in DLBCL. Using quantitative RT-PCR, these researchers assessed a series of 66 DLBCL and determined the 6 most predictive genes (LM02, BCL6, FN1, CCND2, SCYA3, and BCL2).(Lossos, 2004) Only 2 genes were identified in both the Lymphochip and RT-PCR papers (BCL6 and FN1) for a total of 34 genes previously identified in high profile papers as being significantly associated with patient outcome in DLBCL. Through analysis of the Lymphochip data set, identification of several redox related genes highly correlated with patient survival had been identified and a "Redox Score" including the representative genes, manganese superoxide dismutase and catalase had been created (Tome, 2005). All 4 of these papers used quantification of gene expression key set of genes that correlate with survival in DLBCL. Each came up with a small gene set with strong correlations to outcome in their particular set of cases. All used techniques that relied on snap frozen materials as the basis of analysis, making comparisons between the gene sets or expansion to wider groups of patients difficult. Thus, a comparison of these gene sets, in particular using a method that is applicable to paraffin embedded samples is the next step in determining the clinical utility of these prognostic gene studies.

Quantitative nuclear protection assays hold the promise of quantifying mRNA without the difficult steps of mRNA extraction followed by quantitative RT-PCR and therefore may be applicable to formalin fixed, paraffin embedded tissues. The method relies on ArrayPlate Assay that has been described previously for pharmaceutical applications (Martel, 2002). In this assay, tissues are lysed in 96 well plates. Probes designed for the genes of interest are incubated with the lysate to allow hybridization of specific probe-RNA duplexes. Unhybridized probes and RNA are digested and alkaline hydrolysis is used to separate the mRNA from the duplexes, leaving intact probes with concentrations proportional to the amounts of specific mRNA originally present. These remaining probes are then transferred to a second 96 well plate which use linker and detection probes and chemiluminescent detection and quantification. The platform is easy to perform and has the potential to handle high sample volume.

Using this technique, an assay was developed for all 36 genes of interest as well as housekeeping genes and genes aimed at determining the cellular composition of the sample (B cell, T cell, and macrophage genes). Several studies for testing and validation of the performance of the assay in both frozen and paraffin embedded materials was performed. The paraffin blocks from DLBCL cases which had been previously analyzed in the paper by Rosenwald et al. were re-used for the determining whether the technique could similarly quantify the prognostically significant genes and whether this would correlate with patient outcome. The overall goal was to develop and validate an assay that would be useable for outcome prediction in all patients with DLBCL, not limited to the fortunate few with available snap-frozen tissue. Comparison of frozen samples to paraffin-embedded, new paraffin blocks to nearly 20 year old paraffin blocks, and evaluation of the relationship between the results and patient hazard ratio of death in a previously published set of 40 patients was conducted. These studies demonstrate the usefulness of this technique especially as applied to archived materials with consequent far-reaching applications for the field.

### Patient and cell line materials, preparation of FFPE and frozen blocks:

To validate the design of the ArrayPlate, a DB cell line (American Type Culture Collection, Manassas, VA), which is an MHC II-negative DLBCL cell line, was used. Additionally, a recently created DB transfectant, DB-CIITA-3.1 which includes a CIITA expression vector that induces expression of major histocompatibility class II genes including HLA-DRA, -DRB, -DPA, and -DQA.(Glinsmann-Gibson, 2006), was also utilized. The cells were grown in RPMI with 10% fetal bovine serum to a density of 4 million per ml. 16 million cells were centrifuged to create cell pellets that were either (1) made into FFPE material by subjecting to 4 hours of fixation in formalin, routine overnight tissue processing on a TissueTek instrument, and paraffin embedding, or (2) made into snap-frozen material by snap freezing in liquid nitrogen quenched in isopentane, either with or without embedding media (optimal cutting temperature or OCT, Sakura Finetechnical Co, Torrance, CA).

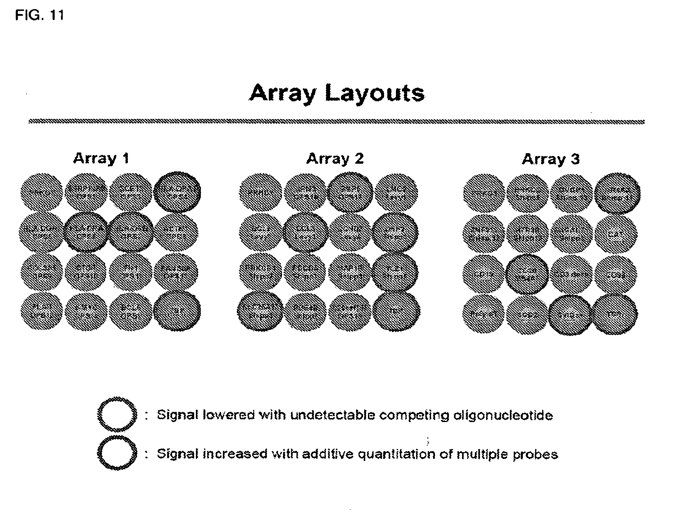

FFPE tissue used for testing the performance of the ArrayPlate included 1 benign lymph node (reactive follicular hyperplasia) and 2 DLBCL (1 centroblastic, 1 immunoblastic) from the hospital files chosen for having both multiple snap frozen and paraffin blocks, and containing nearly 100% tumor. A new paraffin block was created from a previously snap-frozen block of benign lymphoid tissue from 1989 by thawing until cold, then immediately fixing in 4% formalin and processing as described below. The arrangement of the array is described in Fig. 11.

To investigate whether the ArrayPlate results on FFPE samples are sufficiently similar to GEP discovery results from snap frozen tissue, samples which had been previously analyzed by GEP were used. Previously, 45 snap frozen samples to the LLMPP study of DLBCL had been contributed. Of these, 24 were included in the NEJM paper by Rosenwald et al describing the clinical utility of an OPS in reflecting patient survival in de novo DLBCL. Of these cases, 18 paraffin blocks were accessed. Also utilized were paraffin blocks on 21 cases profiled by the LLMPP but not included in the de novo DLBCL comprising 9 transformed and 12 relapsed DLBCL cases.

### Sample Preparation for ArrayPlate

Frozen and FFPE sections were cut at 5 microns thickness and immediately placed into the lab Buffer (25 µL/section), vortexed briefly, heated at 95C for 10 minutes, re-vortexed briefly, and then frozen at -70ºC until analysis. 1 cut of tissue was used per well on the ArrayPlate. Samples were from incisional biopsies which are fixed in approximately 5 X 5 X 5mm portions. It was important to cut the paraffin blocks into thin sections, as FFPE tissue not thinly cut gave poor results.

### ArrayPlate Assay

The ArrayPlate Assay has been described previously (Martel, 2002). Briefly, after cells or tissue have been lysed, denatured, and permeabilized by heating in the lab buffer as above, the frozen sample was sent to the lab for analysis. At the lab, the probes specific for the genes of interest were incubated with the samples 6 hours at 60ºC, forming specific probe-RNA duplexes, then unhybridized probes and RNA were digested by S1 Nuclease. DLBCL genes are listed in the table of Fig. 10. Next, alkaline hydrolysis was utilized to separate the mRNA from the duplexes, leaving intact probes with concentrations proportional to the amounts of specific mRNA originally present. After neutralization, samples were then hybridized to the detection plates. Detection plates are formed from a set of 16 unique anchor DNA oligos spotted in a 4 x 4 grid on the bottoms of the wells of a 96-well plate. This universal array was programmed for the genes of interest by addition of 16 linker probes that contained sequence that binds a gene of interest on one end, and sequence that binds one of the anchor oligos on the other. Three separate sets of linker probes were used, at the rate of 3 wells per assay, for the genes of interest.

After hybridization, the sample probes were bound to the plate by the linker probes. The detection linker was added, which contained sequence that bound the sample probe on the end not bound by the linker probe on one end, and common sequence that bound a detection probe on the other. Then the detection probe was added, which bound to all the detection linkers. The detection probe contained enzyme which acted on a chemiluminescent peroxidase substrate, which was added at the end.

The plates were imaged from the bottom by an Omix Imager and analyzed using Vuescript (the lab) which calculated average pixel intensity for all elements to determine expression levels for each gene. Expression levels were normalized to the housekeeping gene TBP at an arbitrary level of 1000.

Some wells contained spots where the gene expression of a few elements was out of the range of the rest of the spots in the well. If the range was too high, the signal was decreased with known quantities of undetectable competing oligonucleotides, which could bind the sample probe but not the plate. If the range was too low, the signal was increased by the use of multiple sample probes for the same gene, with different sequences for the part that binds sample RNA, and the same sequence to bind the linker probe.

### Selection of genes

As described in the last paragraph of the Introduction, key genes identified as prognostically important in 4 previous prominent papers in DLBCL were used. These genes accounted for 36 genes of interest (Lossos, 2004; Rosenwald, 2002; Shipp, 2002; Tome, 2005). Because of the heterogeneity of cellular composition in human tumor samples, also included were probes designed to test the tumor composition for B-cells (CD19, CD20), T-cells (CD3) and histiocytes (CD68). Finally, two housekeeping genes, TBP and PRKG1 were chosen based previously published work assessing the utility of different endogenously expressed genes as housekeeping genes, which identified these 2 genes as very stably expressed at low or moderate levels in different types of lymphomas.(Lossos, 2003) These 2 housekeeping genes were repeated in each of the 3 wells used to create the assay. An oligo dT probe was added in order to assess the quantity of mRNA in the sample (since an oligo dT probe should detect all mRNA which has a poly-A tail). However, for technical reasons this probe was non-functional and not further utilized. A cytochrome oxidase probe was also initially included because it is coded in mitochondrial DNA, and should be expressed at high levels. This turned out to bind both DNA and RNA, and so gave an extremely bright and generally oversaturated signal and was therefore not further considered, except that it could be used to distinguish whether there was insufficient material for the assay, or whether, if it had disappeared entirely, the sample was too degraded for use. These genes are listed in the table of Fig. 10.

### Immunohistochemistry and photography (IHC)

IHC was performed for the protein products for genes present on the arrays. Clinical IHC assays had been routinely performed for these genes. These included CD20, CD3, CD68, BCL2, BCL6, and HLA-DR. All staining was performed on the Ventana Benchmark XT instrument with Ventana I-View detection (Ventana Medical Systems Incorporated, Tucson, Arizona)(VMSI). The standard clinical laboratory staining procedures with on-instrument antigen retrieval were employed. Monoclonal antibodies were used against CD20 (VMSI, clone L26), CD3 (VMSI, clone PS1), CD68 (VMSI, clone KP1), BCL2 (VMSI, clone B4-2/100/D5), BCL6 (clone IG191E/AB), and HLA-DR (Biogenix, clone LN3). Photography was performed on a Labophot-2 microscope using a 10X eyepiece and a 40X/0.65 objective lens (Nikon, Melville, NY). A SPOT-RT 2.2.0 color camera and SPOT Advanced 4.0.9 software (Diagnostic Instruments, Sterling Heights, MI) were used to capture and digitally acquire images, which were then inserted into PowerPoint 10 (Microsoft, Redman, WA) for processing.

### Statistical methods:

Statistical analyses were performed on the 18 cases with results from both ArrayPlate and Affymetix/Lymphochip analysis of gene expression. Spearman rank-order correlations for each pairwise comparison of 3 studied methods were derived for each gene. For a given comparison, analyses with fewer than 10 available observations were not performed due to power considerations. The median overall correlation between available genes for each pairwise comparison was computed; only genes with available data for all 3 pairwise comparisons were included to avoid bias. Univariate analysis results (hazard ratios, 95% confidence intervals, and p-values) of gene expression as measured by array plate on overall survival were obtained from the Cox regression model.(Cox DR. Regression models and life tables. J Royal Stat Soc B. 1972;B34:187-220.)

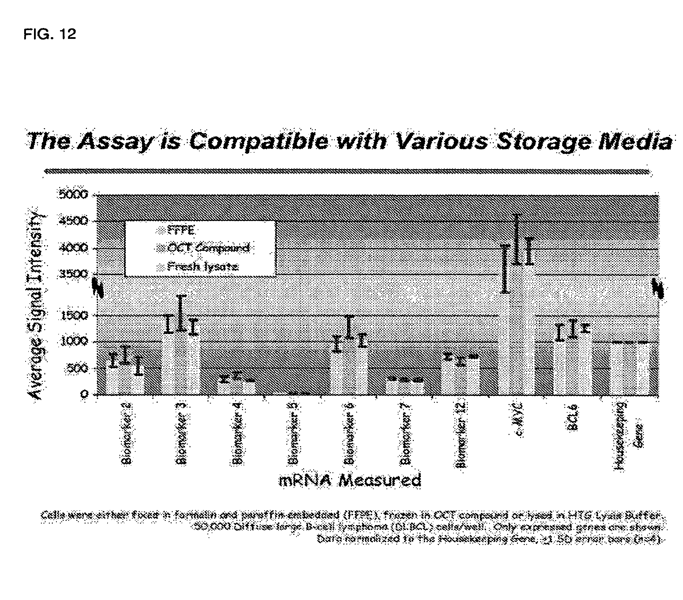

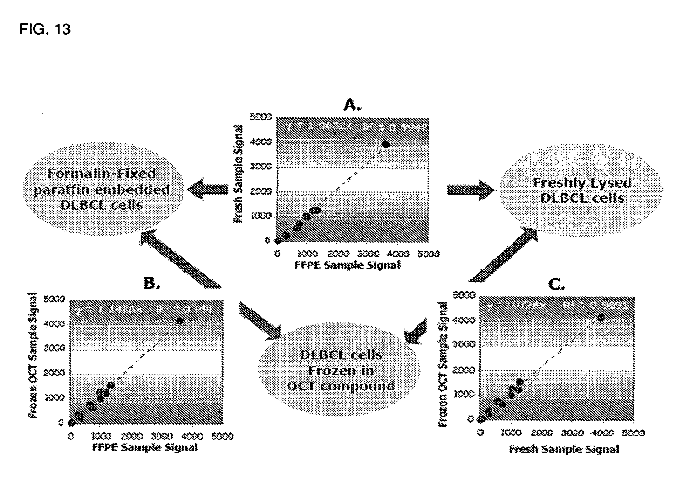

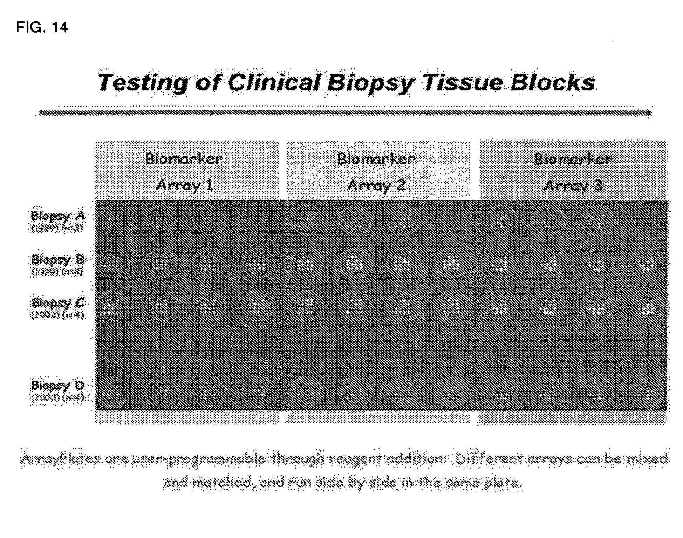

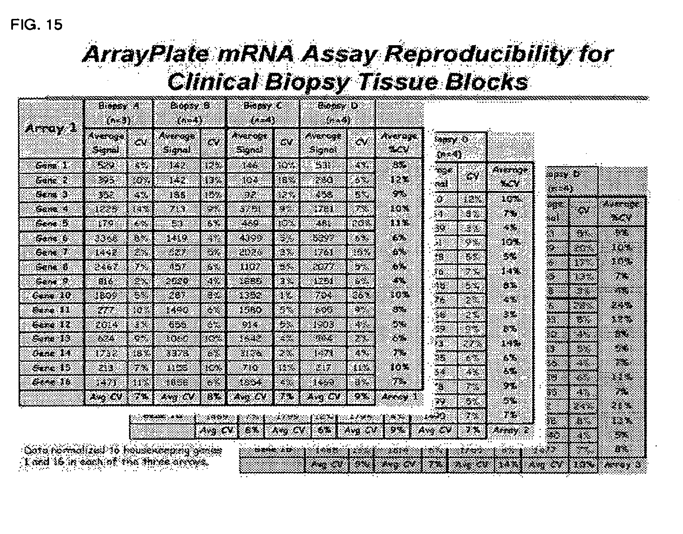

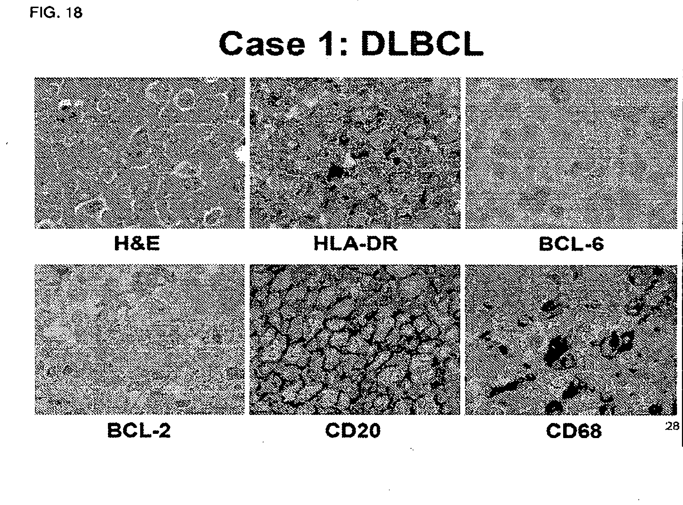

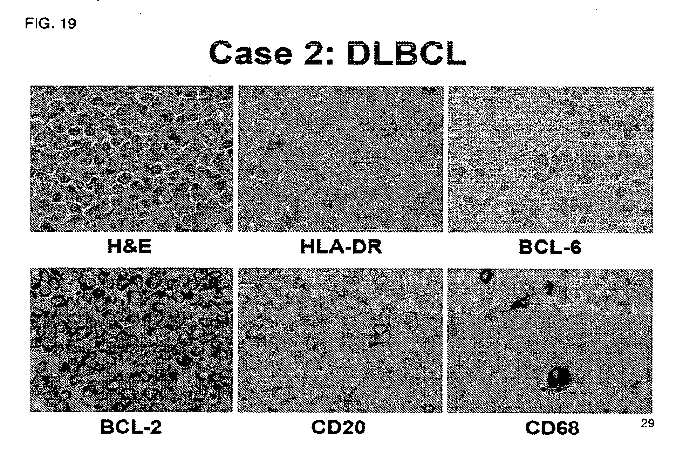

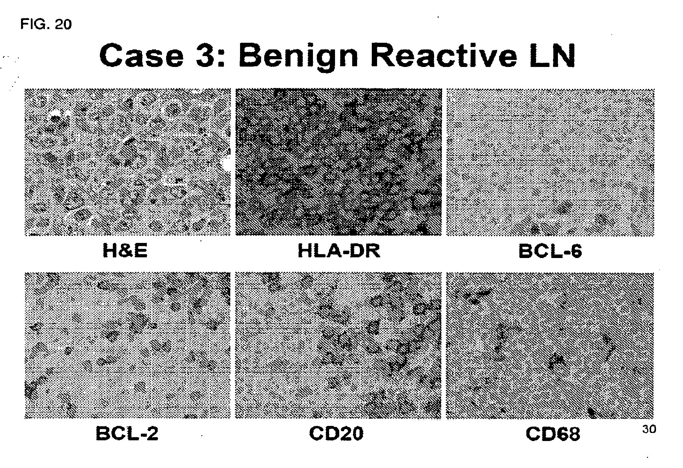

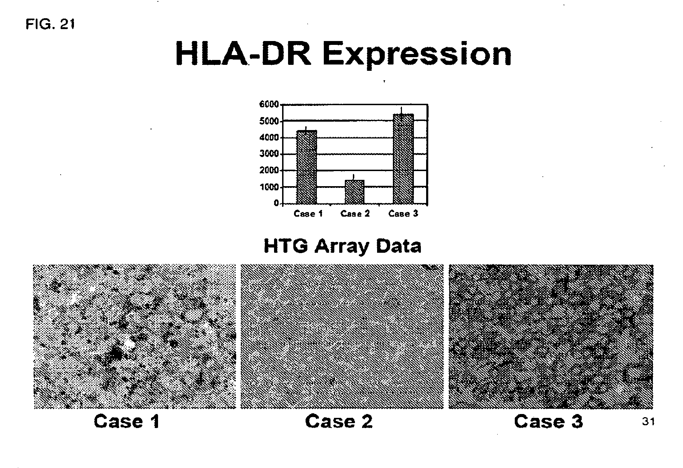

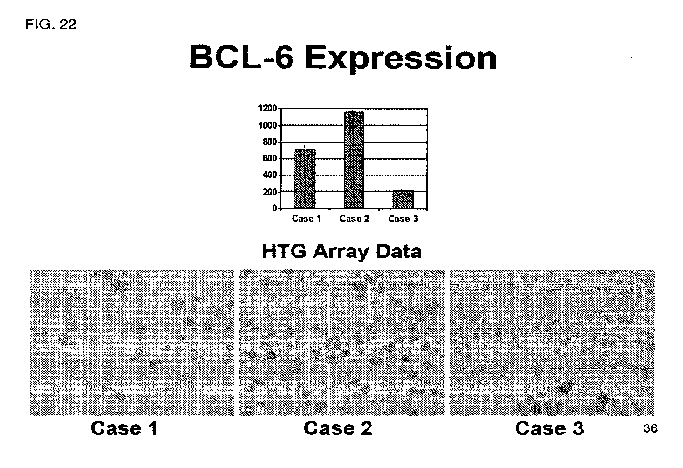

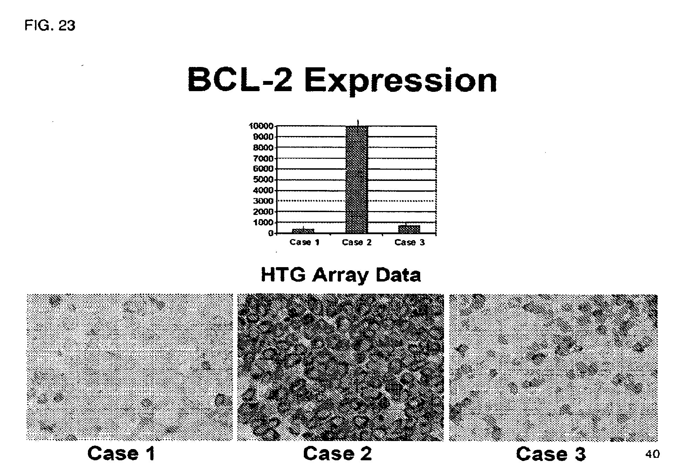

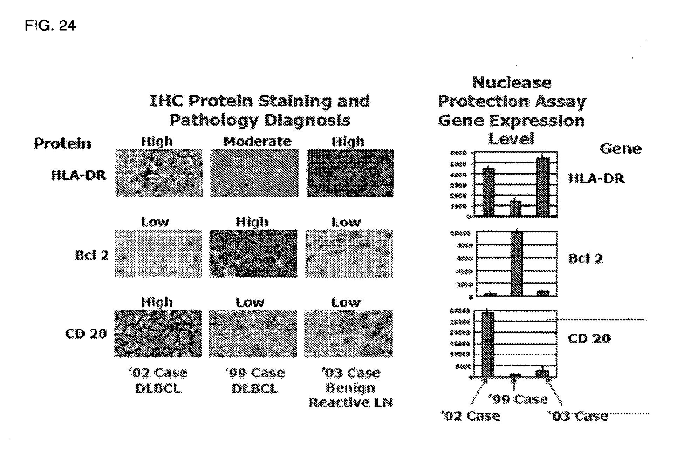

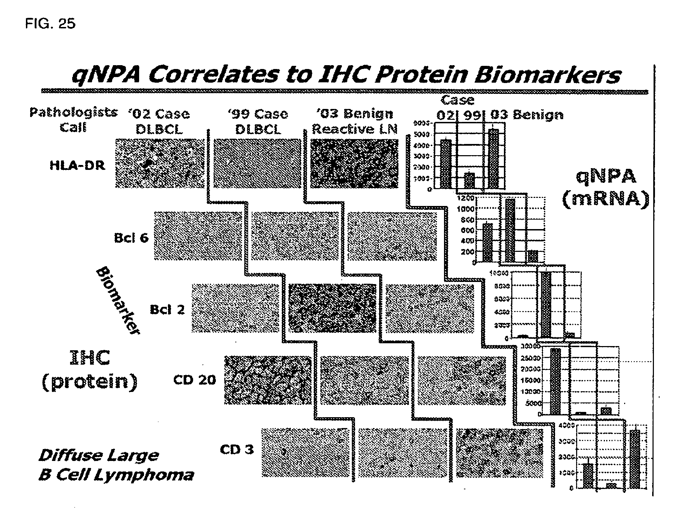

The clinical study on the gene expression in diffuse large B-cell lymphoma (DLBCL) cells are depicted in Figs. 12-25.

### Results

*Test performance on frozen tissues and comparison to FFPE:* For all 44 genes of interest, a suitable specific probe and linker probe could be designed. For 8 genes, the signal had to be lowered with undetectable competing oligonucleotide. For 4 genes, the signal had to be increased with additive quantitation of multiple probes as demonstrated in . Between the 2 housekeeping genes, PRKG1 and TBP, TBP had stronger and more uniform luminescence results. All data were therefore normalized to TBP which was artificially set at 1000 (data not shown). Assay performance was linear down to 0.125 mg of sample/well. RNase and DNase treatments demonstrated the assay was specific for RNA detection only. Testing of supernatant and cell lysate determined that RNA was not extracted from tissue (positive results using cell lysate only). This probably explains the good correlation between frozen and FFPE tissue since RNA extraction was not necessary from the tissues. CVs for quadruplicate runs on frozen benign lymph node ranged from 8-15% (data not shown).Comparisons between different types of tissue preparations were excellent for lysate vs. snap frozen (R2=0.989), snap frozen vs. FFPE (R2=0.991), lysate vs FFPE (R2=0.994) (data not shown).

### Verification of ArrayPlate technology to RT-PCR and IHC:

The ArrayPlate results accurately reflected the increase in mRNA of HLA-DRA, HLA-DRB, HLA-DQA, and HLA-DPA in the DB cell line transfectant clone 1-5 as demonstrated with quantitative RT-PCR (data not shown). Coefficients of variation from quadruplicate experiments on FFPE of these embedded cell lines ranged from 7-9% (Figure 4A). The ArrayPlate results correlated well with immunohistochemistry staining patterns of prognostically significant genes and gene products (Figures 5A-5D).

### Correlation between new and archived paraffin blocks of the same sample:

The correlation between the new versus the old paraffin block from the same biopsy was very good (Figure 4B). The new paraffin block was taken from a snap frozen tissue portion that had been frozen at the time of biopsy which was quickly defrosted, fixed in formalin, and embedded. The results were compared to a sister paraffin block made from the same biopsy at the time of the patient's surgery 18 years ago. This result indicates the applicability of the assay to very old archival material.

### Correlations between different mRNA quantification techniques.

Forty additional cases of archived FFPE DLBCL were analyzed with ArrayPlate analysis. 39 cases were successfully analyzed. One case had no signal at all. In situ hybridization with a polyT probe, demonstrated that this case did not have any intact mRNA. Results on the remaining 39 cases were compared to previous Affymetrix and competitive GEP arrays results using Spearman Rank Statistics, as shown in Table 2. Since not all the gene results were available on all platforms, there were frequent missing data as indicated in the table. The median correlation for ArrayPlate versus Affymetrix was 0.52, for ArrayPlate versus Lymphochip 0.55, and Affymetrix versus Lymphochip was 0.78. The latter correlation is understandably higher since those 2 analyses were performed on aliquots of RNA derived from the same frozen tissue block while the ArrayPlate analysis was performed on different block (although from the same specimen). Overall these are excellent correlations for this type of technology.

### Hazard ratios for prognostic genes as assessed with ArrayPlate as compared to other GEP techniques.

The results for the 44 genes were then compared for the 39 cases successfully analyzed using the ArrayPlate as compared to survival. At first, a univariate analyses of gene expression levels versus patient survival was performed. However, none of the genes was significantly correlated with survival, which was attributed to the low number of cases in this study group (since all of these genes were associated with survival in larger groups of patients). The hazard ratios of death for each gene was calculated. For hazard ratios >1, there is an increased risk of death and for ratios <1, there is a decreased risk of death. These hazard ratios usually trended in the same directions for each gene as they did in the larger datasets from which their significance was derived. Comparisons of agreement (results for hazard ratios above or below 1) was performed. For the comparison of Affymetrix versus Lymphochip data, the 2 methods agree for 29 of 33 genes (88%), for ArrayPlate versus Affymetrix, the 2 methods agree for 30 of 40 genes (75%), for ArrayPlate versus Lymphochip for 27 of 34 genes (79%).

Often the quantitative measurement of gene expression does not correlate well to the measurement of the levels of the protein products. This figure demonstrates that the measurement of gene expression from formalin fixed tissue where total RNA is measured, in this case by nuclease protection, can give results that correlate quantitatively to the levels of protein product measured *in situ* by immunohistochemical methods. Fixed tissue samples from three patients who had been diagnosed with diffuse large B-cell lymphoma or benign reactive lymph node were measured by the nuclease protection assay. The levels for three genes (HLA-DR, Bcl 2, and CD 20) are depicted by the bar graphs. For each patient the protein products of these genes were measured by immunohistochemistry (IHC). The stained slides are depicted, along with the relative quantitative level of each protein marker (high, medium, low expression). Note that the IHC protein levels correlated to the gene expression levels. The diagnosis of diffuse large B-cell lymphoma is based on histology and *in situ* measurements. These data suggest it can also be based on gene expression levels measured from the tissue using an assay where the spatial context has been lost, but total oligonucleotide levels are quantitatively measured, or additional prognostic and diagnostic information can be obtained using such methods.

### Example 3: Utilization of qNPA technique in research and/or diagnostics

The present invention can be utilized for basic science research and in diagnostic applications. For example, drug-induced changes in expression of genes/proteins can be routinely analyzed using hereinbefore described methods. A representative example is described below.

### Rifampicin-mediated modulation of genes

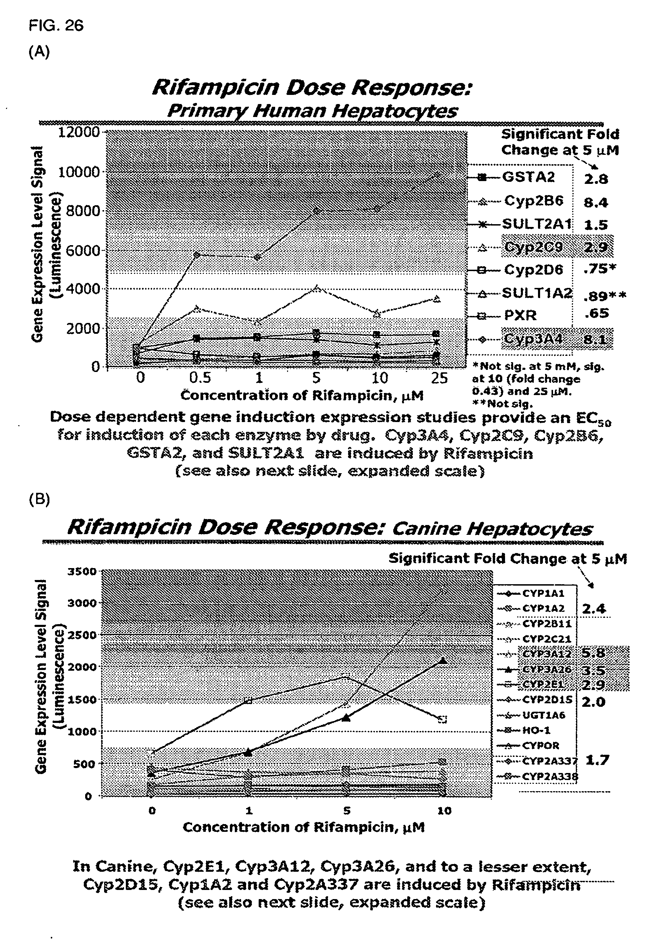

Modulation of gene expression by rifampicin, an antibiotic with known hepatotoxic effects, was analyzed using the aforementioned technique of qNPA. Human or canine primary liver cells were incubated with 0-10 µM rifampicin and the levels of the primary transcripts of various metabolic enzymes were analyzed using qNPA. These results are presented in Figs. 26-27.

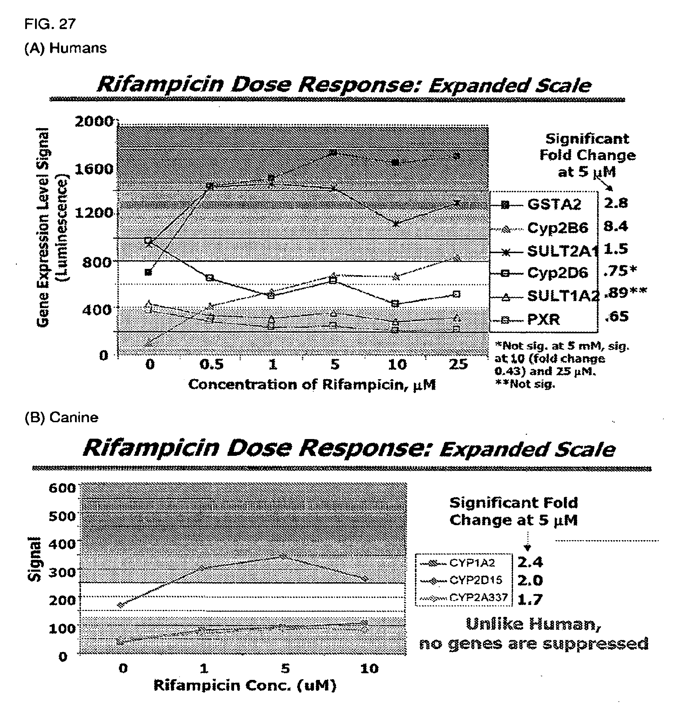

As presented in Panel (A) of Fig. 26, these gene expression studies provide a means for the pharmacological measurement (for example EC₅₀ levels) of enzyme induction by rifampicin. Additionally, comparative studies could be performed in humans and canine samples. In humans, the cytochrome P450 enzymes Cyp3A4, Cyp2C9, Cyp2B6, GSTA2, and SULT2A1 were induced by rifampicin. Cyp2D6 and PXR were suppressed, indicating adverse drug reactions. However, suppression of these enzymes were less than 50% (Panel A of Fig. 27)

In canine hepatocytes, Cyp1A1, Cyp2D15, and Cyp2A337 genes were upregualted. However, unlike human cells, rifampicin treatment did not result in the suppression of any analyzed genes. These results are shown in Panel (B) of Figs. 26 and 27.

From the foregoing description, one skilled in the art can easily ascertain the essential characteristics of this invention, and without departing from the scope thereof, can make changes and modifications of the invention to adapt it to various usage and conditions.

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

In the foregoing and in the examples, all temperatures are set forth uncorrected in degrees Celsius and, all parts and percentages are by weight, unless otherwise indicated.

The preceding examples can be repeated with similar success by substituting the generically or specifically described reactants and/or operating conditions of this invention for those used in the preceding examples.

## Claims

1. A method of quantifying the soluble and insoluble forms of at least one nucleic acid target in a fixed and/or cross-linked biological sample comprising:
(i) contacting said sample with at least one nuclease protection molecule (NPM), which is a nucleic acid that specifically binds to said target, under conditions effective for specific binding of said NPM to said target forms,
(ii) exposing said sample to one or more reagents under conditions that are effective to eliminate any unbound NPM,
(iii) separating the bound NPM from the target, and
(iv) detecting the presence of said NPM quantitatively.

2. A method according to Claim 1 wherein said target comprises a ribonucleic acid (RNA) molecule or a deoxyribonucleic (DNA) molecule, or an antisense polynucleotide that may contain unnatural bases.

3. A method according to Claim 1 or 2 wherein said NPM is a single stranded (ssDNA) or branched DNA (bDNA) molecule, or contains LNA or PNA or other unnatural bases.

4. A method according to any of Claims 1 to 3, wherein step (ii) comprises treatment with a nuclease effective to eliminate any unbound NPM.

5. A method according to any of Claims 1 to 4, wherein said target is an RNA molecule, siRNA, miRNA, or antisense RNA that may contain unnatural bases.

6. A method according to Claim 4 wherein said nuclease is an S1 nuclease.

7. A method according to any of Claims 1 to 6, wherein said fixing comprises treatment of said sample with ethanol, formalin, dithio-bis(succinimidyl propionate) (DSP), or wherein said cross-linking comprises treatment with bis[sulfosuccinimidyl] suberate (BS3), disuccinimidylsuberate (DSS), disuccinimidylglutarate (DSG), disuccinimidyl tartrate (DST), glutaraldehyde, or a derivative thereof.

8. A method according to any of Claims 1 to 7 further comprising amplification of the bound NPM before detection.

9. A method according to any of Claims 1 to 8 further comprising use of base and/or heat to separate the bound NPM from the target.

10. A method according to any of Claims 1 to 9 wherein the insoluble target is detected without extraction or solubilization of said insoluble target from the sample.

11. A method according to any of Claims 1 to 10 comprising detecting said NPM with a probe which specifically binds to said NPM or a portion thereof.

12. A method according to Claim 11, wherein a signal emitted by said hybridized probe is stoichiometrically related to the level of said target in said sample.

13. A method according to any of Claims 1 to 12 wherein said soluble and insoluble forms of the target are contacted with an excess of the at least one nuclease protection molecule (NPM) in step (i), and the presence of said NPM is detected quantitatively in step (iv).

14. A method according to any of Claims 1 to 13 in which both the soluble and insoluble forms of at least one nucleic acid target in a biological sample are detected independent of each other.

15. A method according to any of Claims 1 to 14, wherein the detection step comprises direct or indirect labeling of the NPM, capture of the NPM on a surface, or the use of electrophoresis, chromatography, mass spectroscopy, amplification or sequencing to detect the NPM.

16. A method according to any of Claims 1 to 15, wherein the soluble and insoluble forms of two or more nucleic acid targets in a fixed and/or cross-linked biological sample are detected.

17. A method according to any of Claims 1 to 16, wherein the soluble and insoluble forms of the at least one nucleic acid target is detected in multiple fixed and/or cross-linked biological samples.

18. A method according to Claim 17, wherein the soluble and insoluble forms of two or more nucleic acid targets is detected in the multiple fixed and/or cross-linked biological samples.

## Patentansprüche

1. Verfahren zum Quantifizieren der löslichen und unlöslichen Formen wenigstens eines Nucleinsäuretargets in einer fixierten und/oder vernetzten biologischen Probe, wobei
(i) die Probe mit wenigstens einem Nuclease-Protection-Molekül (NPM) kontaktiert wird, welches eine Nucleinsäure ist, die sich unter für die spezifische Bindung der NPM an die Targetformen wirksamen Bedingungen spezifisch an das Target bindet,
(ii) die Probe einem oder mehreren Reagenzien unter für die Entfernung von ungebundener NPM wirksamen Bedingungen ausgesetzt wird,
(iii)das gebundene NPM vom Target getrennt wird, und
(iv) das Vorhandensein des NPMs quantitativ erfasst wird.

2. Verfahren nach Anspruch 1, wobei das Target ein Ribonucleinsäure (RNA)-Molekül oder ein Desoxyribonucleinsäure(DNA)-Molekül oder ein Antisense-Polynucleotid umfasst, das optional unnatürliche Basen enthalten kann.

3. Verfahren nach Anspruch 1 oder 2, wobei das NPM ein einzelsträngiges (ssDNA)- oder verzweigtes DNA-(bDNA)-Molekül ist oder LNA oder PNA oder andere unnatürliche Basen enthält.

4. Verfahren nach einem der Ansprüche 1 bis 3, wobei Schritt (ii) die Behandlung mit einer zur Eliminierung von ungebundenem NPM wirksamen Nuclease einschließt.

5. Verfahren nach einem der Ansprüche 1 bis 4, wobei das Target ein RNA-, siRNA-, miRNA- oder Antisense-RNA-Molekül ist, das unnatürliche Basen enthalten kann.

6. Verfahren nach Anspruch 4, wobei die Nuclease eine Sl-Nuclease ist.

7. Verfahren nach einem der Ansprüche 1 bis 6, wobei das Fixieren die Behandlung der Probe mit Ethanol, Formalin, Dithiobis(succinimidyl-Propionat) (DSP) einschließt, oder wobei das Vernetzen die Behandlung mit Bis[sulfosuccinimidyl]-Suberat (BS3), Disuccinimidyl-Suberat (DSS), Disuccinimidyl-Glutarat (DSG), Disuccinimidyl-Tartrat (DST), Glutaraldehyd oder einem Derivat davon einschließt.

8. Verfahren nach einem der Ansprüche 1 bis 7, weiterhin umfassend die Amplifikation von gebundenem NPM vor dem Erfassen.

9. Verfahren nach einem der Ansprüche 1 bis 8, weiterhin umfassend die Verwendung einer Base und/ oder von Hitze zum Trennen von gebundenem NPM vom Target.

10. Verfahren nach einem der Ansprüche 1 bis 9, wobei das unlösliche Target ohne Extraktion oder Solubilisierung des unlöslichen Targets von der Probe erfasst wird.

11. Verfahren nach einem der Ansprüche 1 bis 10, umfassend das Erfassen des NPMs mit einer Sonde, die sich spezifisch an das NPM oder an einen Teil davon bindet.

12. Verfahren nach Anspruch 11, wobei ein von der hybridisierten Sonde ausgesandtes Signal stöchiometrisch auf das Niveau des Targets in der Probe bezogen ist.

13. Verfahren nach einem der Ansprüche 1 bis 12, wobei die löslichen und unlöslichen Formen des Targets mit einem Überschuss des wenigstens einen Nuclease-Protection-Moleküls (NPM) in Schritt (i) kontaktiert werden, und das Vorhandensein des NPMs in Schritt (iv) quantitativ erfasst wird.

14. Verfahren nach einem der Ansprüche 1 bis 13, wobei sowohl die löslichen als auch die unlöslichen Formen wenigstens eines Nucleinsäuretargets in einer biologischen Probe unabhängig voneinander erfasst werden.

15. Verfahren nach einem der Ansprüche 1 bis 14, wobei der Erfassungsschritt direktes oder indirektes Markieren des NPMs, Einfangen des NPMs auf einer Oberfläche oder das Verwenden von Elektrophorese, Chromatographie, Massenspektrometrie, Amplifikation oder Sequenzierung zum Erfassen des NPMs umfasst.

16. Verfahren nach einem der Ansprüche 1 bis 15, wobei die löslichen und unlöslichen Formen von zwei oder mehr Nucleinsäuretargets in einer fixierten und/oder vernetzten biologischen Probe erfasst werden.

17. Verfahren nach einem der Ansprüche 1 bis 16, wobei die löslichen und unlöslichen Formen des wenigstens einen Nucleinsäuretargets in einer fixierten und/oder vernetzten biologischen Probe erfasst werden.

18. Verfahren nach Anspruch 17, wobei die löslichen und unlöslichen Formen von zwei oder mehr Nucleinsäuretargets in den mehrfach fixierten und/oder vernetzten biologischen Proben erfasst werden.

## Revendications

1. Procédé de quantification des formes solubles et insolubles d'au moins un acide nucléique cible dans un échantillon biologique fixé et/ou réticulé comprenant:
(i) contacter cet échantillon avec au moins une molécule de protection contre la nucléase (MPN), qui se lie spécifiquement à cette cible, sous des conditions efficaces pour le liage spécifique de cette MPN à ces formes cibles,
(ii) exposer cet échantillon à un ou plusieurs réactifs sous des conditions, qui sont efficaces à éliminer de la MPN non liée,
(iii) séparer la MPN liée de la cible, et
(iv) détecter la présence de cette MPN de façon quantitative.

2. Procédé selon la revendication 1, dans lequel cette cible comprend une molécule d'acide ribonucléique (ARN) ou une molécule d'acide désoxyribonucléique (ADN), ou un polynucléotide antisens, qui optionnellement peut contenir des bases non naturelles.

3. Procédé selon la revendication 1 ou 2, dans lequel cette MPN est une molécule d'ADN à simple brin (ssADN) ou d'ADN ramifié (bDNA), ou contient un ALN ou APN ou d'autres bases non naturelles.

4. Procédé selon une des revendications 1 à 3, dans lequel l'étape (ii) comprend le traitement avec une nucléase efficaces à éliminer de la MPN non liée.

5. Procédé selon une des revendications 1 à 4, dans lequel cette cible est une molécule ARN, siRNA, miRNA, ou ARN antisens, qui peut contenir des bases non naturelles.

6. Procédé selon la revendication 4, dans lequel cette nucléase est une nucléase S1.

7. Procédé selon une des revendications 1 à 6, dans lequel cette fixation comprend le traitement de cet échantillon avec éthanol, formaline, dithio-bis(succinimidyl propionate) (DSP), ou dans lequel cette réticulation comprend le traitement avec subérate de bis[sulfosuccinimidyl] (BS3), subérate de disuccinimidyl (DSS), glutarate de disuccinimidyl (DSG), tartrate de disuccinimidyl (DST), glutaraldéhyde ou un dérivé de ceux-ci.

8. Procédé selon une des revendications 1 à 7, en outre comprenant l'amplification de la MPN liée avant la détection.

9. Procédé selon une des revendications 1 à 8, en outre comprenant l'utilisation d'une base et/ou de la chaleur pour séparer la MPN liée de la cible.

10. Procédé selon une des revendications 1 à 9, dans lequel la cible insoluble est détectée sans extraction ou solubilisation de cette cible insoluble de l'échantillon.

11. Procédé selon une des revendications 1 à 10 comprenant la détection de cette MPN avec une sonde, qui se lie spécifiquement à cette MPN ou une partie de celle-ci.

12. Procédé selon la revendication 11, dans lequel un signal émis par cette sonde hybridisée est stoeichiométriquement rapporté au niveau de cette cible dans cet échantillon.

13. Procédé selon une des revendications 1 à 12, dans lequel ces formes solubles et insolubles de la cible sont contactées avec un excès de l'au moins une molécule de protection contre la nucléase (MPN) dans l'étape (i), et la présence de cette MPN est détectée de façon quantitative dans l'étape (iv).

14. Procédé selon une des revendications 1 à 13, dans lequel les formes solubles aussi bien qu'insolubles d'au moins un acide nucléique cible dans un échantillon biologique sont détectées indépendantes les unes des autres.

15. Procédé selon une des revendications 1 à 14, dans lequel l'étape de détection comprend le marquage direct ou indirect de la MPN, la capture de la MPN sur une surface ou l'utilisation de l'électrophorèse, de la chromatographie, de la spectrométrie de masse, de l'amplification ou du séquençage pour détecter la MPN.

16. Procédé selon une des revendications 1 à 15, dans lequel les formes solubles et insolubles de deux ou d'une pluralité d'acides nucléiques cibles dans un échantillon biologique fixé et/ou réticulé sont détectées.

17. Procédé selon une des revendications 1 à 16, dans lequel les formes solubles et insolubles de l'au moins un acide nucléique cible sont détectées dans un échantillon biologique fixé et/ou réticulé.

18. Procédé selon la revendication 17, dans lequel les formes solubles et insolubles de deux ou d'une pluralité d'acides nucléiques cibles sont détectées dans des échantillons biologiques fixés et/ou réticulés multiples.
